# EUROPEAN PATENT APPLICATION

(11) **EP 4 095 132 A1**
(43) Date of publication of application: **30.11.2022**
(21) Application number: 21744678.0
(22) Date of filing: 20.01.2021
(51) Int. Cl.: C07D 401/04, A61K 31/497, A61P 35/00, C07D 401/14

(54) **ISOINDOLINE DERIVATIVE, AND PHARMACEUTICAL COMPOSITION AND USE THEREOF**

(30) Priority: 20.01.2020 CN 202010067409; 15.05.2020 CN 202010413162
(71) Applicant: Kangpu Biopharmaceuticals, Ltd., Shanghai 201203 (CN)
(72) Inventor: LIU, Hui, Shanghai 201203 (CN); DENG, Yanjun, Shanghai 201203 (CN); GE, Chuansheng, Shanghai 201203 (CN); ZHANG, Lei, Shanghai 201203 (CN); XING, Feng, Shanghai 201203 (CN)
(74) Representative: dompatent von Kreisler Selting Werner - Partnerschaft von Patent- und Rechtsanwälten mbB
(86) International application number: PCT/CN2021/072840
(87) International publication number: WO 2021/147889

(57) **Abstract**

Disclosed are a isoindoline derivative, an intermediate thereof, a preparation method therefor, a pharmaceutical composition thereof and the use thereof. The isoindoline derivatives of the present invention can specifically target and regulate various proteins by binding to Cereblon, thereby effectively treating cancers and other related diseases.

## Description

### Cross-Reference to Related Applications

The present application claims the priority to Chinese patent application No. CN 202010067409.0 filed on January 20, 2020 and Chinese patent application No. CN 202010413162.3 filed on May 15, 2020. Each application is incorporated herein by reference in its entirety.

### Technical Field

The present invention relates to an isoindoline derivative, an intermediate thereof, a preparation method therefor, a pharmaceutical composition thereof and the use thereof.

### Background Art

Cereblon is a protein encoded by CRBN gene in humans. Cereblon forms an E3 ubiquitin ligase complex with damaged DNA-binding protein 1 (DDB1), Cullin-4A (CUL4A), and regulator of cullin 1 (ROC1). This complex ubiquitinates a variety of other proteins. Through a mechanism that has not been fully elucidated, cereblon ubiquitination of target proteins leads to increased levels of fibroblast growth factor 8 (FGF8) and fibroblast growth factor 10 (FGF10), and FGF8 in turn regulates multiple developmental processes.

Multiple studies show that lenalidomide immunomodulators can bind to the protein cereblon and alter the specificity of the complex to induce ubiquitination and degradation of Ikaros (IKZF1) and Aiolos (IKZF3). IKZF1 and IKZF3 are key transcription factors in multiple myeloma. A single amino acid substitution of IKZF3 confers resistance to degradation induced by lenalidomide immunomodulators and is tolerant to cell growth inhibition induced by lenalidomide immunomodulators. Similarly, we found that lenalidomide immunomodulator-induced IL2 production in T cells is due to depletions of IKZF1 and IKZF3. These findings reveal a novel therapeutic mechanism of action, that is alteration of the activity of an E3 ubiquitin ligase, leading to selective degradation of specific targets (Jan Krönke, et al., Lenalidomide Causes Selective Degradation of IKZF1 and IKZF3 in Multiple Myeloma Cells, Science, 2014, 343(6168): 301-5).

There is a growing need in the art for effective treatment of tumors, as well as an urgent need in the art to develop a small-molecule therapeutic agent that specifically targets and regulates various tumor-related proteins by utilizing the substrate specificity of cereblon.

### Summary of the Invention

The present invention provides an isoindoline derivative, a preparation method therefor, a pharmaceutical composition thereof and the use thereof. The isoindoline derivatives of the present invention can specifically target and regulate various tumor-related proteins by binding to Cereblon, thereby effectively treating cancers and other related diseases.

The present invention provides an isoindoline derivative represented by general formula (I), a pharmaceutically acceptable salt, a solvate, a polymorph, a metabolite, a prodrug or a stereoisomer thereof: 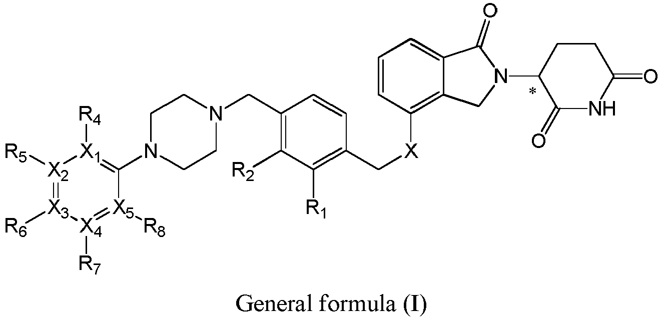 wherein, R₁ and R₂ are each independently selected from H, halogen, -CN, substituted or unsubstituted (C₁-C₁₂)alkyl, substituted or unsubstituted (C₁-C₁₂)alkoxy, or -OH, provided that: R₁ and R₂ are not both H;
X is selected from O or NH;
X₁, X₂, X₃, X₄, and X₅ are each independently selected from C or N;
R₄, R₅, R₆, R₇, and R₈ are each independently absent or selected from H, halogen, substituted or unsubstituted (C₁-C₁₂)alkyl, substituted or unsubstituted (C₁-C₁₂)alkoxy, substituted or unsubstituted (C₃-C₆)cycloalkyl, -CH=CH₂, -C≡CH , -OH, -CN, -NO₂, 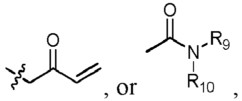 wherein R₉ and R₁₀ are each independently selected from H, D, or substituted or unsubstituted (C₁-C₁₂)alkyl, provided that: at least one of R₄, R₅, R₆, R₇ and R₈ is selected from -CN, -NO₂, -CH=CH₂, -C≡CH , 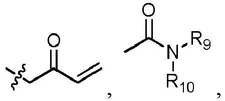 (C₁-C₁₂)alkyl substituted with one or more halogen, (C₁-C₁₂)alkoxy substituted with one or more halogen, (C₁-C₁₂)alkyl substituted with one or more D, or (C₁-C₁₂)alkoxy substituted with one or more D;
the substituents in the substituted (C₁-C₁₂)alkyl and substituted (C₁-C₁₂)alkoxy are selected from one or more D, one or more halogen, or one or more (C₃-C₆)cycloalkyl;
the carbon labeled with ^{∗} is an asymmetric center.

Preferably, in general formula (I), at least one of R₁ and R₂ is selected from halogen,-CN, substituted or unsubstituted (C₁-C₁₂)alkyl, substituted or unsubstituted (C₁-C₁₂)alkoxy or -OH.

Preferably, in general formula (I), at least one of R₁ and R₂ is selected from F, Cl, Br,-CN, -CH₃, -OCH₃, -CF₃, or -OCF₃.

More preferably, in general formula (I), at least one of R₁ and R₂ is selected from F.

Preferably, in general formula (I), X is selected from O.

In one embodiment, in general formula (I), X is selected from O, and X₁, X₂, X₃, X₄, and X₅ are each independently selected from C.

In one embodiment, X is selected from O, and at least one of X₁, X₂, X₃, X₄, and X₅ is selected from N.

Preferably, in general formula (I), X is selected from NH

In one embodiment, in general formula (I), X is selected from NH, and X₁, X₂, X₃, X₄ and X₅ are each independently selected from C.

In one embodiment, X is selected from NH, and at least one of X₁, X₂, X₃, X₄ and X₅ is selected from N.

In one embodiment, in general formula (I), the R₄ is selected from -CN, -NO₂, 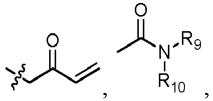 (C₁-C₁₂)alkyl substituted with one or more halogen, (C₁-C₁₂)alkoxy substituted with one or more halogen, (C₁-C₁₂)alkyl substituted with one or more D, or (C₁-C₁₂)alkoxy substituted with one or more D, wherein R₉ and R₁₀ are each independently selected from H, D, or substituted or unsubstituted (C₁-C₁₂)alkyl; and R₅, R₆, R₇ and R₈ are each independently selected from H or halogen.

In one embodiment, R₄ is selected from -CN, (C₁-C₁₂)alkyl substituted with one or more halogen, (C₁-C₁₂)alkoxy substituted with one or more halogen, (C₁-C₁₂)alkyl substituted with one or more D, or (C₁-C₁₂)alkoxy substituted with one or more D, and R₅, R₆, R₇ and R₈ are each independently selected from H.

In one embodiment, in general formula (I), the R₅ is selected from -CN, -NO₂, 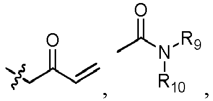 (C₁-C₁₂)alkyl substituted with one or more halogen, (C₁-C₁₂)alkoxy substituted with one or more halogen, (C₁-C₁₂)alkyl substituted with one or more D, or (C₁-C₁₂)alkoxy substituted with one or more D, wherein R₉ and R₁₀ are each independently selected from H, D, or substituted or unsubstituted (C₁-C₁₂)alkyl; and R₄, R₆, R₇ and R₅ are each independently selected from H or halogen.

In one embodiment, R₅ is selected from -CN, (C₁-C₁₂)alkyl substituted with one or more halogen, (C₁-C₁₂)alkoxy substituted with one or more halogen, (C₁-C₁₂)alkyl substituted with one or more D, or (C₁-C₁₂)alkoxy substituted with one or more D, and R₄, R₆, R₇ and R₈ are more preferably each independently selected from H.

In one embodiment, in general formula (I), the R₆ is selected from -CN, -NO₂, 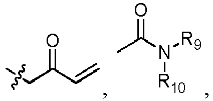 (C₁-C₁₂)alkyl substituted with one or more halogen, (C₁-C₁₂)alkoxy substituted with one or more halogen, (C₁-C₁₂)alkyl substituted with one or more D, or (C₁-C₁₂)alkoxy substituted with one or more D, wherein R₉ and R₁₀ are each independently selected from H, D, or substituted or unsubstituted (C₁-C₁₂)alkyl; and R₄, R₅, R₇ and R₈ are each independently selected from H or halogen.

In one embodiment, R₆ is selected from -CN, (C₁-C₁₂)alkyl substituted with one or more halogen, (C₁-C₁₂)alkoxy substituted with one or more halogen, (C₁-C₁₂)alkyl substituted with one or more D, or (C₁-C₁₂)alkoxy substituted with one or more D, and R₄, R₅, R₇ and R₈ are more preferably each independently selected from H.

In one embodiment, in general formula (I), the R₇ is selected from -CN, -NO₂, 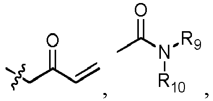 (C₁-C₁₂)alkyl substituted with one or more halogen, (C₁-C₁₂)alkoxy substituted with one or more halogen, (C₁-C₁₂)alkyl substituted with one or more D, or (C₁-C₁₂)alkoxy substituted with one or more D, wherein R₉ and R₁₀ are each independently selected from H, D, or substituted or unsubstituted (C₁-C₁₂)alkyl; and R₄, R₅, R₆ and R₈ are each independently selected from H or halogen.

In one embodiment, R₇ is selected from -CN, (C₁-C₁₂)alkyl substituted with one or more halogen, (C₁-C₁₂)alkoxy substituted with one or more halogen, (C₁-C₁₂)alkyl substituted with one or more D, or (C₁-C₁₂)alkoxy substituted with one or more D, and R₄, R₅, R₆ and R₈ are more preferably each independently selected from H.

In one embodiment, in general formula (I), the R₈ is selected from -CN, -NO₂, 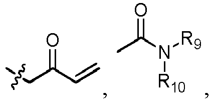 (C₁-C₁₂)alkyl substituted with one or more halogen, (C₁-C₁₂)alkoxy substituted with one or more halogen, (C₁-C₁₂)alkyl substituted with one or more D, or (C₁-C₁₂)alkoxy substituted with one or more D, wherein R₉ and R₁₀ are each independently selected from H, D, or substituted or unsubstituted (C₁-C₁₂)alkyl; and R₄, R₅, R₆ and R₇ are each independently selected from H or halogen.

In one embodiment, R₈ is selected from -CN, (C₁-C₁₂)alkyl substituted with one or more halogen, (C₁-C₁₂)alkoxy substituted with one or more halogen, (C₁-C₁₂)alkyl substituted with one or more D, or (C₁-C₁₂)alkoxy substituted with one or more D, and R₄, R₅, R₆ and R₇ are more preferably each independently selected from H.

Preferably, in general formula (I), the asymmetric center refers to an achiral carbon, an (S)-configured carbon, an enriched (S)-configured carbon, a (R)-configured carbon, an enriched (R)-configured carbon or a racemate.

Preferably, in general formula (I), the halogen is F, Cl, Br or I.

Preferably, in general formula (I), the substituted or unsubstituted (C₁-C₁₂)alkyl is preferably selected from substituted or unsubstituted (C₁-C₆)alkyl, and more preferably selected from substituted or unsubstituted (C₁-C₄)alkyl, wherein the substituted or unsubstituted (C₁-C₄)alkyl is most preferably selected from substituted or unsubstituted methyl, substituted or unsubstituted ethyl, substituted or unsubstituted n-propyl, substituted or unsubstituted isopropyl, substituted or unsubstituted n-butyl, substituted or unsubstituted isobutyl, or substituted or unsubstituted tert-butyl; and the substituent described with reference to the phrase "substituted or unsubstituted" is selected from one or more of D, halogen, or (C₃-C₆)cycloalkyl, preferably selected from D, F, Cl, Br, I, cyclopropyl, cyclobutyl, cyclopentyl or cyclohexyl, more preferably selected from D, F, Cl, cyclopropyl or cyclobutyl, and most preferably selected from F.

In one embodiment, the substituted or unsubstituted (C₁-C₁₂)alkyl is preferably selected from methyl, ethyl, n-propyl, isopropyl, n-butyl, isobutyl, tert-butyl, -CD₃, -CD₂H, -CDH₂, -CF₃, -CH₂F, -CHF₂, 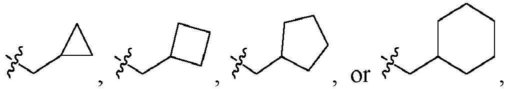 more preferably selected from methyl, ethyl, n-propyl, isopropyl, -CD₃, -CF₃, -CH₂F, -CHF₂, 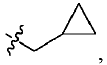 or 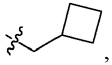 and most preferably selected from methyl, ethyl, -CD₃, -CF₃, -CH₂F, or -CHF₂.

Preferably, in the isoindoline derivative represented by general formula (I), the pharmaceutically acceptable salt, the solvate, the polymorph, the metabolite, the prodrug or the stereoisomer thereof, the substituted or unsubstituted (C₁-C₁₂)alkoxy is preferably selected from substituted or unsubstituted (C₁-C₆)alkoxy, and more preferably selected from substituted or unsubstituted (C₁-C₄)alkoxy, wherein the substituted or unsubstituted (Ci-C₄)alkoxy is most preferably selected from substituted or unsubstituted methoxy, substituted or unsubstituted ethoxy, substituted or unsubstituted n-propoxy, substituted or unsubstituted isopropoxy, substituted or unsubstituted n-butoxy, substituted or unsubstituted isobutoxy, or substituted or unsubstituted tert-butoxy; and the substituent described with reference to the phrase "substituted or unsubstituted" is selected from one or more of D, halogen, or (C₃-C₆)cycloalkyl, preferably selected from D, F, Cl, Br, I, cyclopropyl, cyclobutyl, cyclopentyl, or cyclohexyl, more preferably selected from D, F, Cl, cyclopropyl, or cyclobutyl, and most preferably selected from D or F.

In one embodiment, the substituted or unsubstituted (C₁-C₁₂)alkoxy is preferably selected from methoxy, ethoxy, n-propoxy, isopropoxy, n-butoxy, isobutoxy, tert-butoxy, - OCD₃, -OCD₂H, -OCDH₂, -OCF₃, -OCH₂F, -OCHF₂, 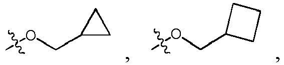 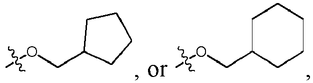 more preferably selected from methoxy, ethoxy, n-propoxy, isopropoxy, -OCD₃, -OCF₃, -OCH₂F, -OCHF₂, 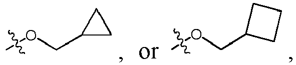 and most preferably selected from methoxy, ethoxy, -OCD₃, -OCF₃, -OCH₂F, or -OCHF₂.

In one embodiment, the substituted or unsubstituted (C₃-C₆)cycloalkyl is selected from substituted or unsubstituted cyclopropyl, substituted or unsubstituted cyclobutyl, substituted or unsubstituted cyclopentyl, or substituted or unsubstituted cyclohexyl.

In one embodiment, R₁ is selected from H, F, Cl, Br, -CN, methyl, methoxy, or -OH; and R₁ is preferably selected from F, Cl, -CH₃, -CN, or -OH, and R₁ is more preferably selected from F.

In one embodiment, R₂ is selected from H, F, Cl, Br, -CN, methyl, methoxy, or -OH; and R₂ is selected from preferably selected from F, Cl, -CH₃, -CN, or -OH, and R₂ is more preferably selected from F.

In one embodiment, R₁ is selected from F, Cl, Br, -CN, -CH₃, -OCH₃, -CF₃, or -OCF₃; and R₂ is selected from H.

In one embodiment, R₂ is selected from F, Cl, Br, -CN, -CH₃, -OCH₃, -CF₃, or -OCF₃; and R₁ is selected from H.

In one embodiment, R₁ is selected from F; and R₂ is selected from H.

In one embodiment, R₂ is selected from F; and R₁ is selected from H.

Preferably, in general formula (I), one of R₄, R₅, R₆, R₇, and R₈ is selected from -CN,-NO₂, -CH=CH₂, -C≡CH , 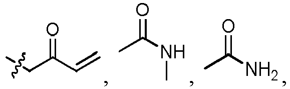 -OCF₃, -OCH₂F, -OCHF₂, -CD₃, - OCD₃, or -CF₃.

In one embodiment, R₄ is selected from H, F, Cl, Br, -CH₃, -CH₂CH₃, -CF₃, -OCH₃, - OCH₂CH₃, -CH=CH₂, -C≡CH, -OCF₃, -OCH₂F, -OCHF₂, -CD₃, -OCD₃, -CN, -NO₂, 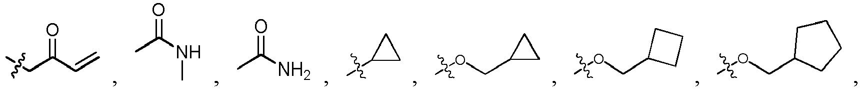 or 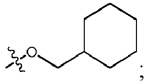 R₄ is preferably selected from H, F, -CN, -OCH₃, -OCF₃, -OCD₃, or -CD₃; R₄ is more preferably selected from H or F; and R₄ is most preferably selected from H.

In one embodiment, R₅ is selected from H, F, Cl, Br, -CH₃, -CH₂CH₃, -CF₃, -OCH₃, - OCH₂CH₃, -CH=CH₂, -C≡CH , -OCF₃, -OCH₂F, -OCHF₂, -CD₃, -OCD₃, -CN, -NO₂, 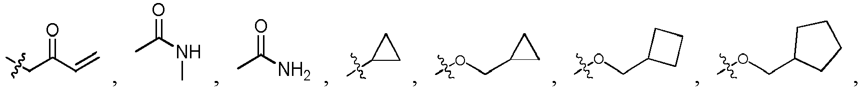 or 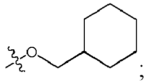 R₅ is preferably selected from H, F, -CN, -OCH₃, -OCF₃, -OCD₃, or -CD₃; R₅ is more preferably selected from H or F; and R₅ is most preferably selected from H.

In one embodiment, R₆ is selected from H, F, Cl, Br, -CH₃, -CH₂CH₃, -CF₃, -OCH₃, - OCH₂CH₃, -CH=CH₂, -C≡CH , -OCF₃, -OCH₂F, -OCHF₂, -CD₃, -OCD₃, -CN, -NO₂, 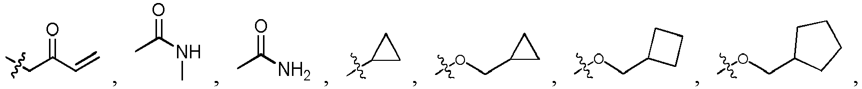 or 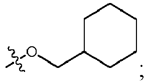 R₆ is preferably selected from H, F, -CN, -OCH₃, -OCF₃, -OCD₃, or -CD₃; R₆ is more preferably selected from F or -CN; and R₆ is most preferably selected from -CN.

In one embodiment, R₇ is selected from H, F, Cl, Br, -CH₃, -CH₂CH₃, -CF₃, -OCH₃, - OCH₂CH₃, -CH=CH₂, -C≡CH , -OCF₃, -OCH₂F, -OCHF₂, -CD₃, -OCD₃, -CN, -NO₂, 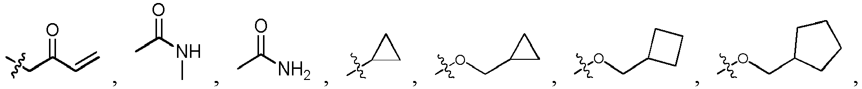 or 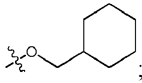 ; R₇ is preferably selected from H, F, -CN, -OCH₃, -OCF₃, -OCD₃, or -CD₃; R₇ is more preferably selected from H, F or -OCH₃; and R₇ is most preferably selected from - OCH₃.

In one embodiment, R₈ is selected from H, F, Cl, Br, -CH₃, -CH₂CH₃, -CF₃, -OCH₃,-OCH₂CH₃, -CH=CH₂, -C≡CH, -OCF₃, -OCH₂F, -OCHF₂, -CD₃, -OCD₃, -CN, -NO₂, 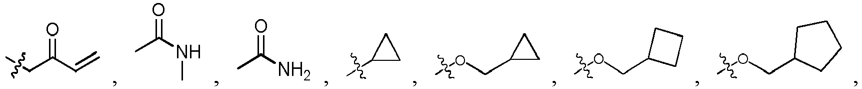 or 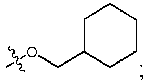 R₈ is preferably selected from H, F, -CN, -OCH₃, -OCF₃, -OCD₃, or -CD₃; R₈ is more preferably selected from F or H; and R₈ is most preferably selected from F.

Preferably, the isoindoline derivative represented by general formula (I) is selected from any one of the following compounds:

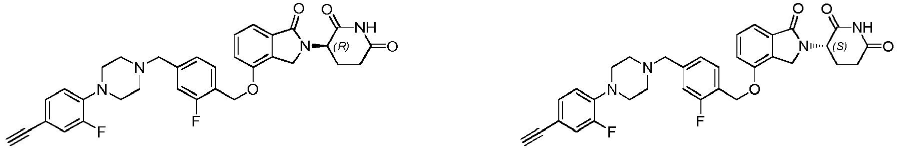

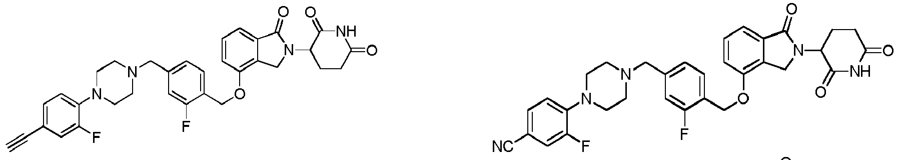

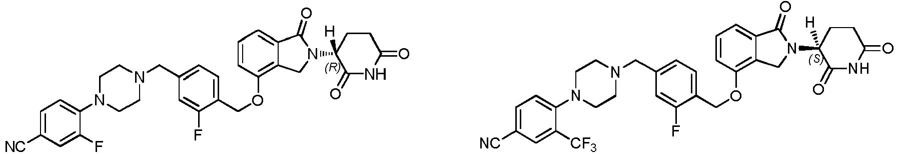

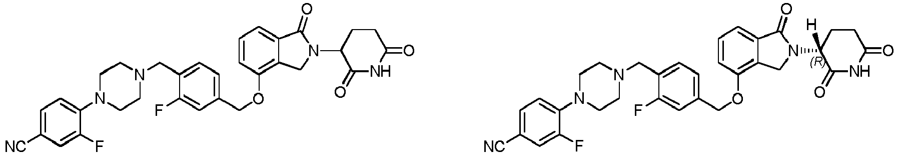

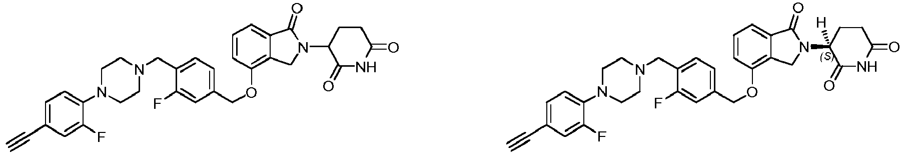

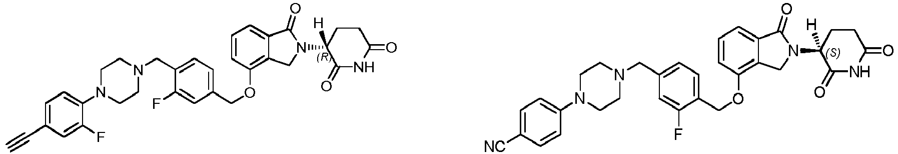

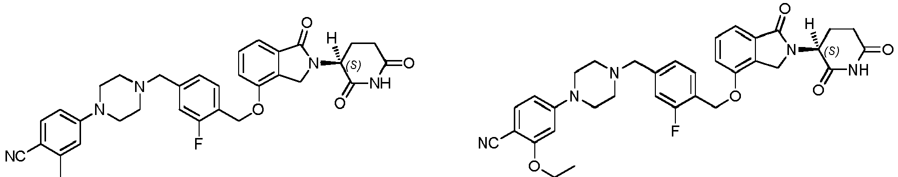

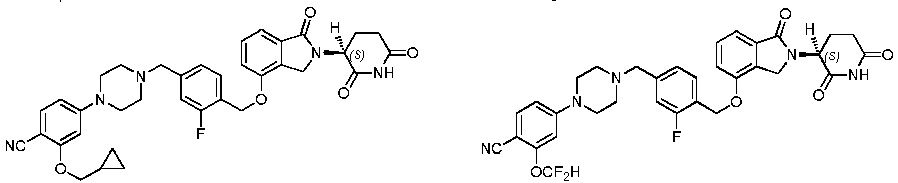

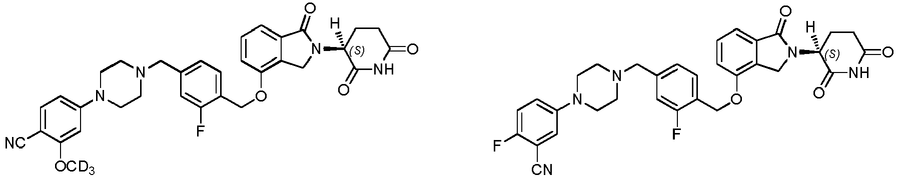

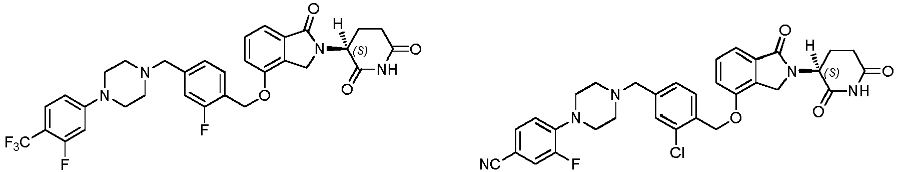

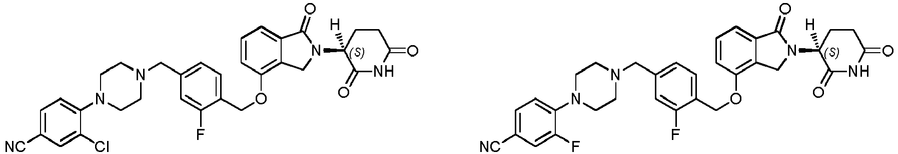

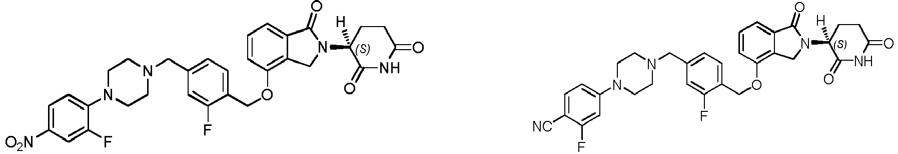

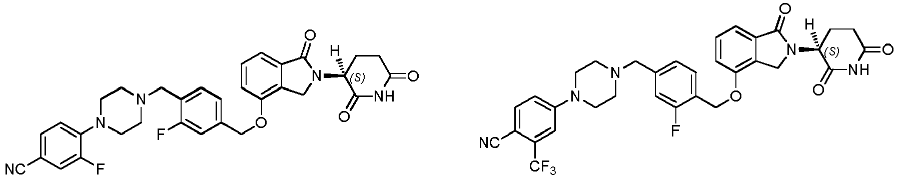

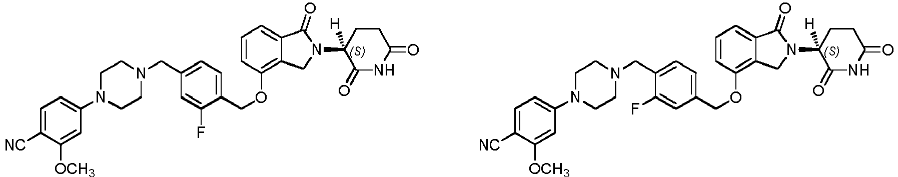

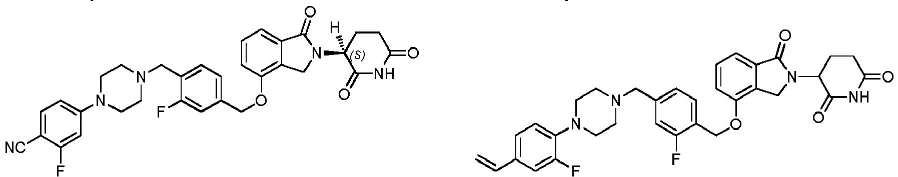

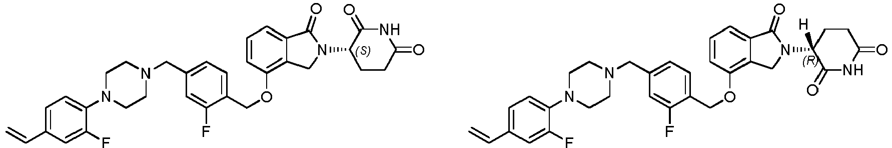

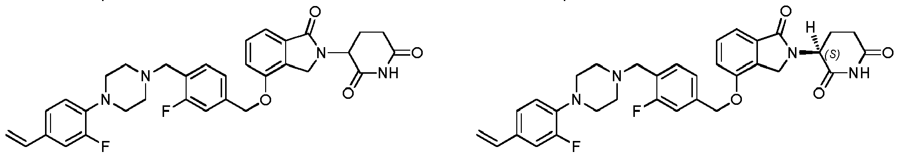

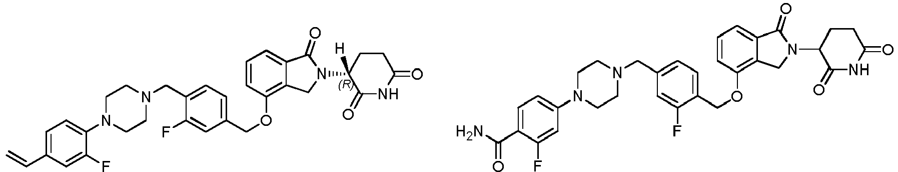

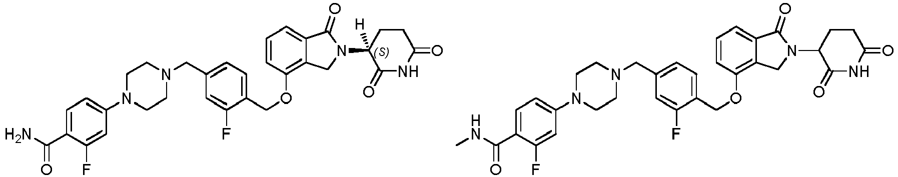

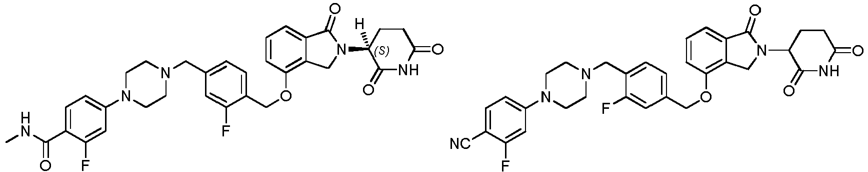

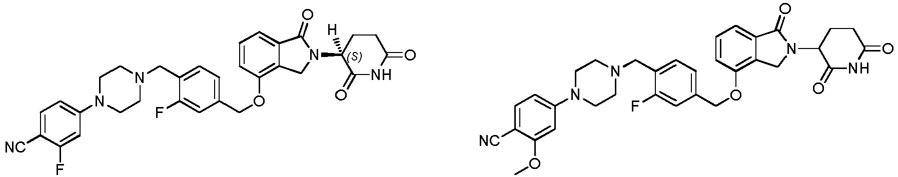

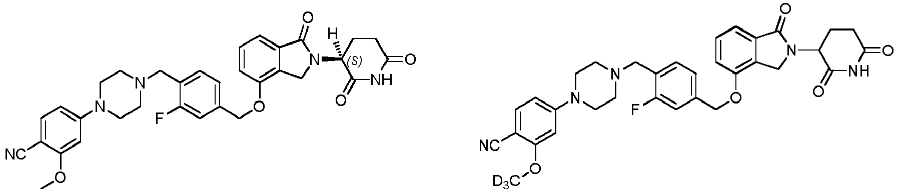

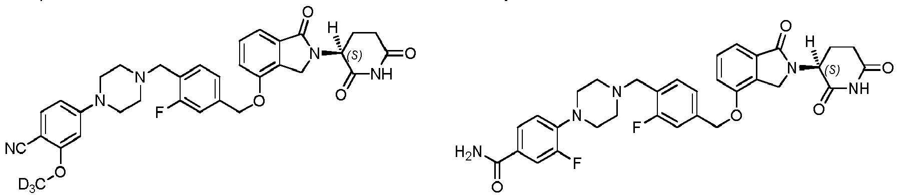

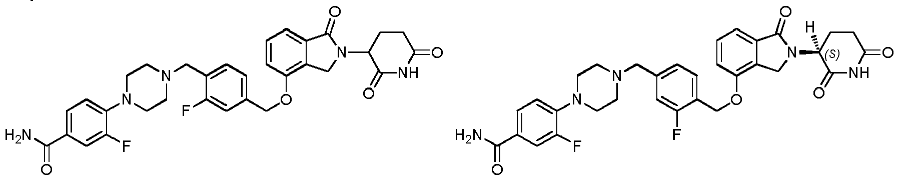

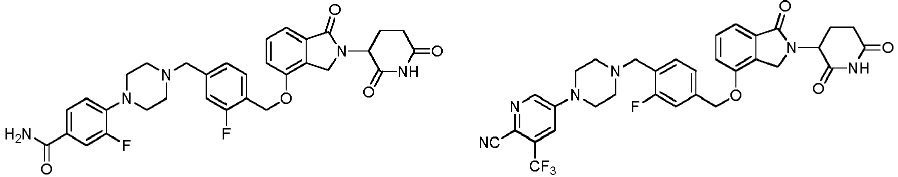

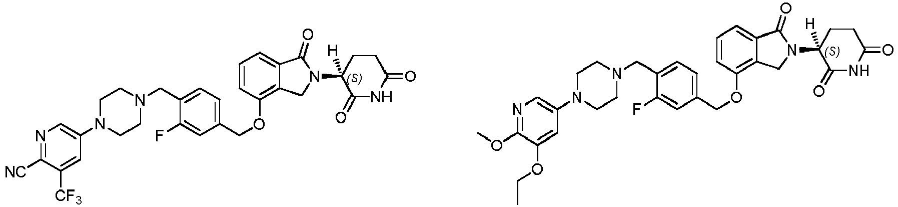

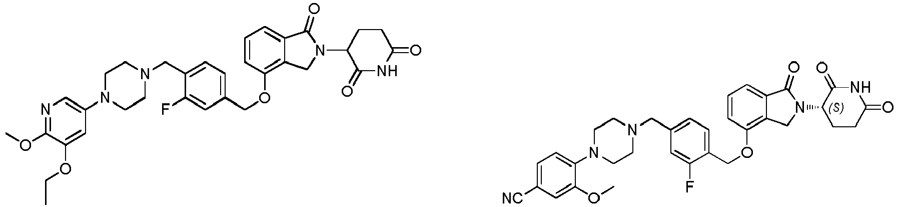

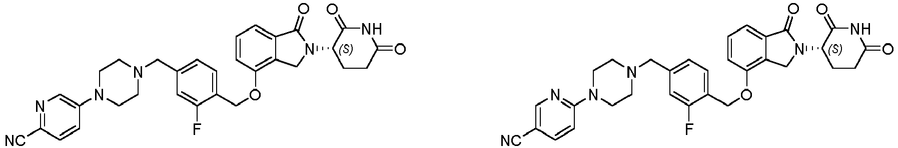

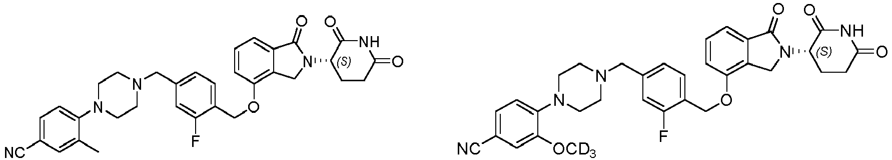

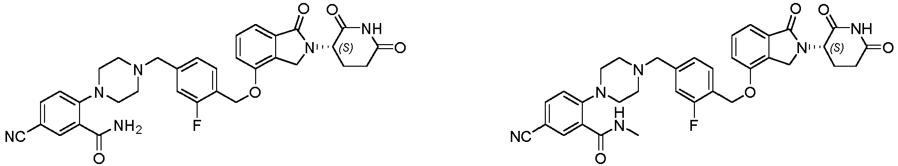

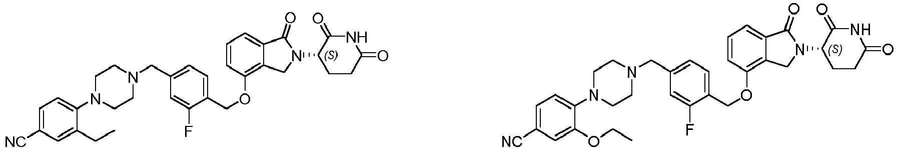

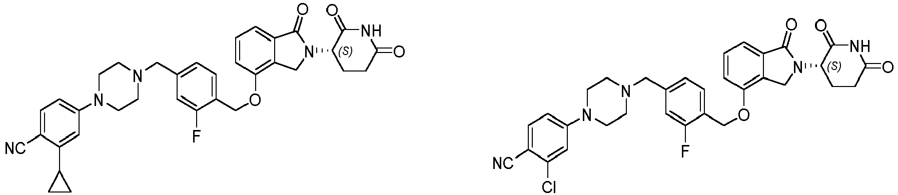

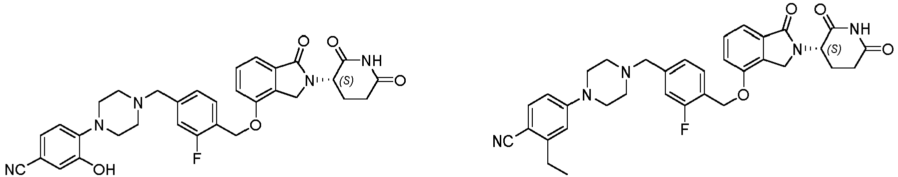

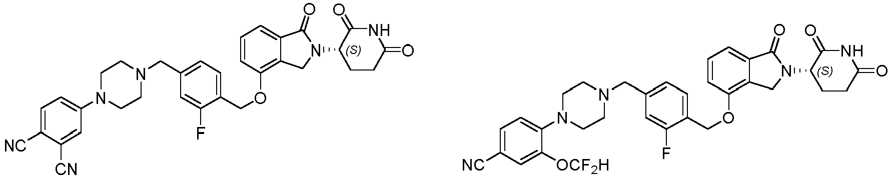

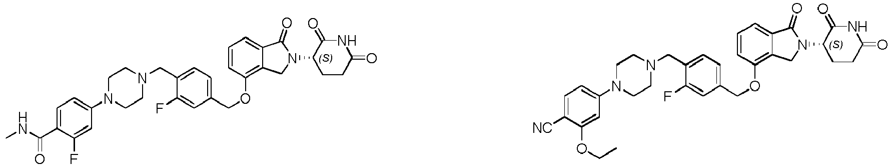

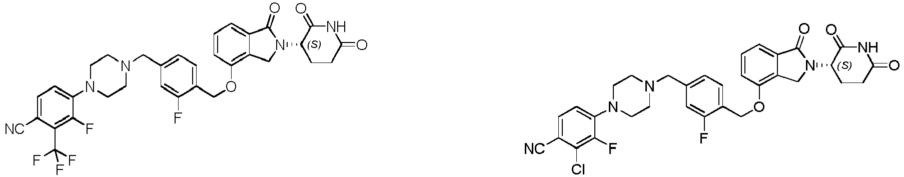

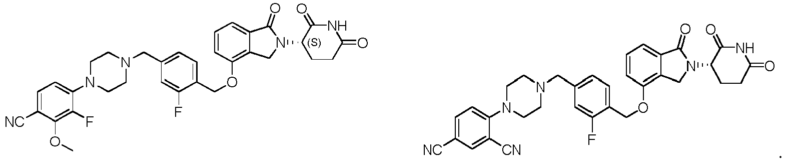

Deuterium (D or ²H) is a stable non-radioactive isotope of hydrogen and the atomic weight thereof is 2.0144. Hydrogen exists in the form of an isotopic mixture of H (hydrogen or protium), D (²H or deuterium) and T (³H or tritium) in nature, wherein the deuterium abundance is 0.0156%. According to the common technical knowledge in the art, in all the compounds whose structures contain natural hydrogen atoms, the hydrogen atom actually represents a mixture of H, D and T. Therefore, if a compound contains a deuterium whose abundance is greater than the natural abundance thereof, i.e., 0.0156 % at any position, these compounds should be considered to be non-natural or deuterium enriched, and thus these compounds are novel relative to the non-enriched analogues thereof.

In the present invention, "D", "deuterium" or "deuterium enriched" compound refers to a compound of general formula (I), or a compound of a pharmaceutically acceptable salt, a solvate, a polymorph, a stereoisomer, a metabolite or a prodrug thereof, wherein the deuterium abundance at any relevant position is greater than the natural abundance thereof at the position. Therefore, in the "D", "deuterium" or "deuterium enriched" compound, the deuterium abundance at any of the relevant positions is likely between greater than 0.0156% and 100%. The deuterium enriched position is represented by D, whereas the non-deuterium enriched position is represented by H. According to the common technical knowledge in the art, the symbol H may be elided at the non-deuterium enriched position. An example of a method for obtaining a deuterium enriched compound is replacing the hydrogen with the deuterium, or using deuterium-enriched starting material to synthesize the compound.

In the present invention, the percentage of the deuterium in the enriched deuterium or the deuterium abundance refers to molar percentage.

In the present invention, the phrase "non-deuterium enriched" refers to the hydrogen in nature, namely, the hydrogen in the form of an isotopic mixture of H (hydrogen or protium), D (²H or deuterium) and T (³H or tritium).

The present invention also provides a method for preparing the isoindoline derivative represented by general formula (I), which can be synthesized from commercially available raw materials by means of methods commonly used in the field of chemical synthesis, for example, reference is made to the synthetic methods disclosed in WO 2016065980 A1, WO 2019014100 A1, etc.

In one embodiment, the compound of general formula (I) can be obtained by reacting compound A-01, 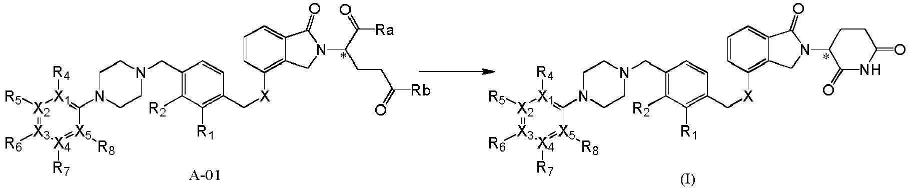 wherein, the definitions of X, X₁-X₅, ^{∗}, R₁, R₂, R₄, R₅, R₆, R₇, and R₈ are the same as described above, one of Ra and Rb is 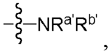 and the other is 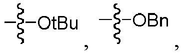 or 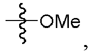 and R^{a'} and R^{b'} are independently H.

The present invention also provides a pharmaceutical composition, comprising a therapeutically and/or prophylactically effective amount of the foregoing isoindoline derivative represented by general formula (I), the pharmaceutically acceptable salt, the solvate, the polymorph, the metabolite, the prodrug or the stereoisomer thereof.

According to the embodiments of the present invention, the pharmaceutical composition may be formulated into any form for administration, including injection (intravenous), mucosal administration, oral administration (solid and liquid preparations), inhalation, ocular administration, rectal administration, topical or parenteral (infusion, injection, implantation, subcutaneous, intravenous, intraarterial and intramuscular) administration. The pharmaceutical composition of the present invention can also be a controlled release or delayed release dosage form. Examples of solid oral preparations include, but are not limited to, powders, capsules, caplets, soft capsules, and tablets. Examples of liquid preparations for oral or mucosal administration include, but are not limited to, suspensions, emulsions, elixirs, and solutions. Examples of topical preparations include, but are not limited to, emulsions, gels, ointments, creams, patches, pastes, foams, lotions, drops, or serum preparations. Examples of preparations for parenteral administration include, but are not limited to, solutions for injection, dry preparations which can be dissolved or suspended in a pharmaceutically acceptable carrier, suspensions for injection and emulsions for injection. Examples of other suitable preparations of the compound of general formula (I), the pharmaceutically acceptable salt, the solvate, the polymorph, the metabolite, the prodrug or the stereoisomer thereof, include, but are not limited to, eye drops and other ophthalmic preparations; aerosol: such as nasal sprays or inhalants; liquid dosage forms suitable for parenteral administration; suppositories and pastilles.

The pharmaceutical composition according to the present invention may further comprise a pharmaceutically acceptable excipient, such as those widely used in drug manufacture field. The excipient is mainly used to provide a safe, stable and functionalized pharmaceutical composition, and can also provide a method for dissolving the active ingredients at a desired rate after the subject receives administration or for promoting the effective absorption of the active ingredients after the subject is administered with the composition. The excipient may be inert fillers or provide certain functions, such as stabilizing the overall pH value of the composition or preventing the degradation of the active ingredients of the composition.

According to the embodiments of the present invention, the pharmaceutically acceptable excipient may comprise one or more adhesives, suspending agents, emulsifiers, diluents, fillers, granulating agents, adhesives, disintegrating agents, lubricants, anti-adhesive agents, glidants, wetting agents, gelling agents, absorption retarders, dissolution inhibitors or reinforcing agents, adsorbents, buffers, chelating agents, preservatives, colorants, flavoring agents and sweetening agents. The pharmaceutically acceptable carrier can take a variety of forms depending on the form of preparation desired for administration. For example, for liquid oral preparation, the suitable carriers and additives include water, glycols, oils, alcohols, flavor enchancements, preservatives, colorants, etc. As another illustrative example, for solid oral preparation, suitable carriers and additives include starch, sugar, diluents, granulating agents, lubricants, adhesives, disintegrating agents, etc. The pharmaceutically acceptable carriers or excipients usually should be non-toxic. The pharmaceutical composition according to the present invention may comprise one or more suitable carrier(s)/excipient(s). The amount and type of the excipient will vary with the requirements. Those of ordinary skill in the art can easily determine the appropriate carrier(s)/excipient(s) to be added to the pharmaceutical composition of the present invention based on the present disclosure.

The pharmaceutical composition of the present invention, i.e., the composition comprising a therapeutically or prophylactically effective amount of the compound of general formula (I), the pharmaceutically acceptable salt, the solvate, the polymorph, the metabolite, the prodrug or the stereoisomer thereof provided in the present invention, can be prepared according to the disclosure using any method known to those skilled in the art. For example, the pharmaceutical composition according to the present invention can be prepared by mixing the compound of general formula (I), the pharmaceutically acceptable salt, the solvate, the polymorph, the stereoisomer, the metabolite, or the prodrug thereof with a pharmaceutically acceptable carrier according to conventional drug compounding technologies, which include but are not limited to conventional mixing, dissolving, granulating, emulsifying, grinding, encapsulating, embedding or lyophilization processes.

According to the embodiments of the present invention, in addition to one or more of the compound of general formula (I), the pharmaceutically acceptable salt, the solvate, the polymorph, the metabolite, the prodrug or the stereoisomer thereof, the pharmaceutical composition can further comprise one or more additional therapeutic agents. More details of the additional therapeutic agents that may be included in the pharmaceutical combination of the present invention will be disclosed below. The amount and type of the additional therapeutic agents will depend on the diseases, disorders or conditions to be treated or prevented; the severity of the diseases, disorders or conditions; factors of the subject administrated with the composition, such as age, weight, and physical conditions; and the route of administration, etc.

In certain embodiments of the present invention, the therapeutic or prophylactic amount of the compound of general formula (I), the pharmaceutically acceptable salt, the solvate, the polymorph, the metabolite, the prodrug or the stereoisomer thereof, or any pharmaceutical composition, preparation thereof, etc., may be administered to the subject within a period of time (administration period) followed by a period of no administration of the compound (non-administration period) by means of the method of the present invention. The administration period and non-administration period can be repeated for desired times. The desired length and times of the administration period or non-administration period will depend on the type and/or severity of the diseases, disorders or conditions being treated or prevented, as well as the sex, age, weight, and other parameters (e.g. the individual subject's biological, physical, and physiological status, etc.) of the individual subject. According to the content disclosed in the application, those of ordinary skill in the art have the skill level sufficient to determine the appropriate length and times of the administration period and/or non-administration period.

According to the embodiments of the present invention, the combined use of the compound represented by general formula (I), the pharmaceutically acceptable salt, the solvate, the polymorph, the metabolite, the prodrug or the stereoisomer thereof according to the present invention with the additional therapeutic agents may play a synergistic effect in the treatment or prevention of any disease, disorder or condition according to the content disclosed in the present invention.

According to the embodiments of the present invention, the additional therapeutic agents may be naturally occurring, semi-synthetic or synthetic compounds. In another embodiment, the additional therapeutic agents may be a small molecule, such as synthetic organic or inorganic molecules; or larger molecules or biomolecules, such as proteins or nucleic acids having a pharmacological activity. In another embodiment, the additional therapeutic agents may be an anti-angiogenic compound, an immunomodulatory compound, an immunotherapeutic compound, a chemotherapeutic compound or a hormone compound.

In one embodiment of the present invention, a composition comprising the compound of general formula (I), the pharmaceutically acceptable salt, the solvate, the polymorph, the metabolite, the prodrug or the stereoisomer thereof and an additional therapeutic agent is administered to a subject simultaneously. In another embodiment, the compound of general formula (I), the pharmaceutically acceptable salt, the solvate, the polymorph, the metabolite, the prodrug or the stereoisomer thereof and an additional therapeutic agent are administered in a sequential order. In another embodiment, the compound of general formula (I), the pharmaceutically acceptable salt, the solvate, the polymorph, the metabolite, the prodrug or the stereoisomer thereof and an additional therapeutic agent are administered separately. The additional therapeutic agent may be administered before, sequentially with or after the administration of the compound of general formula (I), the pharmaceutically acceptable salt, the solvate, the polymorph, the metabolite, the prodrug or the stereoisomer thereof according to the present invention.

One or more additional therapeutic agents that may be administered in combination with the compound of general formula (I) or the pharmaceutically acceptable salt, the solvate, the polymorph, the metabolite, the prodrug or the stereoisomer thereof according to the present invention would depend on a variety of factors, such as diseases, disorders or conditions which need to be prevented or treated. Those of ordinary skill in the art can easily determine the appropriate additional therapeutic agents for use in combination with the isoindoline derivative represented by general formula (I), the pharmaceutically acceptable salt, the solvate, the polymorph, the metabolite, the prodrug, or the stereoisomer thereof according to the content disclosed in the present invention.

According to one embodiment of the present invention, when the compound of general formula (I), the pharmaceutically acceptable salt, the solvate, the polymorph, the metabolite, the prodrug or the stereoisomer thereof according to the present invention is administered in combination with the additional therapeutic agents, the therapeutically effective amount of the compound represented by general formula (I), the pharmaceutically acceptable salt, the solvate, the polymorph, the metabolite, the prodrug or the stereoisomer thereof is less than the therapeutically effective amount of same that will be required when the compound of general formula (I), the pharmaceutically acceptable salt, the solvate, the polymorph, the metabolite, the prodrug, or the stereoisomer thereof is not administered in combination with the additional therapeutic agents. In another embodiment, the therapeutically effective amount of the additional therapeutic agent is less than the effective amount of same when the compound of general formula (I), the pharmaceutically acceptable salt, the solvate, the polymorph, the metabolite, the prodrug or the stereoisomer thereof is not administrated.

According to the embodiments of the present invention, when the compound of general formula (I), the pharmaceutically acceptable salt, the solvate, the polymorph, the metabolite, the prodrug, or the stereoisomer thereof and the additional therapeutic agents are administered to a subject for treating or preventing diseases, disorders or conditions, the compound of general formula (I), the pharmaceutically acceptable salt, the solvate, the polymorph, the metabolite, the prodrug, or the stereoisomer thereof and the additional therapeutic agents may be administered by the same route or by a different route. The additional therapeutic agents may be administered by any route described herein, including but not limited to oral administration, inhalation, injection, ocular administration, mucosal administration, rectal administration, emulsion, liposome, long-acting implant or sustained controlled release process. The specific route of administration of the additional therapeutic agent will depend on the additional therapeutic agent itself and the preparation, as well as the diseases, disorders or conditions to be prevented or treated. According to the content of the present disclosure, a person of ordinary skill in the art has the skill level sufficient to determine the route of administration of the additional therapeutic agent.

The compound of general formula (I), the pharmaceutically acceptable salt, the solvate, the polymorph, the metabolite, the prodrug or the stereoisomer thereof according to the present invention may be employed for a variety of uses, which include, but are not limited to, the use in the preparation of a drug for treating related diseases, disorders or conditions by inducing ubiquitination and degradation of target proteins in cells.

Therefore, in another general aspect, the present invention relates to the use of the compound of general formula (I), the pharmaceutically acceptable salt, the solvate, the polymorph, the metabolite, the prodrug or the stereoisomer thereof in the preparation of a drug for treating or preventing diseases, disorders or conditions. In another aspect, the present invention relates to a method for treating or preventing related diseases, disorders or conditions by inducing ubiquitination and degradation of target proteins in cells, wherein the method comprises administering to a subject a therapeutically or prophylactically effective amount of the isoindoline derivative represented by general formula (I), the pharmaceutically acceptable salt, the solvate, the metabolite, the prodrug or the stereoisomer thereof. According to the method of the present invention, examples of such diseases, disorders and conditions to be treated or prevented include, but are not limited to, cancers.

In a preferred embodiment, the diseases, disorders or conditions are cancers, preferably multiple myeloma.

Various publications, articles, and patents are cited or described herein. The citation or description of these references or the incorporation herein in their entirety or the discussion about them intends to illustrate the background of the present invention, but not to mean that the content thereof form a part of the prior art of the present invention.

Unless defined otherwise, all technical and scientific terms used herein have the same meaning as commonly understood by those of ordinary skill in the field to which the present invention belongs. Otherwise, certain terms used herein have the meanings set forth in this description. All patents, published patent applications, and publications cited herein are incorporated by reference as if fully set forth herein. It should be noted that the singular forms as used herein and in the appended claims include the plural meaning unless the context clearly dictates otherwise.

As used herein, when a specific salt, composition, and excipient, etc. are mentioned to be "pharmaceutically acceptable", it means that the salt, the composition, the excipient, etc. are generally non-toxic, safe, and suitable for use in a subject, preferably in a mammal subject, more preferably in a human subject.

The term "pharmaceutically acceptable salt" used herein refers to a pharmaceutically acceptable organic or inorganic salt.

As used herein, the term "metabolite" refers to an active substance produced after a drug molecule undergoes changes in chemical structure *in vivo,* and the active substance is generally a derivative of the aforementioned drug molecule, and can also be chemically modified.

As used herein and unless otherwise specified, the term "polymorph" refers to one or more crystal structures formed by differently arranged molecules in the lattice space when crystallized.

As used herein, the term "solvate" refers to a crystal form of the compound of general formula (I), the pharmaceutically acceptable salt, the polymorph, the stereoisomer, the metabolite or the prodrug thereof, which further comprises one or more solvent molecule(s) incorporated into the crystal structure. The solvate may include a stoichiometric amount or a non-stoichiometric amount of a solvent, and the solvent molecule in the solvent may exist by means of ordered or non-ordered arrangement. The solvate containing a non-stoichiometric amount of solvent molecules may be obtained by the loss of at least one solvent molecule (but not all) from the solvate. In a particular embodiment, a solvate refers to a hydrate, which means the crystal form of the compound further comprises water molecules as the solvent.

As used herein and unless otherwise specified, the term "prodrug" refers to a derivative of the compound comprising a biologically reactive functional group such that the biological reactive functional group can be cleaved from the compound or react in other ways to provide the compound under biological conditions (*in vitro* or *in vivo*)*.* Usually, the prodrug is inactive, or at least has an activity lower than that of the compound itself, so that the compound starts to exhibit its activity until it is cleaved from the biologically reactive functional group. The biologically reactive functional group may be hydrolyzed or oxidized under biological conditions to provide the compound. For example, the prodrug may comprise a biologically hydrolyzable group. Examples of the biologically hydrolyzable group include but are not limited to: a biologically hydrolyzable phosphate, a biologically hydrolyzable ester, a biologically hydrolyzable amide, a biologically hydrolyzable carbonate, a biologically hydrolyzable carbamate and a biologically hydrolyzable ureide.

The compound of general formula (I), the pharmaceutically acceptable salt, the solvate, the polymorph, the stereoisomer, the metabolite or the prodrug thereof of the present invention may contain one or more asymmetric centers ("stereoisomers"). As used herein, the term "stereoisomer" refers to all stereoisomers including enantiomers, diastereoisomers, epimers, endo-exo isomers, atropisomers, regioisomers, cis- and trans-isomers, etc. The "stereoisomer" herein also includes "pure stereoisomer" and "enriched stereoisomer" or "racemate" of the various aforementioned stereoisomers. These stereoisomers can be separated, purified and enriched by means of asymmetric synthetic methods or chiral separation methods (including but not limited to thin layer chromatography, rotation chromatography, column chromatography, gas chromatography and high-pressure liquid chromatography) and can also be obtained by means of chiral resolution via forming bonds (chemical bonding, etc.) or forming salts (physical bonding) with other chiral compounds, etc. The "pure stereoisomer" herein refers to a stereoisomer of the compound concerned with the mass content of no less than 95% relative to other stereoisomers of the compound. The "enriched stereoisomer" herein refers to a stereoisomer of the compound concerned with the mass content of no less than 50% relative to other stereoisomers of the compound. The term "racemate" herein refers to a stereoisomer of the compound concerned with the mass content equal to that of other stereoisomers of the compound.

As used herein, the term "subject" refers to any animal that will be or has been administered the compound or the composition according to the embodiments of the present invention, preferably mammals, and most preferably humans. As used herein, the term "mammals" includes any mammal. Examples of mammals include but are not limited to cattle, horse, sheep, pig, cat, dog, mouse, rat, rabbit, guinea pig, monkey, human, etc., most preferably humans.

In one embodiment, "treat" or "being treated" refers to improvement, prevention or reversal of a disease or disorder or at least one identifiable symptom thereof, such as treating cancers and undesirable angiogenesis-related disorders by reducing or stabilizing the symptoms of the cancers or the disorders. In another embodiment, "treat" or "being treated" refers to improvement, prevention or reversal of at least one measurable body parameter of a disease or disorder being treated which may have not been identified in mammals. However, in another embodiment, "treat" or "being treated" refers to slowing of progression of a disease or disorder, either physically, for example stabilization of an identifiable symptom, or physiologically, for example stabilization of a body parameter, or both. In another embodiment, "treat" or "being treated" refers to delaying of the onset of a disease or disorder.

In some embodiments, the compound of interest is administered as a precaution. As used herein, "prevent" or "being prevented" refers to a reduction in the risk of suffering from a given disease or disorder. In a preferred mode of embodiments, the specified compound is administered as a precaution to a subject, such as a subject having a family history of or tendency to have cancers or autoimmune diseases.

As used herein, "therapeutically effective amount" refers to an amount of the compound or the composition that can lead to a biological or medical response (which is being sought by researchers, veterinarians, physicians, or other clinicians) in a tissue system, an animal or a person, which may include the relief of symptoms of the disease or disorder being treated. In a preferred embodiment, the therapeutically effective amount is an amount enough to effectively treat, improve the treatment of or prevent cancers, disorders or undesirable blood vessel-related conditions.

The term "prophylactically effective amount" refers to an amount of the active compound or medicament that can inhibit the onset of a disorder (sought by researchers, veterinarians, physicians or other clinicians) in a subject. A prophylactically effective amount of the compound refers to an amount of the therapeutic agent used alone or in combination with an additional therapeutically active compound, which can provide a therapeutic benefit in the treatment or prevention of diseases, disorders or conditions.

Unless otherwise specified, the singular form ("a" or "an") of the term used herein also includes a plural meaning.

Unless otherwise specified, the term "or" or "and" used herein refers to "and/or".

Unless otherwise specified, " 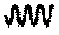 " or " 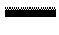 " appearing in specific groups herein refers to an attaching position.

On the basis of not departing from common knowledge in the art, the above-mentioned various preferred conditions can be combined in any manner, such that various preferred examples of the present invention are obtained.

Reagents and raw materials used in the present invention are all commercially available.

The positive effect of the present invention lies in that:
the isoindoline derivative represented by general formula (I) of the present invention can induce the ubiquitination and degradation of target proteins in cells, thereby effectively treating cancers and other related diseases.

The experimental data shows that the compound of the present application has a good inhibitory effect on multiple myeloma.

### Detailed Description of Embodiments

### Example 1

### Compound I-1

### Synthetic route:

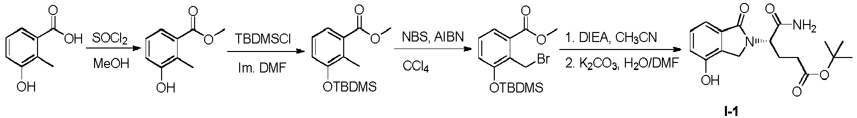

Step A. Under nitrogen protection, 3-hydroxy-2-methylbenzoic acid (CAS 603-80-5, 30.0 g, 197 mmol) was dissolved in anhydrous methanol (400 mL), and DMF (2 mL) and thionyl chloride (70.3 g, 591 mmol) were added dropwise at 0°C. After the dropwise addition was completed, the reaction solution was heated to reflux and reacted overnight. Then the reaction solution was cooled down to room temperature (25°C) and concentrated to dryness under reduced pressure. The residue was dissolved in ethyl acetate (250 mL). The mixture was washed successively with saturated aqueous sodium bicarbonate solution (250 mL), water (250 mL) and saturated brine (250 mL), dried over anhydrous sodium sulfate, and concentrated under reduced pressure to obtain methyl 3-hydroxy-2-methylbenzoate as a gray solid (30.5 g, yield: 93%).

¹H NMR (CDCl₃, 300 MHz): δ 7.41 (dd, *J* = 8.1, 1.2 Hz, 1H), 7.10 (td, *J* = 7.8, 0.6 Hz, 1H), 6.94 (dd, *J* =8.1, 1.2 Hz, 1H),5.25 (s, 1H), 3.89 (s, 3H), 2.45 (s, 3H).

Step B. Under nitrogen protection, methyl 3-hydroxy-2-methylbenzoate (30.5 g, 184 mmol) and imidazole (31.2 g, 459 mmol) were dissolved in anhydrous DMF (200 mL) and the resulting solution was cooled to 0°C in an ice bath. TBDMSCl (tert-butyldimethylsilyl chloride, 33.2 g, 220 mmol) was added in portions to the solution. The resulting mixture was warmed to room temperature and stirred overnight. The reaction solution was concentrated under reduced pressure, ethyl acetate (250 mL) and water (250 mL) were added and the organic phase was separated. The aqueous phase was back extracted with ethyl acetate (250 mL). The organic phases were combined, washed successively with water (250 mL) and saturated brine (250 mL), dried over anhydrous sodium sulfate, and concentrated under reduced pressure to obtain methyl 3-((tert-butyldimethylsilyl)oxy)-2-methylbenzoate as a yellow liquid (51.5 g, yield: 100%), which was directly used in the next reaction.

¹H NMR (CDCl₃, 300 MHz): δ 7.42 (dd. *J* = 7.8, 1.2 Hz, 1H), 7.05-7.11 (m, 1H), 7.42 (dd, *J* = 8.1, 1.2 Hz, 1H), 3.87 (s, 3H), 2.42 (s, 3H), 1.02 (s, 9H), 0.21 (s, 6H).

Step C. The compound methyl 3-((tert-butyldimethylsilyl)oxy)-2-methylbenzoate (51.5 g, 184 mmol), AIBN (1.49 g, 9.2 mmol) and NBS (39.3 g, 220 mmol) were added to carbon tetrachloride (600 mL). The reaction solution was refluxed and reacted overnight. The reaction solution was concentrated under reduced pressure, and then the residue was subjected to column chromatography (petroleum ether/ethyl acetate=20/1) to obtain methyl 2-bromomethyl 3-((tert-butyldimethylsilyl)oxy)benzoate as a light yellow liquid (63.1 g, yield: 96%).

¹H NMR (CDCl₃, 300 MHz): δ 7.51 (dd, *J* = 7.5,1.2 Hz, 1H), 7.22 (t, *J=* 8.1 Hz, 1H), 6.98 (dd. *J=* 8.4. 1.5 Hz, 1H), 5.02 (s, 2H), 3.92 (s, 3H), 1.06 (s, 9H), 0.30 (s, 6H).

Step D. The compound methyl 2-bromomethyl 3-((tert-butyldimethylsilyl)oxy)benzoate (5.00 g, 13.9 mmol) and compound 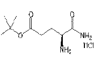 (CAS 108607-02-9, 3.65 g, 15.3 mmol) were dissolved in anhydrous acetonitrile (50 mL), and diisopropylethylamine (5.20 mL, 29.2 mmol) was added dropwise under nitrogen atmosphere at room temperature (30°C). The reaction solution was warmed to 40°C and stirred overnight. The reaction solution was concentrated under reduced pressure. The residue was diluted with dichloromethane (100 mL), washed with water (50 mL × 2) and saturated brine (30 mL), dried over anhydrous sodium sulfate, and concentrated under reduced pressure to obtain a light yellow oil. The obtained oil was dissolved in DMF (17.2 mL) and a solution of K₂CO₃ (884 mg, 6.40 mmol) in water (1.89 mL) was added dropwise at 0°C. After the dropwise addition was completed, the reaction solution was warmed to room temperature (25°C) and stirred for 50 minutes. The reaction solution was adjusted to neutral by dropwise adding IN dilute hydrochloric acid (11 mL) at 0°C and concentrated under reduced pressure. Then the residue was subjected to column chromatography (dichloromethane/methanol=50/1) to obtain I-1 as a white foam (1.40 g, yield: 22%).

1H NMR (CDCl₃, 300 MHz): δ 8.81 (br s, 1H), 7.20-7.31 (m, 2H), 6.95-6.98 (m, 2H), 6.09 (br s, 1H), 4.94-4.99 (m, 1H), 4.56 (d, J = 17.7 Hz, 1H), 4.43 (d, J = 17.4 Hz, 1H), 2.08-2.35 (m, 4H), 1.39 (s, 9H).

### Example 2

### Compound 1-2

### Synthetic route:

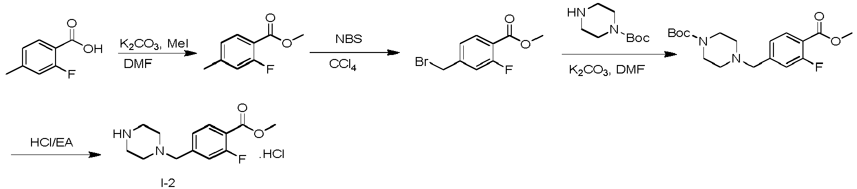

Step A. 2-fluoro-4-methylbenzoic acid (50 g, 324.4 mmol, CAS 7697-23-6) was dissolved in 400 mL of DMF, and K₂CO₃ (67 g, 486 mmol) was added. The reaction solution was stirred at room temperature for 0.5 hours, iodomethane (26.3 mL, 422 mmol) was added at 0°C, and the resulting mixture was reacted at room temperature for 16 hours. The reaction solution was poured into 1500 mL of water. The mixture was extracted with EtOAc (500 mL × 2), washed with saturated brine (500 mL × 2), dried and concentrated under reduced pressure to obtain a product, methyl 2-fluoro-4-methylbenzoate (51 g, yield: 94%) as a white solid, which was directly used in the next step without purification.

Step B. Methyl 2-fluoro-4-methylbenzoate (48 g, 285 mmol) was dissolved in 400 mL of CCl₄, NBS (N-bromosuccinimide, 55.9 g, 314 mmol) and BPO (13.8 g, 57.2 mmol) were added, and the reaction solution was heated to 80°C in an oil bath and reacted overnight. The reaction solution was cooled down to room temperature, filtered and concentrated under reduced pressure, and the residue was dispersed in 500 mL of EtOAc, then washed with saturated brine (500 mL × 2), dried and concentrated under reduced pressure to obtain a product, methyl 4-(bromomethyl)-2-fluorobenzoate as a yellow solid (70 g). The above-mentioned solid product methyl 4-(bromomethyl)-2-fluorobenzoate (70 g) and N-Boc piperazine (35 g, 0.188 mol, CAS 57260-71-6) were added to DMF (500 mL) and K₂CO₃ (65 g, 0.47 mol) was added. The mixture was heated to 70°C in an oil bath and reacted for 16 h. The reaction solution was cooled down to room temperature, then filtered and washed with EtOAc (200 mL), and extracted with EtOAc (500 mL × 3). The extract was washed with water and brine and dried and the filtrate was concentrated to dryness under reduced pressure to obtain a product, tert-butyl 4-(3-fluoro-4-(methoxycarbonyl)benzyl)piperazine-1-carboxylate, as a yellow oil (80 g). MS(ESI) *m*/*z* 353.4 [M+H]⁺.

Step C. The compound tert-butyl 4-(3-fluoro-4-(methoxycarbonyl)benzyl)piperazine-1-carboxylate (80 g) was dissolved in EtOAc (500 mL) and HCl/EtOAc (5 N, 400 mL) was added. The reaction solution was stirred for 16 hours at room temperature, the precipitated solid was filtered, and the resulting solid was concentrated to dryness under reduced pressure to obtain a hydrochloride 1-2. MS (ESI) m/z 253.2[M+H]⁺.

### Example 3

### Compound CM-1

### Synthetic route:

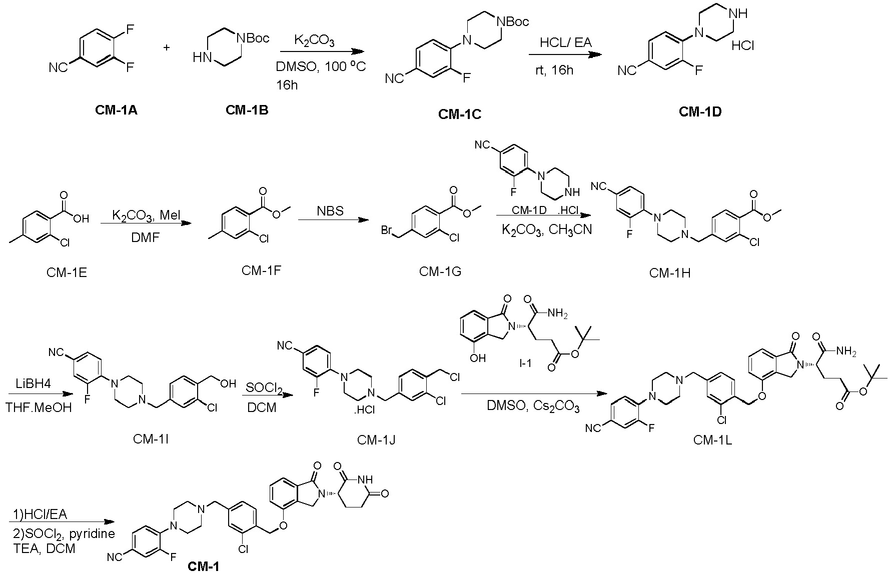

Step A. Compound CM-1A (3,4-difluorobenzonitrile, 11.8 g, 84.83 mmol, CAS 64248-62-0) and N-Boc-piperazine (18.96 g, 101.8 mmol) were added to DMSO (100 mL) and K₂CO₃ (17.56 g, 127.2 mmol) was added. The resulting mixture was heated to 100°C in an oil bath overnight, cooled down to room temperature, and then poured into 600 mL of water. The mixture was stirred for 15 minutes, then filtered and washed with water, and the filter cake was concentrated to dryness under reduced pressure using an oil pump to obtain CM-1**C** as a white solid (28 g). Then HCl/EA (5 M, 300 mL) was added, the resulting mixture was stirred at room temperature overnight and filtered, and the filter cake was concentrated to dryness under reduced pressure using an oil pump to obtain CM-1**D** (3-fluoro-4-(piperazin-1-yl)benzonitrile hydrochloride, 23.0 g) as a white solid, MS: [M+H]⁺= 206.1.

Step B. CM-IE, namely 2-chloro-4-methylbenzoic acid (5 g, 29.3 mmol, CAS 7697-25-8) was dissolved in 40 mL of DMF, and K₂CO₃ (6.1 g, 44 mmol) was added. The resulting mixture was stirred at room temperature for 0.5 hours, iodomethane (5.4 g, 38.1 mmol) was added at 0°C, and the reaction solution was reacted at room temperature for 4 hours. The reaction solution was poured into 300 mL of water. The resulting mixture was extracted with EtOAc (200 mL × 2), then washed with saturated brine (200 mL × 2), dried and concentrated under reduced pressure to obtain a product CM-1F as a yellow oil (methyl 2-chloro-4-methylbenzoate, 5.2 g, yield: 96%), which was directly used in the next step without purification.

Step C. CM-1F (5.0 g, 27.1 mmol, CAS 195318-63-9) was dissolved in 100 mL of CCl4, NBS (5.7 g, 32.5 mmol) and BPO (354 mg, 1.46 mmol) were added, and the reaction solution was heated to 80°C in an oil bath and reacted overnight. The reaction solution was cooled down to room temperature and filtered. The filtrate was washed with saturated brine (100 mL × 2), dried and concentrated under reduced pressure, and the residue was purified by silica gel column chromatography (PE/EtOAc=100 : 1-80 : 1) to obtain CM-1**G** as a yellow liquid (methyl 4-(bromomethyl)-2-chlorobenzoate, 4.4 g, yield: 61%).

¹H NMR (400 MHz, CDCl₃) δ 7.83-7.81 (m , 1H), 7.69 (s, 1H), 7.54-7.52 (m, 1H), 4.74 (s, 2H), 3.86 (s, 3H).

Step D. Compound CM-1**G** (4.0 g, 15 mmol) and CM-1**D** (3.62 g, 15 mmol) were added to CH₃CN (80 mL) and then K₂CO₃ (2.34 g, 38 mmol) was added. The resulting mixture was heated to 80°C in an oil bath for 16 h, cooled down to room temperature, then filtered and washed with EtOAc, and extracted with EtOAc (50 mL × 2). The extract was washed with water and brine and dried and the filtrate was subjected to evaporation under reduced pressure to dryness to obtain CM-1**H** (methyl 2-chloro-4-((4-(4-cyano-2-fluorophenyl)piperazin-1-yl)methyl)benzoate, 4.5 g, 11.6 mmol, 77%) as a yellow solid. MS: [M+H]⁺ = 388.1.

Step E. With reference to the synthetic method of steps B-D in example 7 (compound CM-3), compound CM-1L, tert-butyl-(*S*)-5-amino-4-(4-((2-chloro-4-((4-(4-cyano-2-fluorophenyl)piperazin-1-yl)methyl)benzyl)oxy)-1-oxoisoindolin-2-yl)-5-oxopentanoate, can be synthesized by replacing CM-3C with CM-1H.

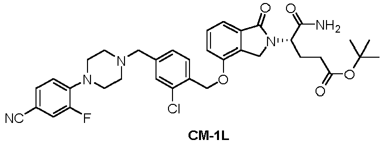

MS: [M+H]⁺= 676.2.

Step F. With reference to the synthetic method of steps E-F in example 9 (compound CM-6), compound CM-1 can be synthesized by replacing CM-6G, i.e., tert-butyl (S)-5-amino-4-(4-((4-((4-(4-cyano-3-(trifluoromethyl)phenyl)piperazin-1-yl)methyl)-2-fluorobenzyl)oxy)-1-oxoisoindolin-2-yl)-5-oxopentanoate, with CM-1L.

¹H NMR (400 MHz, DMSO-*d₆*) δ 10.96 (s, 1H), 7.70-7.57 (m, 2H), 7.49-7.48 (m, 3H), 7.38-7.34 (m, 3H), 7.14-7.09 (m, 1H), 5.29 (s, 2H), 5.13-5.09 (m, 1H), 4,41 (d, *J* = 17.6 Hz, 1H), 4.26 (d, *J* = 17.6 Hz, 1H), 3.57 (s, 2H), 3.20-3.18 (m, 4H), 2.95-2.86 (m, 1H), 2.59-2.39 (m, 6H), 2.00-1.95 (m, 1H).

MS: [M+H]⁺= 602.2.
e.e.= 99.8%

### Example 4

### Compound CM-2

### Synthetic route

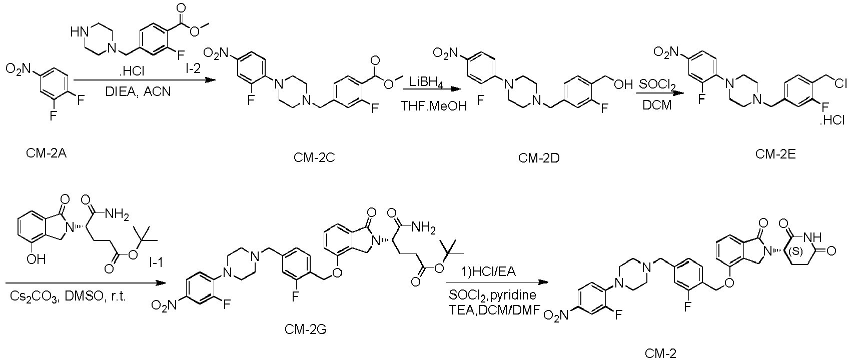

1,2-difluoro-4-nitrobenzene (1.50 g, 9.43 mmol), 1-2 (2.72 g, 9.43 mmol) and N,N,-diisopropylethylamine (4.26 g, 33.00 mmol) were added to acetonitrile (30 mL) and the resulting mixture was heated to 80°C in an oil bath and stirred for 16 hours. The reaction solution was concentrated under reduced pressure and the residue was purified by preparative high-performance liquid chromatography to obtain a product CM-2C as a brown solid (methyl 2-fluoro-4-((4-(2-fluoro-4-nitrophenyl)piperazin-1-yl)methyl)benzoate, 1.72 g, yield: 47%).

¹H NMR (400 MHz, DMSO-*d₆*) δ 8.02-7.98 (m, 2H), , 7.89-7.85 (m, 1H), 7.32-7.30 (m, 2H), 7.18-7.14 (m, 1H), 3.85 (s, 3H), 3.62 (s, 2H), 3.36-3.27 (m, 4H), 2.57-2.50 (m, 4H). MS (ESI) *m*/*z* 392.4 [M+H]⁺.

With reference to the synthetic method of compound CM-6 in example 9, compound CM-2 (i.e., (*S*)-3-(4-((2-fluoro-4-((4-(2-fluoro-4-nitrophenyl)piperazin-1-yl)methyl)benzyl)oxy)-1-oxoisoindolin-2-yl)piperidine-2,6-dione) can be synthesized by replacing compound CM-6C (i.e., methyl 4-((4-(4-cyano-3-(trifluoromethyl)phenyl)piperazin-1-yl)methyl)-2-fluorobenzoate) with a corresponding substrate CM-2C.

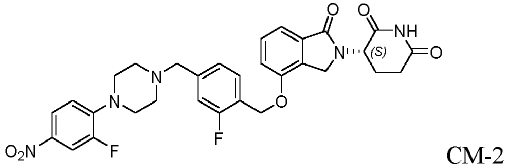

¹H NMR (400 MHz, DMSO-*d₆*) δ 10.95 (s, 1H), 8.06-7.96 (m, 2H), 7.61-7.48 (m, 2H), 7.42-7.31 (m, 2H), 7.28-7.11 (m, 3H), 5.27 (s, 2H), 5.13-5.08 (m, 1H), 4.38 (d, *J* = 17.6 Hz, 1H), 4.22 (d, *J* = 17.6 Hz, 1H), 3.58 (s, 2H), 3.23-3.14 (m, 4H), 2.96-2.83 (m, 1H), 2.60-2.52 (m, 5H), 2.48-2.37 (m, 1H), 1.99-1.96 (m, 1H).

MS (ESI) m/z 606.2 [M+H]⁺. e.e. = 99.4%

### Example 5

### Compound CM-5

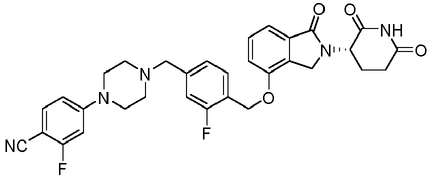

With reference to the synthetic method of compound CM-2 in example 4, compound CM-5 (i.e., (*S*)-4-(4-(4-(((2-(2,6-dioxopiperidin-3-yl)-1-oxoisoindolin-4-yl)oxy)methyl)-3-fluorobenzyl)piperazin-1-yl)-2-fluorobenzonitrile) can be synthesized by replacing the starting compound CM-2A with compound 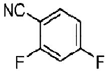 (CAS 3939-09-1).

¹H NMR (400 MHz, DMSO-*d₆*) δ 10.95 (s, 1H), 7.61-7.49 (m, 3H), 7.40-7.34 (m, 2H), 7.24-7.20 (m, 2H), 6.95-6.83 (m, 2H), 5.27 (s, 2H), 5.13-5.08 (m, 1H), 4.38 (d, *J*=17.6 Hz, 1H), 4.22 (d, *J*=17.6 Hz, 1H), 3.56 (s,2H), 3.45-3.36 (m, 4H), 2.96-2.83 (m, 1H), 2.61-2.53 (m, 1H), 2.48-2.40 (m, 5H), 1.99-1.96 (m,1H).

MS (ESI) m/z 586,2 [M+H]⁺.

### Example 6

### Compound CM-7

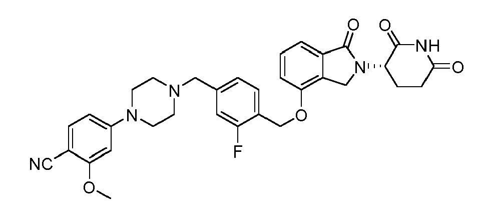

With reference to the synthetic method of compound CM-2 in example 4, compound CM-7 (i.e., (*S*)-4-(4-(4-(((2-(2,6-dioxopiperidin-3-yl)-1-oxoisoindolin-4-yl)oxy)methyl)-3-fluorobenzyl)piperazin-1-yl)-2-methoxybenzonitrile) can be synthesized by replacing the starting compound CM-2A with compound 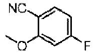 (CAS191014-55-8).

¹H NMR (400 MHz, DMSO-*d₆*) δ 10.95 (s, 1H), 7.58-7.49 (m, 2H), 7.43-7.34 (m, 3H), 7.24-7.21 (m, 2H), 6.60-6.52 (m, 2H), 5.28 (s, 2H), 5.13-5.08 (m, 1H), 4.38 (d, *J* = 17.6 Hz, 1H), 4.22 (d, *J* = 17.6 Hz, 1H), 3.86 (s, 3H), 3.37 (s, 2H), 3,40-3.34 (m, 4H), 2.96-2,85 (m, 1H), 2,60-2.51 (m, 5H), 2.47-2.37 (m, 1H), 1.99- 1.96(m, 1H).

MS (ESI) m/z 598.3 [M+H]⁺.

### Example 7

### Compound CM-3

### Synthetic route:

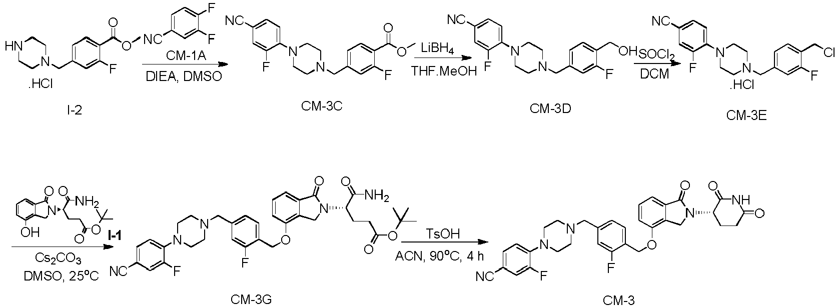

Step A. Compound 1-2 (methyl 2-fluoro-4-(piperazin-1-ylmethyl)benzoate hydrochloride, 2.70 g, 9.35 mmol), CM-1A (3,4-difluorobenzonitrile, 1.18 g, 8.50 mmol) and DIEA (3.29 g, 25.5 mmol) were added to DMSO (15 mL) and the resulting mixture was heated to 100°C in an oil bath and stirred for 3 hours. The reaction solution was poured into ice water and extracted with EtOAc (60 mL × 3), the organic phase was washed with saturated brine, dried and concentrated, and the residue was subjected to reversed phase preparative chromatography to obtain a product CM-3C as a yellow solid (methyl 4-((4-(4-cyano-2-fluorophenyl)piperazin-1-yl)methyl)-2-fluorobenzoate, 2.50 g, 6.74 mmol, yield: 79%). MS: [M+H]⁺=372.1.

Step B. Compound CM-3C (2.50 g, 6.74 mmol) was dissolved in THF/MeOH (30 mL/10 mL), LiBH₄ (0.51 g, 23.55 mmol) was added in portions at 0°C, and the resulting mixture was stirred under nitrogen protection at room temperature for 16 hours. The reaction solution was poured into ice water and the mixture was extracted with EtOAc (50 mL × 3). The organic phase was washed with saturated brine, dried and concentrated, and the residue was subjected to reversed phase preparative chromatography to obtain a product CM-3**D** as an off-white solid (3-fluoro-4-(4-(3-fluoro-4-(hydroxymethyl)benzyl)piperazin-1-yl)benzonitrile, 1.20 g, yield: 52%).

¹H NMR (400 MHz, CDCl₃) δ 7.39-7.34 (m, 2H), 7.28-7.24 (m, 1H), 7.13-7.08 (m, 2H), 6.92-6.88 (m, 1H), 4.75 (s, 2H), 3.56 (s, 2H), 3.49 (s, 1H), 3.24-3.22 (m, 4H), 2.63-2.60 (m, 4H). MS [M+H]⁺ = 344.4.

Step C. SOCl₂ (1.25 g, 10.50 mmol) was slowly added to a solution of compound CM-3**D** (1.20 g, 3.50 mmol) in DCM (15 mL) at 0°C, and the resulting mixture was stirred at room temperature for 3 hours. The reaction solution was concentrated to obtain a crude product, CM-3**E** (4-(4-(4-(chloromethyl)-3-fluorobenzyl)piperazin-1-yl)-3-fluorobenzonitrile hydrochloride, 1.4 g) as a white solid.

MS [M+H]⁺=362.1.

Step D. Compound I-1 (611 mg, 1.83 mmol), CM-3**E** (800 mg.2.01 mmol) and Cs₂CO₃ (1.49 g, 4.57 mmol) were added to DMSO (15 mL) and the resulting mixture was stirred at room temperature for 2 hours. The reaction solution was filtered, the filter cake was washed with EtOAc and the filtrate was poured into ice water. The mixture was extracted with EtOAc (50 mL × 3), the organic phase was washed with saturated brine (100 mL × 3), dried and concentrated, and the obtained crude was subjected to reversed phase preparative chromatography to obtain a product, CM-3**G** (tert-butyl (S)-5-amino-4-(4-((4-((4-(4-cyano-2-fluorophenyl)piperazin-1-yl)methyl)-2-fluorobenzyl)oxy)-1-oxoisoindolin-2-yl)-5-oxopentanoate, 600 mg, 0.91 mmol, yield: 50%) as an off-white solid.

¹H NMR (400 MHz, CDCl₃) δ 7.48-7.35 (m, 4H), 7.29-7.25 (m, 1H), 7.18-7.12 (m, 3H), 6.93-6.89 (m, 1H), 6.32 (m, 1H), 5.32 (m, 1H), 5.19 (s, 2H), 4.93-4.85 (m, 1H), 4.55-4.36 (m, 2H), 3.59 (s, 2H), 3,28-3.22 (m, 4H), 2.70-2.58 (m, 4H), 2.42-2.08 (m, 4H), 1.42 (s, 9H). MS [M+H]⁺ = 660.2.

Step E. A solution of compound CM-3**G** (600 mg, 0.91 mmol) and p-toluenesulfonic acid (469 mg, 2.73 mmol) in CH₃CN (15 mL) was stirred at 90°C for 4 hours. The reaction solution was concentrated and then diluted with EtOAc/THF (30 mL/30 mL), and aqueous NaHCO₃ solution was added at 0°C-5°C to pH>7. The organic phase was washed with saturated brine (80 mL), dried and concentrated, and the obtained crude was purified through Prep-HPLC to obtain a product, CM-3 ((*S*)-4-(4-(4-(((2-(2,6-dioxopiperidin-3-yl)-1-oxoisoindolin-4-yl)oxy)methyl)-3-fluorobenzyl)piperazin-1-yl) -3 -fluorobenzonitrile, 170 mg, 0.29 mmol, yield: 32%) as an off-white solid.

¹H NMR (400 MHz, DMSO-*d₆*) δ 10.95 (s, 1H), 7.71-7.65 (m, 1H), 7.60-7.47 (m, 3H), 7.42-7.31 (m, 2H), 7.25-7.08 (m, 3H), 5.27 (s, 2H), 5.15-5.05 (m, 1H), 4.38 (d, *J* = 17.6 Hz, 1H), 4.22 (d, *J* = 17.6 Hz, 1H), 3.57 (s, 2H), 3.23-3.14 (m, 4H), 2.96-2.83 (m, 1H), 2.60-2.52 (m, 5H), 2.48-2.37 (m, 1H), 2.02-1.92 (m, 1H).

MS (ESI) m/z 586.2 [M+H]⁺.

### Example 8

### Compound CM-4

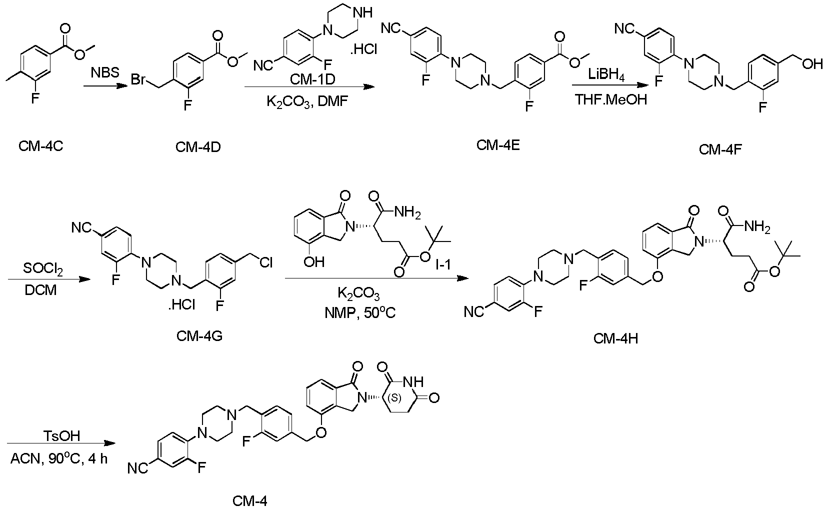

Step A. Methyl 3-fluoro-4-methylbenzoate (5 g, 29.8 mmol, CAS: 87808-48-8), NBS (5.56 g, 31.25 mmol) and BPO (1.44 g, 5.95 mmol) were added to CCl₄ (60 mL) and the resulting mixture was heated to 80°C in an oil bath and reacted overnight. The reaction solution was filtered, the filtrate was concentrated under reduced pressure, and the residue was purified by silica gel column chromatography (PE/EtOAc=100/1-30/1) to obtain compound CM-4**D** (methyl 4-(bromomethyl)-3-fluorobenzoate, 7 g, crude) as a yellow oil.

Step B. Compound CM-4**D** (2.23 g, 9.04 mmol), CM-ID (2.40 g, 9.94 mmol) and K₂CO₃ (3.12 g, 22.6 mmol) were added to DMF (25 mL) and the resulting mixture was stirred at 70°C for 16 hours. The reaction solution was filtered, the filtrate was concentrated under reduced pressure, and the residue was purified by preparative high-performance liquid chromatography to obtain a product, CM-4**E** (methyl 4-((4-(4-cyano-2-fluorophenyl)piperazin-1-yl)methyl)-3-fluorobenzoate, 2.4 g, yield: 71%) as an off-white solid. MS(ESI) *m*/*z* 372.4 [M+H]⁺.

Step C. Compound CM-4**E** (2.40 g, 6.47 mmol) was dissolved in THF/MeOH (20 mL/20 mL) and LiBH₄ (0.35 g, 16.17 mmol) was added in portions at 0°C. The resulting mixture was stirred at room temperature for 16 hours. The reaction solution was poured into ice water and the mixture was extracted with EtOAc (50 mL × 3). The organic phase was washed with saturated brine, dried and concentrated and the residue was purified by preparative high-performance liquid chromatography to obtain a product, CM-4F (3-fluoro-4-(4-(2-fluoro-4-(hydroxymethyl)benzyl)piperazin-1-yl) benzonitrile, 1.3 g, yield: 58%) as a white solid.

¹H NMR (400 MHz, DMSO-*d₆*) δ 7.70-7.66 (m, 1H), 7.57-7.54 (m, 1H), 7.38-7.34 (m, 1H), 7.13-7.08 (m, 3H), 5.27 (t, *J* = 6.4 Hz, 1H), 4.49 (d, *J* = 6.4 Hz, 2H), 3.57 (s, 2H), 3.18-3.16 (m, 4H), 2.54-2.49 (m, 4H). MS (ESI) *m*/*z* 344.1 [M+H]⁺.

Step D. SOC1₂ (1.35, 11.37 mmol) was slowly added to a solution of compound CM-4**F** (1.3 g, 3.79 mmol) in DCM (30 mL) at 0°C and the resulting mixture was stirred at room temperature for 3 hours. The reaction solution was concentrated under reduced pressure to obtain a product, CM-4**G** (4-(4-(4-(chloromethyl)-2-fluorobenzyl)piperazin-1-yl)-3-fluorobenzonitrile hydrochloride, 1.4 g) as an off-white solid.

MS (ESI) *m*/*z* 362.1, 364.1 [M+H]⁺.

Step E. A mixed solution of compound CM-4**G** (1.50 g, 3.77 mmol), compound **I-1** (1.15 g, 3.44 mmol) and K₂CO₃ (1.18 g, 8.57 mmol) in NMP (N-methylpyrrolidine, 15 mL) were stirred at 50°C for 16 hours. The reaction solution was filtered and the filtrate was poured into ice water. The mixture was extracted with EtOAc (50 mL × 3) and the organic phase was washed with saturated brine (100 mL), dried and concentrated under reduced pressure. The residue was purified through preparative high-performance liquid chromatography to obtain a product, CM-4**H** (tert-butyl (*S*)-5-amino-4-(4-((4-((4-(4-cyano-2-fluorophenyl)piperazin-1-yl)methyl)-3-fluorobenzyl)oxy)-1-oxoisoindolin-2-yl)-5-oxopentanoate, 1.80 g, yield: 79%) as an off-white solid.

¹H NMR (400 MHz, DMSO-*d₆*) δ 7.70-7.66 (m, 1H), 7.57-7.44 (m, 4H), 7.34-7.27 (m, 4H), 7.17-7.08 (m, 2H), 5.26 (s, 2H), 4.76-4.70 (m, 1H), 4.58-4.42 (m, 2H), 3.60 (s, 2H), 3.22-3.10 (m, 4H), 2.60-2.52 (m, 4H), 2.19-2,00 (m, 4H), 1,32 (s, 9H). MS (ESI) m/z 660.2 [M+H]⁺.

Step F. A solution of compound CM-4**H** (0.90 g, 1.36 mmol) and p-toluenesulfonic acid (0.705 g, 4.10 mmol) in CH₃CN (20 mL) was heated to 90°C in an oil bath and stirred for 4 hours. The reaction solution was concentrated under reduced pressure and then diluted with EtOAc/THF (30 mL/30 mL), and aqueous NaHCO₃ solution was added at 0°C-5°C to pH>7. The organic phase was washed with saturated brine (100 mL), dried and concentrated under reduced pressure and the residue was purified through preparative high-performance liquid chromatography to obtain a product, CM-4 ((*S*)-4-(4-(4-(((2-(2,6-dioxopiperidin-3-yl)-1-oxoisoindolin-4-yl)oxy)methyl)-2-fluorobenzyl)piperazin-1-yl)-3-fluorobenzonitrile, 415 mg, yield: 52%) as a white solid.

¹H NMR (400 MHz, DMSO-*d₆*) δ 10.97 (s, 1H), 7.70-7.66 (m, 1H), 7.7-7.44 (m, 3H), 7.35-7.31 (m, 4H), 7.13-7.08 (m, 1H), 5.26 (s, 2H), 5.14-5.09 (m, 1H), 4.45 (d, *J*= 17.2 Hz, 1H), 4.29 (d, *J* = 17.2 Hz, 1H), 3.60 (s, 2H), 3.24-3.11 (m, 4H), 2.98-2.84 (m, 1H), 2.64-2.52 (m, 5H), 2.49-2.37 (m, 1H), 2.05-1.93 (m, 1H).

MS (ESI) m/z 586.2 [M+H]⁺.

### Example 9

### Compound CM-6

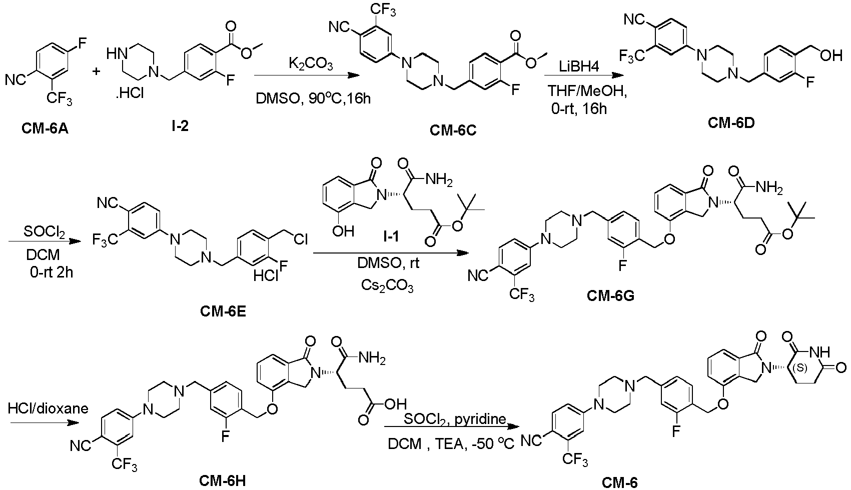

Step A. Compound CM-6A (4-fluoro-2-(trifluoromethyl)benzonitrile, 1.5 g, 7.93 mmol, CAS 194853-86-6), 1-2 (methyl 2-fluoro-4-(piperazin-1-ylmethyl)benzoate hydrochloride, 2.29 g, 7.93 mmol), and K₂CO₃ (2.74 g, 19.83 mmol) were added to DMSO (20 mL) and the resulting mixture was heated to 90°C in an oil bath and reacted for 7 h. The reaction solution was cooled down to room temperature, then filtered and washed with EtOAc. The filtrate was concentrated to dryness under reduced pressure and the residue was purified by silica gel column chromatography (PE : EtOAc=5 : 1) to obtain a product, CM-6C (methyl 4-((4-(4-cyano-3-(trifluoromethyl)phenyl)piperazin-1-yl)methyl)-2-fluorobenzoate, 3.0 g, yield 90%) as a yellow solid. MS (ESI) m/z 422.3 [M+H]⁺.

Step B. Compound CM-6**C** (3.0 g, 7.12 mmol) was dissolved in THF/MeOH (50 mL, 5 mL) and LiBH₄ (0.93 g, 42.8 mmol) was added in portions to the reaction flask in an ice bath. The resulting mixture was stirred at room temperature for 16 hours. The reaction was quenched by adding water in an ice bath. The reaction mixture was extracted with EtOAc (100 mL × 2), then washed with brine (100 mL), dried and concentrated under reduced pressure and the residue was purified by silica gel column chromatography (PE : EtOAc=5 1, 1 1, EtOAc) to obtain a product, CM-6**D** (4-(4-(3-fluoro-4-(hydroxymethyl)benzyl)piperazin-1-yl)-2-(trifluoromethyl)benzonitrile, 2.5 g, yield: 90%) as a white solid.

¹H NMR (400 MHz, DMSO-*d₆*) δ 7.82 (d, *J* = 8.8 Hz, 1H), 7.42-7.30 (m, 1H), 7.29-7.08 (m, 3H), 5.21 (t, *J=* 5.6 Hz, 1H), 4.53 (d, *J=* 5.6 Hz, 2H), 3.53 (s, 2H), 3.47-3.44 (m, 4H), 2.51-2.47 (m, 4H).

Step C. Compound CM-6**D** (2.40 g, 6.10 mmol) was dissolved in CH₃CN (15 mL) and SOC1₂ (2.18 g, 18.3 mmol) was added to the reaction flask in an ice bath and the resulting mixture was stirred at room temperature for 2 h. The reaction solution was concentrated to dryness under reduced pressure and the residue was slurried with EtOAc, then filtered and concentrated under reduced pressure to obtain a product, CM-6**E** (4-(4-(4-(chloromethyl)-3-fluorobenzyl)piperazin-1-yl)-2-(trifluoromethyl)benzonitrile hydrochloride, 2.4 g, yield: 88%) as an off-white solid.

¹H NMR (400 MHz, DMSO-*d₆*) δ 11.96 (s, 1H), 7.92 (d, *J* = 8.8 Hz, 1H), 7.67-7.62 (m, 2H), 7.49-7.40 (m, 2H), 7.31-7.28 (m, 1H), 4.82 (s, 2H), 4.39 (s, 2H), 4.23-4.20 (m, 2H), 3.55-3.49 (m, 2H), 3.36-3.33(m, 2H), 3.12-3.10 (m, 2H).

Step D. Compound CM-6E (1.70 g, 3.79 mmol) was dissolved in DMSO (10 mL) and Cs₂CO₃ (3.1 g, 9.5 mmol) and compound I-1 (1.27 g, 3.80 mmol) were added at room temperature. The resulting mixture was stirred at 25°C for 2 hours. The reaction solution was filtered through diatomite and then washed with EtOAc. The filtrate was poured into ice water and the mixture was extracted with EtOAc (50 mL × 3). The organic phase was washed with saturated brine (100 mL × 3), dried and concentrated under reduced pressure and the obtained crude was purified through preparative high-performance liquid chromatography to obtain a product, CM-6**G** (tert-butyl (S)-5-amino-4-(4-((4-((4-(4-cyano-3-(trifluoromethyl)phenyl)piperazin-1-yl)methyl)-2-fluorobcnzyl)oxy)-1-oxoisoindolin-2-yl)-5-oxopentanoate, 2.0 g, yield: 74%) as a yellow solid. MS(ESI) m/z 710.3 [M+H]⁺.

Step E. Compound CM-6**G** (1.05 g, 1.48 mmol) was dissolved in DMF (3 mL) and 4 N HCl/dioxane (30 mL) was added. The reaction solution was stirred at room temperature for 16 hours and concentrated under reduced pressure to obtain CM-6**H** ((*S*)-5-amino-4-(4-((4-((4-(4-cyano-3-(trifluoromethyl)phenyl)piperazin-1-yl)methyl)-2-fluorobenzyl)oxy)-1-oxoisoindolin-2-yl)-5-oxopentanoic acid, 1.0 g) as a yellow oil. MS(ESI) m/z 654.6 [M+H]⁺.

Step F. Compound CM-6**H** (1.2 g, crude) was dissolved in DMF/DCM (8 mL/25 mL) and the resulting mixture was cooled down to -40°C. SOCl₂ (881 mg, 7.40 mmol) was added dropwise and after the resulting mixture was reacted for 1.5 h, pyridine (1.17 g, 14.8 mmol) was added. After the mixture was stirred at -40°C for 1 hour, Et₃N (747 mg, 7.40 mmol) was slowly added dropwise and the resulting mixture was stirred for 1 hour. The reaction was quenched by slowly adding 10 mL of water and maintained at a temperature below -40°C. The reaction mixture was adjusted to pH=8 with aqueous NaHCO₃ solution at -20°C, then extracted with EtOAc/THF (60/30 mL, × 3), dried over Na₂SO₄ and concentrated to dryness under reduced pressure and the residue was purified by preparative HPLC to obtain a product, CM-6 ((*S*)-4-(4-(4-(((2-(2,6-dioxopiperidin-3-yl)-1-oxoisoindolin-4-yl)oxy)methyl)-3-fluorobenzyl)piperazin-1-yl)-2-(trifluoromethyl)benzonitrile, 202 mg, two-step yield: 21%) as a white solid.

¹H NMR (400 MHz, DMSO-*d₆*) δ 10.95 (s, 1H), 7.84-7.82 (m, 1H), 7.57-7.51 (m, 2H), 7.40-7.20 (m, 6H), 5.28 (s, 2H), 5.13-5.08 (m, 1H), 4.39 (d, *J=* 17.6 Hz, 1H), 4,23 (d, *J=* 17.6 Hz, 1H), 3.57-3.45 (m, 6H), 2.95-2.86 (m, 1H), 2.59-2.42 (m, 6H), 1.99-1.95 (m, 1H).

MS (ESI) m/z 636.3 [M+H]⁺.

### Example 10

With reference to the synthetic method for compound CM-6 in example 9, compounds CM-8, CM-9, CM-11, CM-27, CM-28, CM-29, CM-30, CM-31 and CM-35 were prepared by using corresponding reactants.

### Compound CM-8

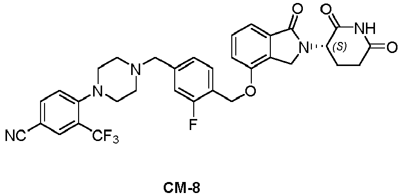

¹H NMR (400 MHz, DMSO-*d₆*) δ 10.95 (s, 1H), 8.16 (d, *J=* 2.0 Hz, 1H), 8.07-8.05 (m, 1H), 7.58-7.49 (m, 3H), 7.40-7.34 (m, 2H), 7.24-7.20 (m, 2H), 5.27 (s, 2H), 5.12-5.08 (m, 1H), 4.38 (d, *J=* 17.6 Hz, 1H), 4.22 (d, *J* = 17.6 Hz, 1H), 3.57 (s, 2H), 3.09-2.97 (m, 4H), 2.96-2.83 (m, 1H), 2.61-2.42 (m, 6H), 2.04-1.90 (m, 1H).

MS (ESI) m/z 636.2 [M+H]⁺.

### Compound CM-9

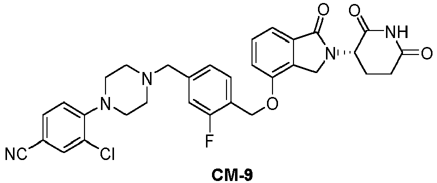

¹HNMR (400 MHz, DMSO-*d₆*) δ 10.95 (s, 1H), 7.98-7.93 (m, 1H), 7.76-7.73 (m, 1H), 7.58-7.49 (m, 2H), 7.40-7.34 (m, 2H), 7.25-7.20 (m, 3H), 5.27 (s, 2H), 5.13-5.08 (m, 1H), 4.38 (d, *J*= 17.6 Hz, 1H), 4.23 (d, *J=* 17.6 Hz, 1H), 3.59 (s, 2H), 3.12 (br s, 4H), 2.95-2.86 (m, 1H), 2.56-2.38 (m, 6H), 2.00-1.95 (m, 1H).

MS (ESI)m/z 602.2 [M+H]⁺.

### Compound CM-11

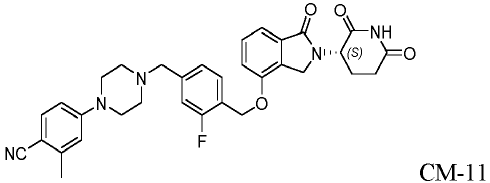

¹H NMR (400 MHz, DMSO-*d₆*) δ 10.95 (s, 1H), 7.58-7.48 (m, 3H), 7.40-7.34 (m, 2H), 7.24-7.20 (m, 2H), 6.92 (s, 1H), 6.84-6.82 (m, 1H), 5.27 (s, 2H), 5.13-5.08 (m, 1H), 4.38 (d, *J=* 18.0 Hz, 1H), 4.23 (d, *J* = 18.0 Hz, 1H), 3.56 (s, 2H), 3.34-3.29 (m, 4H), 2.90-2.87 (m, 1H), 2.59-2.54 (m, 1H), 2.51-2.42 (m, 5H), 2.07 (s, 3H), 2.01-1.95 (m, 1H).

MS (ESI)m/z 582.3 [M+H]⁺.

### Compound CM-27

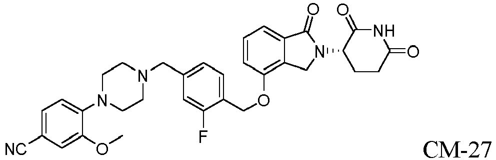

¹H NMR (400 MHz, DMSO-*d₆*) δ 10.96 (s, 1H), 7.59-7.48 (m, 2H), 7.42-7.31 (m, 4H), 7.24-7.19 (m, 2H), 6.96 (d, *J* = 8.0 Hz, 1H), 5.27 (s, 2H), 5.14-5.07 (m, 1H), 4.38 (d, *J*= 17.6 Hz, 1H), 4.22 (d, *J=* 17.6 Hz, 1H), 3.82 (s, 3H), 3.56 (s, 2H), 3.18-3.01 (m, 4H), 2.96-2.82 (m, 1H), 2.61-2.52 (m, 5H), 2.47-2.38 (m, 1H), 2.02-1.90 (m, 1H).

MS (ESI) m/z 598.2 [M+H]⁺.

### Compound CM-28

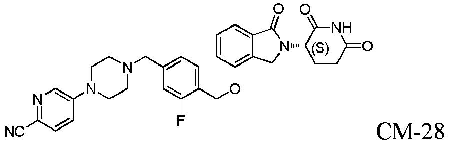

¹H NMR (400 MHz, DMSO-*d₆*) δ 10.96 (s, 1H), 8.41 (d, *J=* 2.4 Hz, 1H), 7.75 (d, *J* = 8.8 Hz, 1H), 7.60- 7.48 (m, 2H), 7.42-7.32 (m, 3H), 7.26-7.19 (m, 2H), 5.27 (s, 2H), 5.14-5.07 (m, 1H), 4.38 (d, *J=* 17.6 Hz, 1H), 4.22 (d, *J=* 17.2 Hz, 1H), 3.57 (s, 2H), 3.46-3.37 (m, 4H), 2.97-2.83 (m, 1H), 2.61-2.53 (m, 1H), 2.49-2.37 (m,5H), 2.02-1.91 (m, 1H).

MS (ESI) m/z 569.2 [M+H]⁺.

### Compound CM-29

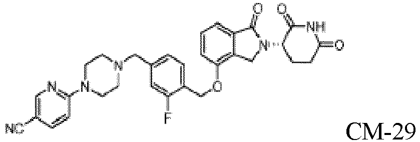

¹H NMR (400 MHz, DMSO-*d₆*) δ 10.97 (s, 1H), 8.47 (s, 1H), 7.86-7.83 (m, 1H), 7.58-7.50 (m, 2H), 7.41- 7.34 (m, 2H), 7.24-7.20 (m, 2H), 6.93-6.91(m, 1H), 5.27 (s, 2H), 5.13-5.08 (m, 1H), 4.41 (d, *J=* 17.6 Hz, 1H), 4.25 (d, *J*=17.6 Hz, 1H), 3.68- 3.66 (m, 4H), 3.55 (s, 2H), 3.12 (m, 4H), 2.95-2.86 (m, 1H), 2.59-2.51 (m, 1H), 2.50-2.42 (m, 5H), 2.00-1.95 (m, 1H).

MS (ESI)m/z 569.2 [M+H]⁺.

### Compound CM-30

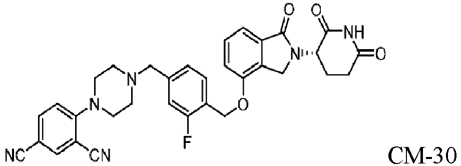

¹H NMR(400 MHz, DMSO-*d₆*) δ 10.96 (s, 1H), 8.25 (s, 1H), 7.97-7.91 (m, 1H), 7.60-7.48 (m, 2H), 7.42-7.32 (m, 2H), 7.26-7.20 (m, 3H), 5.27 (s, 2H), 5.15-5.06 (m, 1H), 4.38 (d, *J=* 17.6 Hz, 1H), 4.22 (d, *J=* 17.6 Hz, 1H), 3.59 (s, 2H), 3.43-3.36 (m, 4H), 2.96-2.83 (m, 1H), 2.61-2.52 (m, 5H), 2.47-2.36 (m, 1H), 2.00-1.91 (m, 1H).

MS (ESI) m/z 593.2 [M+H]⁺.

### Compound CM-31

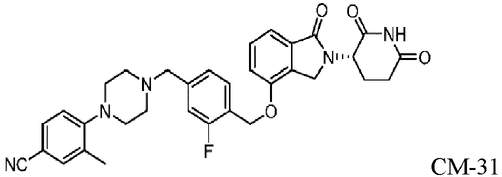

¹H NMR(400 MHz, DMSO-*d₆*) δ 10.97 (s, 1H), 7.60-7.50 (m, 4H), 7.41-7.34(m, 2H), 7.24-7.21 (m, 2H), 7.11-7.08 (m, 1H), 5.27 (s, 2H), 5.13-5.09 (m, 1H), 4.41 (d, *J* = 17.6 Hz, 1H), 4.25 (d, *J* = 17.6 Hz, 1H), 3.58 (s, 2H), 2.95-2.86 (m, 5H), 2.58-2.54 (m, 5H), 2.46-2.42 (m, 1H), 2.25 (s, 3H), 1.99-1.95 (m, 1H).

MS (ESI)m/z 582.2 [M+H]⁺.

### Compound CM-35

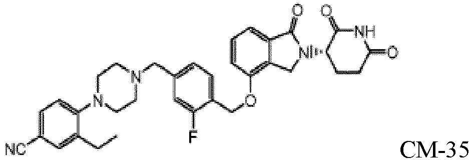

1H NMR (400 MHz, DMSO-*d₆*) δ 10.97 (s, 1H), 7.64-7.50 (m, 4H), 7.41-7.15 (m, 5H), 5.27 (s, 2H), 5.28 (s, 2H), 5.13-5.09 (m, 1H), 4.41 (d, *J=* 17.6 Hz, 1H), 4.20 (d, *J=* 17.6 Hz, 1H), 3.58 (s, 2H), 2.92 (m, 5H), 2.55-2.50 (m, 8H), 1.98-1.96 (m, 1H), 1.22 (t, *J=* 7.2 Hz, 3H).

MS (ESI) m/z 596.2 [M+H]⁺.

### Example 11

### Compound CM-12

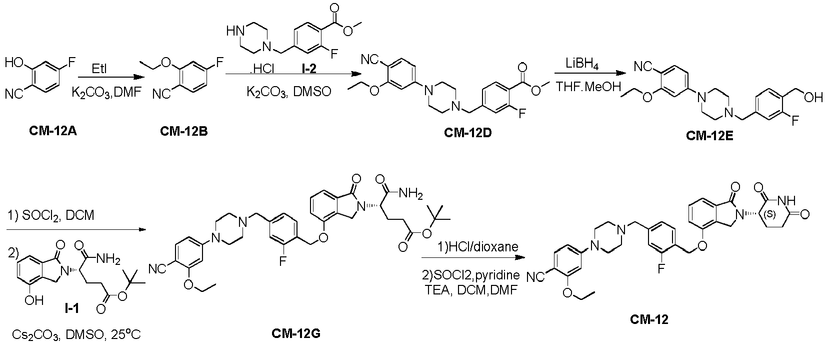

Step **A**: Iodoethane (2.56 g, 16.41 mmol) was added to a mixed solution of compound CM-12A (2-hydroxy-4-fluorobenzonitrile, 1.50 g, 10.94 mmol) and K₂CO₃ (3.02 g, 21.88 mmol) in DMF (20 mL) and the resulting mixture was reacted at room temperature for 16 hours. Water (100 mL) was added and the mixture was extracted with ethyl acetate (100 mL × 2). The organic phase was washed with saturated brine, then dried over anhydrous sodium sulfate, and concentrated to obtain a product, CM-12**B** (2-ethoxy-4-fluorobenzonitrile, 1.60 g, 88% yield) as an off-white solid.

Step B: K₂CO₃ (2.67 g, 19.38 mmol) was added to a solution of compound CM-12**B** (2-ethoxy-4-fluorobenzonitrile, 1.60 g, 9.69 mmol) and **I-2** (methyl 2-fluoro-4-(piperazin-1-ylmethyl)benzoate hydrochloride, 3.15 g, 10.43 mmol) in DMSO (20 mL), and the mixed solution was stirred at 90°C for 16 hours. The reaction solution was poured into water (60 mL) and the mixture was extracted with EtOAc (50 mL × 2). The organic phase was washed with saturated brine (60 mL × 3), then dried over anhydrous Na₂SO₄, filtered and concentrated. The residue was subjected to reversed phase preparative chromatography to obtain a product, CM-12**D** (methyl 4-((4-(4-cyano-3-ethoxyphenyl)piperazin-1-yl)methyl)-2-fluorobenzoate, 1.90 g, yield: 49%) as a yellow solid.

Step C: Compound CM-12**D** (1.90 g, 4.78 mmol) was dissolved in a mixed solution of THF (40 mL) and MeOH (10 mL) and LiBH₄ (0.32 g, 14.36 mmol) was added in portions at 0°C. The reaction solution was stirred at room temperature for 16 hours. The reaction solution was slowly poured into an ice aqueous ammonium chloride solution and the mixture was extracted with ethyl acetate (40 mL × 3). The organic phase was washed with saturated brine (80 mL) and then extracted. The resulting organic phase was washed with saturated brine (80 mL), then dried over anhydrous Na₂SO₄, filtered and concentrated to obtain crude product, and the crude product was subjected to reversed phase preparative chromatography to obtain a product, CM-12**E** (2-ethoxy-4-(4-(3-fluoro-4-(hydroxymethyl)benzyl)piperazin-1-yl)benzonitrile, 1.20 g, yield: 68%) as a white solid.

Step D: SOCl₂ (1.46 g, 12.24 mmol) was slowly added to a solution of compound CM-12**E** (1.20 g, 3.25 mmol) in DCM (30 mL) at 0°C and the resulting mixture was stirred at room temperature for 16 hours. The reaction solution was concentrated to obtain a brown solid (1.38 g, crude). The above-mentioned brown solid (1.38 g, 3.25 mmol), **I-1** (tert-butyl (*S*)-5-amino-4-(4-hydroxy-1-oxoisoindolin-2-yl)-5-oxopentanoate, 1.08 g, 3.25 mmol) and Cs₂CO₃ (3.18 g, 9.75 mmol) were added to DMSO (20 mL) at room temperature and the resulting mixture was stirred at room temperature for 2 hours. The reaction solution was poured into ice water and the mixture was extracted with ethyl acetate (50 mL × 2). The organic phase was washed with saturated brine (80 mL × 3), then dried over anhydrous Na₂SO₄ and filtered, and the filtrate was concentrated. The residue was subjected to reversed phase preparative chromatography to obtain a product, CM-12**G** (tert-butyl(*S*)-5-amino-4-(4-((4-((4-(4-cyano-3-ethoxyphenyl)piperazin-1-yl)methyl)-2-fluorobenzyl)oxy)-1-oxoisoindolin-2-yl)-5-oxopentanoate, 1.50 g, yield: 67%) as a yellow solid.

¹H NMR (400 MHz, DMSO-*d₆*) δ 7.57-7.31 (m, 6H), 7.25-7.16 (m, 3H), 6.57-6.54 (m, 2H), 5.27 (s, 2H), 4.72-4.69 (m, 1H), 4.51 (d, *J* = 17.6 Hz, 1H), 4.37 (d, *J*= 17.6 Hz, 1H), 4.18-4.10 (m, 2H), 3.57 (s, 2H), 3.40-3.32(m, 4H), 2.49-2.43 (m, 4H), 2.19-2.00 (m, 4H), 1.31 (s, 9H).

MS (ESI) *m*/*z* 686.2 [M+H]⁺.

Step F: A solution of HCl in dioxane (4 N, 30 mL) was added to a solution of CM-12**G** (1.50 g, 2.19 mmol) in dioxane (20 mL) at room temperature. The reaction solution was stirred at 30°C for 2 h. The reaction solution was concentrated to obtain an intermediate. SOCl₂ (1.30 g, 10.94 mmol) was added to a solution of the above-mentioned intermediate in DCM/DMF (70 mL/10 mL) at -45°C under nitrogen. The reaction mixture was stirred at -45°C for 1 hour. Pyridine (1.73 g, 21.90 mmol) was slowly added, and the reactants were stirred at -45°C for 1 hour. Triethylamine (1.10 g, 10.94 mmol) was slowly added to the reaction solution at -45°C and the reactants were stirred at -45°C for 1 hour. The reaction solution was quenched with water (10 mL) and then adjusted to pH>7 by adding aqueous NaHCO₃ solution, and the organic phase was separated. The aqueous phase was extracted with EA/THF (30 mL × 3, V/V=1/1). The organic phases were combined and washed with saturated NaCl solution, then dried and concentrated to obtain a crude product, and the crude product was subjected to Prep-HPLC to obtain **CM-12** as an off-white solid (383 mg, yield: 29%).

¹H NMR (400 MHz, DMSO-*d₆*) δ 10.97 (s, 1H), 7.59-7.50 (m, 2H), 7.43-7.34 (m, 3H), 7.25-7.20 (m, 2H), 6.56-6.54 (m, 2H), 5.27 (s, 2H), 5.13-5.09 (m, 1H), 4.38 (d, *J*= 17.6 Hz, 1H), 4.24-4.11 (m, 3H), 3.56 (s, 2H), 3.35-3.33 (m, 4H), 2.90-2.87 (m, 1H), 2.58-2.53 (m, 1H), 2.49-2.42 (m, 5H), 2.99-1.96 (m, 1H), 1.34 (t. *J*= 7.2 Hz, 3H).

MS (ESI) m/z 612.3 [M+H]⁺.

### Example 12

With reference to the synthetic method of compound CM-12 in example 11, CM-10, CM-24, CM-33, CM-34 and CM-39 were synthesized by using corresponding reactants.

### Compound CM-10

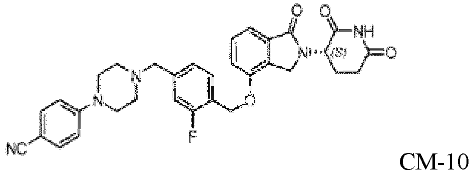

¹H NMR (400 MHz, DMSO-*d₆*) δ 10.96 (s, 1H), 7.58-7.50 (m, 4H), 7.40-7.34 (m, 2H), 7.24-7.20 (m, 2H), 7.01-6.99 (m, 2H), 5.27 (s, 2H), 5.13-5.08 (m, 1H), 4.41 (d, *J=* 17.6 Hz, 1H), 4.25 (d, *J* = 17.6 Hz, 1H), 3.56 (s, 2H), 3.35-3.33 (m, 4H), 2.95-2.86 (m, 1H), 2.59-2.51 (m, 1H), 2.50-2.38 (m, 5H), 2.00-1.96 (m, 1H).

MS (ESI) m/z 568.3 [M+H]⁺.

### Compound CM-24

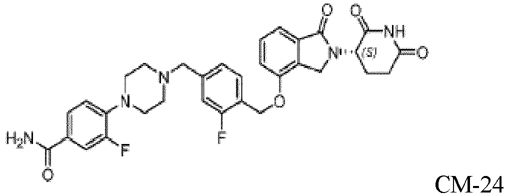

¹H NMR(400 MHz, DMSO-*d₆*) δ 10.96 (s, 1H), 7.86 (s, 1H), 7.65-7.41 (m, 4H), 7.34-7.21 (m, 5H), 7.06- 7.02 (m, 1H), 5.27 (s, 2H), 5.13-5.08 (m, 1H), 4.39 (d, *J* =17.6 Hz, 1H), 4.22 (d, *J=* 17.6 Hz, 1H), 3.57 (s, 2H), 3.16-3.02 (m, 4H), 2.95-2.86 (m, 1H), 2.64-2.52 (m, 5H), 2.48-2.39 (m, 1H), 1.99-1.95 (m, 1H).

MS (ESI)m/z 604.3 [M+H]⁺.

### CM-33

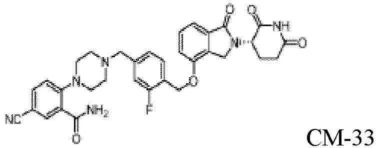

¹H NMR(400 MHz, DMSO-d₆) δ 10.96 (s, 1H), 7.90 (s, 1H), 7.78-7.70 (m, 2H), 7.66-7.46 (m, 3H), 7.44-7.32 (m, 2H), 7.27-7.09 (m, 3H), 5.26 (s, 2H), 5.15-5.06 (m, 1H), 4.38 (d, *J*= 17.6 Hz, 1H), 4.22 (d, *J=* 17.6Hz, 1H), 3.56 (s, 2H), 3.17-3.05 (m, 4H), 2.97-2.84 (m, 1H), 2.62-2.54 (m, 1H), 2.48-2.37 (m, 5H), 2.00-1.91 (m, 1H).

MS (ESI) m/z 611.2 [M+H]⁺.

### CM-34

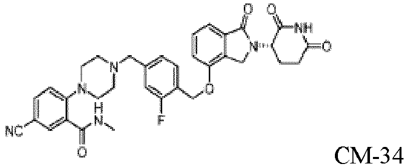

¹H NMR (400 MHz, DMSO-*d₆*) δ 10.96 (s, 1H), 8.39-8.34 (m, 1H), 7.78-7.66 (m, 2H), 7.60-7.47 (m, 2H), 7.43-7.31 (m, 2H), 7.27-7.09 (m, 3H), 5.27 (s, 2H), 5.16-5.05 (m, 1H), 4.38 (d, *J=* 17.6 Hz, 1H), 4.22 (d, *J=* 17.6 Hz, 1H), 3.56 (s, 2H), 3.16-3.00 (m, 4H), 2.97-2.83 (m, 1H), 2.80-2.70 (m, 3H), 2.60-2.53 (m, 1H), 2.49-2.37 (m, 5H), 2.02-1.91 (m, 1H).

MS (ESI) m/z 625.2 [M+H]⁺.

### Compound CM-39

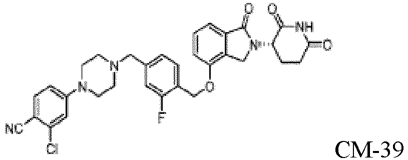

¹H NMR (400 MHz, DMSO-*d*₆) δ 10.96 (s, 1H), 7.66-7.59 (m, 3H), 7.58-7.50 (m, 2H), 7.40-7.14 (m, 3H), 6.98-6.96 (m, 1H), 5.28 (s, 2H), 5.13-5.08 (m, 1H), 4.41 (d, *J=* 17.6 Hz, 1H), 4.25 (d, *J*= 17.6 Hz, 1H), 3.56 (s, 2H), 3.39- 3.32 (m, 4H), 2.95-2.86 (m, 1H), 2.59-2.54 (m, 1H), 2.50-2.42 (m, 5H), 1.99-1.96 (m, 1H).

MS (ESI)m/z 602.2 [M+H]⁺.

### Example 13

### Compound CM-18

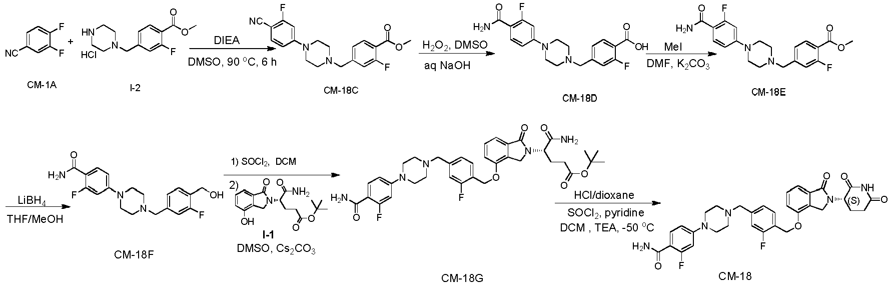

Step A: With reference to the synthesis conditions in example 11, CM-18**C** (i.e., methyl 4-((4-(4-cyano-3-fluorophenyl)piperazin-l-yl)methyl)-2-fluorobenzoate) was obtained as a yellow solid (3.0 g, yield: 45%) from CM-1**A** (3,4-difluorobenzonitrile, 2.5 g, 18.0 mmol, CAS3939-09-1). MS(ESI) m/z 372.2 [M+H]⁺.

Step B: Compound CM-18**C** (3.0 g, 8.08 mmol) was added to a reaction flask and dissolved in DMSO/EtOH (60 mL/160 mL) and then aqueous NaOH solution (1 N, 100 mL, 100.0 mmol) and 30% H₂O₂ (30 mL) were added and stirred at 35°C for 16 hours. The reaction solution was poured into water (500 mL) and the mixture was acidified to pH=1 with concentrated hydrochloric acid, then left to stand to precipitate a white solid and filtered. The filter cake was concentrated to dryness under reduced pressure using an oil pump to obtain a product, CM-18D (4-((4-(4-carbamoyl-3-fluorophenyl)piperazin-1-yl)methyl)-2-fluorobenzoic acid, 3.0 g) as a white solid. MS (ESI) m/z 376.2 [M+H]⁺.

Step C: Compound CM-18D (3.0 g, crude) was added to a reaction flask and dissolved in DMF (40 mL), K₂CO₃ (2.21 g, 16.0 mmol) and MeI (1.13 g, 8.00 mmol) were added, and the mixture was stirred at room temperature overnight. Water (300 mL) was added and the resulting mixture was extracted with EtOAc (150 mL × 2). The organic phases were combined, then washed with brine (300 mL), dried over Na₂SO₄, filtered and concentrated to obtain a product, CM-18**E** (methyl 4-((4-(4-carbamoyl-3-fluorophenyl)piperazin-1-yl)methyl)-2-fluorobenzoate, 1.80 g, 2-step yield: 57%). MS (ESI) m/z 390.2 [M+H]⁺.

Step D: With reference to the synthetic method in example 11, compound CM-18 (i.e., (S)-4-(4-(4-(((2-(2,6-dioxopiperidin-3-yl)-1-oxoisoindolin-4-yl)oxy)methyl)-3-fluorobenzyl)piperazin-1-yl)-2-fluorobenzamide) was obtained from compound CM-18**E**.

¹H NMR (400 MHz, DMSO-*d*₆) δ 10.95 (s, 1H), 7.63-7.50 (m, 3H), 7.41-7.31 (m, 3H), 7.24-7.18 (m, 3H), 6.79-6.69 (m, 2H), 5.27 (s, 2H), 5.13-5.08 (m, 1H), 4.39 (d, *J*= 17.6 Hz, 1H), 4.23 (d, *J=* 17.6 Hz, 1H), 3.56 (s, 2H), 3.30-3.28 (m, 4H), 2.95-2.86 (m, 1H), 2.54-2.32 (m, 6H), 1.99-1.95 (m, 1H).

MS (ESI) m/z 603.8 [M+H]⁺.

### Example 14

### Compound CM-19

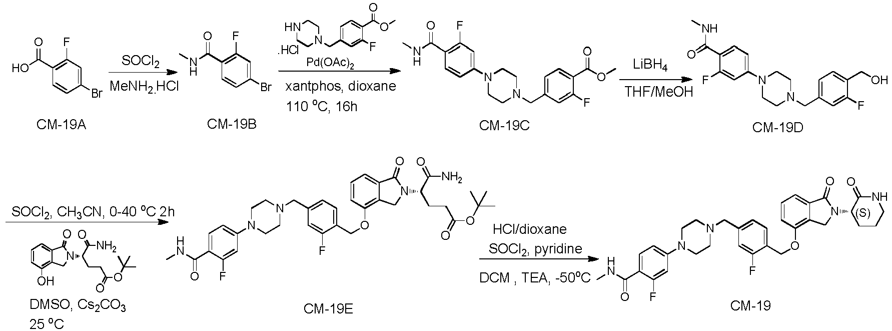

Step A: SOCl₂ (27.2 g, 16.5 mL, 0.228 mol) was added to compound CM-19A (2-fluoro-4-bromobenzoic acid, 10.0 g, 45.70 mmol, CAS 112704-79-7) at 0°C and the mixture was refluxed at 85°C for 2 h. Excess SOCl₂ was evaporated off to obtain a compound as a brown solid. Then the compound was dissolved in DCM (50 mL) and a mixture of MeNH₂.HCl (9.25 g, 0.14 mol) and Et₃N/DCM (25 mL/100 mL) was added at 0°C. The resulting mixture was reacted at room temperature for 2 h, water (100 mL) was added, a solid-liquid separation process was carried out, and the aqueous phase was extracted with DCM (100 mL), then washed with brine (200 mL), dried over Na₂SO₄, filtered and concentrated to obtain a product, CM-19**B** (4-bromo-2-fluoro-N-methylbenzamide, 10.0 g, yield: 94%). MS (ESI) m/z 233.9 [M+1]⁺.

Step B: Compounds CM-19**B** (6.00 g, 25.86 mmol) and **I-2** (methyl 2-fluoro-4-(piperazin-1-ylmethyl)benzoate hydrochloride, 6.51 g, 25.80 mmol), Pd(OAc)₂ (580 mg, 2.59 mmol), Xantphos (3.00 g, 5.18 mmol, CAS 161265-03-8) and Cs₂CO₃ (16.8 g, 51.80 mmol) were added to dioxane (100 mL) and the mixture was heated to 110°C in an oil bath and reacted for 16 h under N₂ protection. The reaction mixture was poured into water (100 mL) and then the resulting mixture was extracted with EtOAc (100 mL × 2). The organic phase was washed with saturated brine (200 mL), then dried over Na₂SO₄, filtered and concentrated to dryness under reduced pressure and the residue was purified by silica gel column chromatography (EA/PE 10%-50%) to obtain a product, CM-19**C** (methyl 2-fluoro-4-((4-(3-fluoro-4-(methylcarbamoyl)phenyl)piperazin-1-yl)methyl)benzoate, 3.70 g, yield: 35%) as a light yellow solid. MS (ESI) m/z 404.1 [M+1]⁺.

With regard to the remaining steps, reference can be made to the synthesis conditions in example 11 and compound CM-19 was obtained by using corresponding reactants.

¹H NMR (400 MHz, DMSO-*d*₆) δ 10.96 (s, 1H), 7.76-7.74 (m, 1H), 7.58-7.50 (m, 3H), 7.41-7.34 (m, 2H), 7.24-7.20 (m, 2H), 6.78-6.71 (m, 2H), 5.27 (s, 2H), 5.13-5.09 (m, 1H), 4.39 (d, *J=* 17.6 Hz, 1H), 4.23 (d, *J=* 17.6 Hz, 1H), 3.56 (s, 2H), 3.32-3.28 (m, 4H), 2.95-2.86 (m, 1H), 2.75-2.74 (m, 3H), 2.58-2.50 (m, 1H), 2.50-2.42 (m, 5H), 1.98-1.95 (m, 1H).

MS (ESI) m/z 618.3 [M+H]⁺.

### Example 15

### Compound CM-17

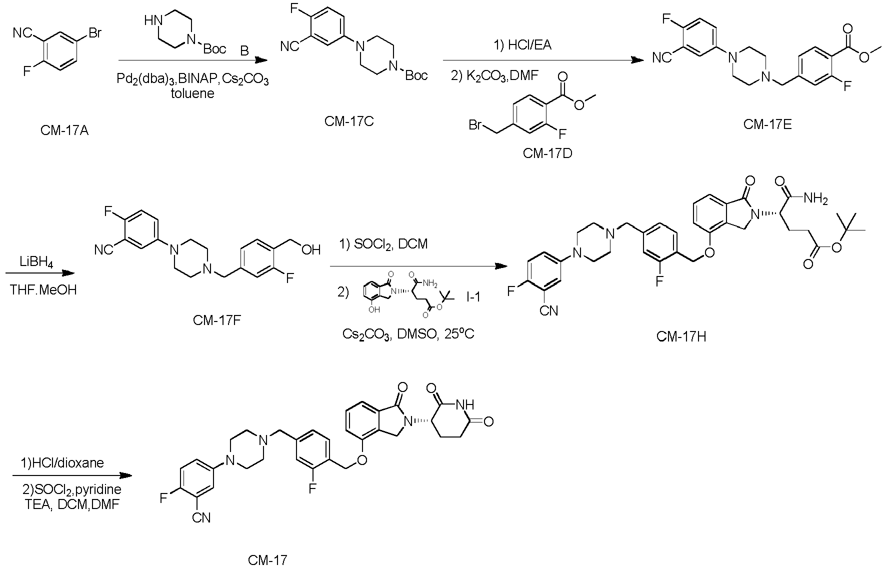

Step A: Raw materials CM-17**A** (2-fluoro-5-bromobenzonitrile, 5.00 g, 25.00 mmol), *N*-Boc piperazine (3.88 g, 20.80 mmol), Pd₂(dba)₃ (0.95 g, 1.04 mmol), BINAP (1.30 g, 2.08 mmol) and Cs₂CO₃ (10.17 g, 31.20 mmol) were added to toluene (50 ml) and the resulting mixture was reacted at 80°C for 16 hours under nitrogen protection. The reaction mixture was cooled down to room temperature, filtered through diatomite and then washed with ethyl acetate, the filtrate was concentrated, and the residue was subjected to column chromatography (PE/EA=10/1 to 5/1) to obtain a product, CM-17**C** (tert-butyl 4-(3-cyano-4-fluorophenyl)piperazine-1-carboxylate, 2.50 g, yield: 39%) as an off-white solid.

Step B: CM-17C (2.50 g, 8.19 mmol) was dissolved in ethyl acetate (20 mL), hydrogen chloride/ethyl acetate solution (4 M, 30 mL) was slowly added, and the mixture was stirred at room temperature for 3 hours and then concentrated to obtain an intermediate (1.98 g, crude). The intermediate (1.98 g, 8.16 mmol), compound CM-17D (methyl 4-(bromomethyl)-2-fluorobenzoate, 2.62 g, 10.61 mmol) and K₂CO₃ (3.37 g, 24.48 mmol) were added to DMF (20 mL) and the mixture was stirred at 55°C for 5 hours. The reaction solution was washed with ethyl acetate, the filtrate was concentrated, and the residue was subjected to reversed phase preparative chromatography to obtain a product, CM-17E (methyl 4-((4-(3-cyano-4-fluorophenyl)pipcrazin-1-yl)methyl)-2-fluorobenzoate, 1.90 g, yield: 62%) as a yellow solid. MS (ESI) m/z 372.1 [M+H]⁺.

With regard to the remaining steps, reference can be made to the synthesis conditions in example 11 and compound CM-17 was obtained by using corresponding reactants.

¹H NMR (400 MHz, DMSO-*d*₆) δ 10.95 (s, 1H), 7.58-7.49 (m, 2H), 7.40-7.33 (m, 5H), 7.24-7.20 (m, 2H), 5.27 (s, 2H), 5.13-5.08 (m, 1H), 4.38 (d, *J=* 17.6 Hz, 1H), 4.22 (d, *J=* 17.6 Hz, 1H), 3.56 (s, 2H), 3.22- 3.11 (m, 4H), 2.98-2.83 (m, 1H), 2.61-2.52 (m, 5H), 2.48-2.37 (m, 1H), 2.03-1.92 (m, 1H).

MS (ESI) m/z 586.2 [M+H]⁺.

### Example 16

### Compound CM-14

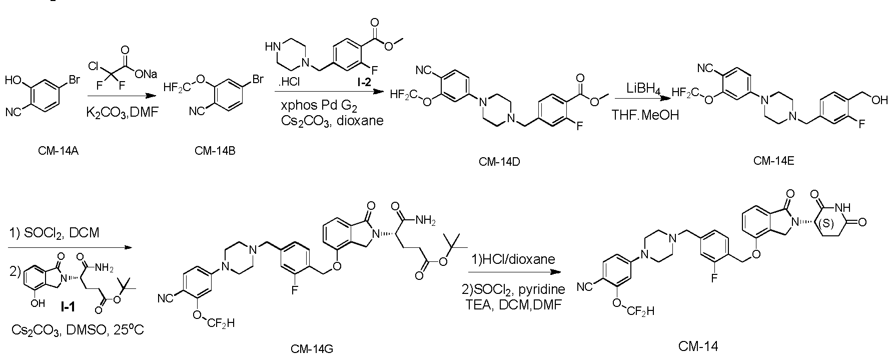

Step A: Sodium difluorochloroacetate (7.70 g, 50.50 mmol, CAS: 1895-39-2) was added to a mixed solution of compound CM-14A (4-bromo-2-hydroxybenzonitrile, 5.00 g, 25.25 mmol) and K₂CO₃ (4.18 g, 30.30 mmol) in DMF/H₂O (50 mL/5 mL) and the mixture was reacted at 100°C for 3 hours. Water (100 mL) was added and the resulting mixture was extracted with EtOAc (80 mL × 2). The organic phase was washed with saturated brine, then dried over anhydrous sodium sulfate and concentrated to obtain a crude and the crude was subjected to silica gel column chromatography (PE/EtOAc=100/1-20/1) to obtain a product, CM-14**B** (4-bromo-2-difluoromethoxybenzonitrile, 4.60 g, yield: 73%) as a light yellow solid.

Step B: Compounds CM-14**B** (4-bromo-2-difluoromethoxybenzonitrile, 2.00 g, 8.06 mmol), **1-2** (methyl 2-fluoro-4-(piperazin-1-ylmethyl)benzoate hydrochloride, 2.21 g, 8.77 mmol), xphos Pd G2 (0.32 g, 0.41 mmol) and Cs₂CO₃ (3.91 g, 12.00 mmol) were added to dioxane (25 mL) and the mixture was reacted under nitrogen protection at 100°C for 16 hours. The reaction solution was filtered through diatomite, the filter cake was rinsed with EtOAc, and the filtrate was subjected to rotary evaporation and concentrated to dryness under reduced pressure using a water pump. The residue was purified by silica gel column chromatography (PE/EtOAc=10/l-4/1) to obtain a product, CM-14**D** (methyl 4-((4-(4-cyano-3-(difluoromethoxy)phenyl)piperazin-1-yl)methyl)-2-fluorobenzoate, 2.00 g, 4.77 mmol, yield: 59%) as a yellow solid. MS (ESI) *m*/*z* 420.4 [M+H]⁺.

With regard to the remaining steps, reference can be made to the synthesis conditions in example 9 so as to obtain compound CM-14.

¹H NMR (400 MHz, DMSO-*d*₆) δ 10.95 (s, 1H), 7.61-7.34 (m, 6H), 7.24-7.20 (m, 2H), 6.88-6.80 (m, 2H), 5.28 (s, 2H), 5.13-5.08 (m, 1H), 4.38 (d, *J=* 17.6 Hz, 1H), 4.22 (d, *J*= 17.6 Hz, 1H), 3.57 (s, 2H), 3.40- 3.38 (m, 4H), 2.95-2.84 (m, 1H), 2.59-2.53 (m, 1H), 2.50-2.40 (m, 5H), 1.99-1.96 (m, 1H).

MS (ESI)m/z 634.2 [M+H]⁺.

### Example 17

### Compound CM-13

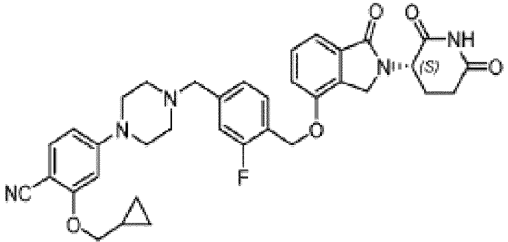

With reference to the synthesis conditions of step A in example 11, 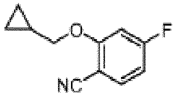 was prepared by replacing iodoethane with iodomethylcyclopropane 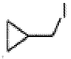 (CAS 33574-02-6); and
with regard to the remaining steps, reference can be made to the method in example 9 so as to prepare compound CM-13.

¹H NMR (400 MHz, DMSO-*d*₆) δ 10.98 (s, 1H), 7.58-7.50 (m, 2H), 7.42-7.34 (m, 3H), 7.24-7.20 (m, 2H), 6.56-6.52 (m, 2H), 5.27 (s, 2H), 5.13-5.09 (m, 1H), 4.38 (d, *J*= 17.6 Hz, 1H), 4.22 (d, *J=* 17.6 Hz, 1H), 3.94 (d, *J*=6.8 Hz, 2H), 3.56 (s, 2H), 3.34-3.32 (m, 4H), 2.95-2.86 (m, 1H), 2.61-2.53 (m, 1H), 2.50-2.42 (m, 5H), 1.98-1.95 (m, 1H), 1.25-1.18 (m, 1H), 0.60-0.56 (m, 2H), 0.35-0.29 (m, 2H).

MS (ESI)m/z 638.3 [M+H]⁺.

### Example 18

### Compound CM-16

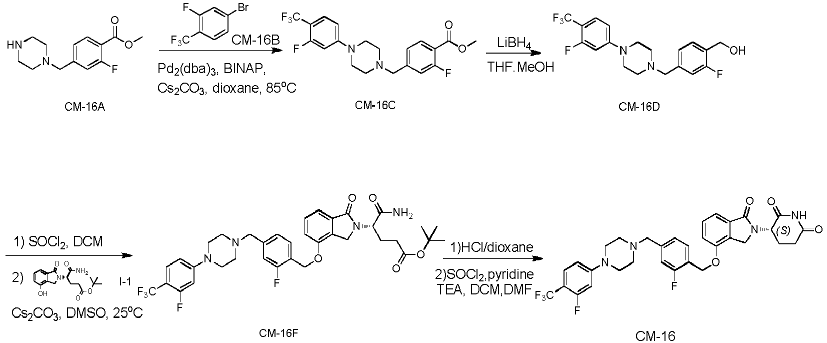

Step A: Raw materials CM-16A (methyl 2-fluoro-4-(piperazin-1-ylmethyl)benzoate, 2.20 g, 8.73 mmol), CM-16B (4-bromo-2-fluoro-1-(trifluoromethyl)benzene, 2.12 g, 8.73 mmol), Pd₂(dba)₃ (0.80 g, 0.87 mmol), BINAP (1.08 g, 1.74 mmol) and Cs₂CO₃ (4.27 g, 13.90 mmol) were added to 1,4-dioxane (50 mL) and the mixture was reacted under nitrogen protection at 85°C for 16 hours. The reaction mixture was cooled down to room temperature, filtered through diatomite and washed with ethyl acetate, the filtrate was concentrated, and the residue was subjected to column chromatography (PE/EA=10/1 to 5/1) to obtain a product, CM-16C (methyl 2-fluoro-4-((4-(3-fluoro-4-(trifluoromethyl)phenyl)piperazin-1-yl)methyl)benzoate, 2.00 g, yield: 53.5%) as a yellow solid. MS (ESI) m/z 415.4 [M+H]⁺.

With regard to the remaining steps, reference can be made to the synthesis method in example 11 and compound CM-16 was obtained by using corresponding reactants.

¹H NMR (400 MHz, DMSO-d₆) δ 10.97 (s, 1H), 7.60-7.33 (m, 5H), 7.26-7.19 (m, 2H), 6.97-6.90 (m, 1H), 6.86-6.81 (m, 1H), 5.27 (s, 2H), 5.15-5.07 (m, 1H), 4.38 (d, *J* = 17.6 Hz, 1H), 4.22 (d, *J* = 17.6 Hz, 1H), 3.56 (s, 2H), 3.32-3.28 (m, 4H), 2.96-2.84 (m, 1H), 2.61-2.52 (m, 1H), 2.49-2.37 (m, 5H), 2.01-1.91 (m, 1H).

MS (ESI) m/z 629.2 [M+H]⁺.

### Example 19

### Compound CM-25

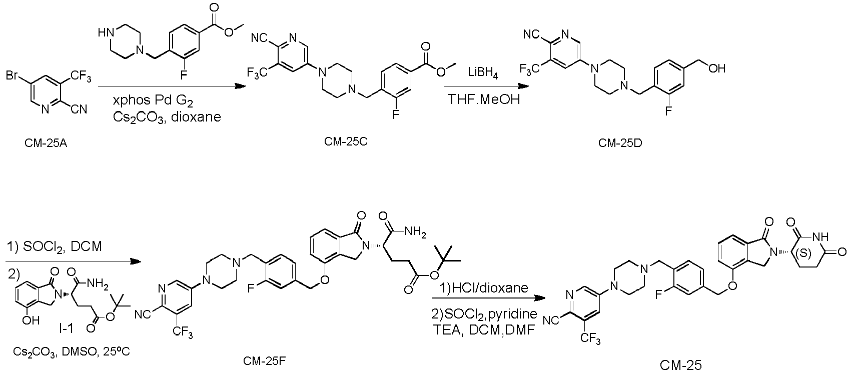

With reference to the synthetic method in example 11, compound CM-25 was obtained by using corresponding reactants.

¹H NMR (400 MHz, DMSO-*d*₆) δ 10.99 (s, 1H), 8.60 (d, *J=* 2.4 Hz, 1H), 7.60 (d, *J*=2.4 Hz, 1H), 7.51- 7.45 (m, 2H), 7.35-7.30 (m, 4H), 5.27 (s, 2H), 5.15-5.10 (m, 1H), 4.35 (d, *J*= 17.6 Hz, 1H), 4.28 (d, *J=* 17.6 Hz, 1H), 3.61-3.54 (m, 6H), 2.99-2.85 (m, 1H), 2.61-2.52 (m, 5H), 2.48-2.37 (m, 1H), 2.01-1.98 (m, 1H).

MS (ESI) m/z 637.3 [M+H]⁺.

### Example 20

### Compound CM-22

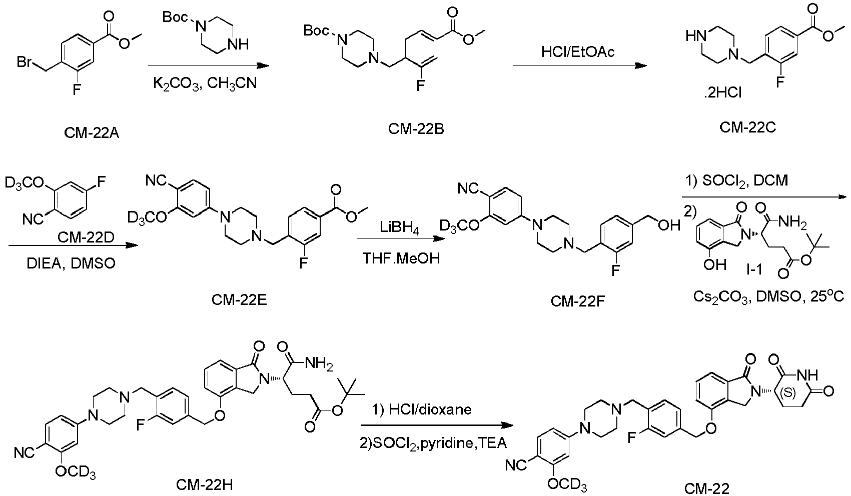

Step A: K₂CO₃ (45.54 g, 0.13 mol) was added to a solution of compound CM-22A (methyl 4-(bromomethyl)-3-fluorobenzoate, 32.00 g, 0.13 mol) and N-Boc piperazine (24.21 g, 0.13 mol) in acetonitrile (250 mL) and the mixture was stirred at 70°C for 16 hours. The reaction solution was filtered, the filtrate was concentrated, and the residue was purified through column chromatography to obtain a product, CM-22**B** (tert-butyl 4-(2-fluoro-4-(methoxycarbonyl)benzyl)piperazine-1-carboxylate, 36.00 g, yield: 78%) as a light yellow solid. MS (ESI) m/z 353.4 [M+H]⁺.

Step B: 4 N HCl/EA (200 mL) was slowly added to a solution of compound CM-22B (36.00 g, 0.10 mol) in ethyl acetate (100 mL) and the mixture was reacted at room temperature for 24 hours. The reaction solution was concentrated to a volume of about 100 mL and then filtered, and the filter cake was dried to obtain a product, CM-22C (methyl 3-fluoro-4-(piperazin-1-ylmethyl)benzoate dihydrochloride, 29.00 g, yield: 89%) as an off-white solid. MS (ESI) m/z 253.2 [M+H]⁺.

Step C: With reference to example 22, compound CM-22D (i.e., 4-fluoro-2-(methoxy-*d₃*)benzonitrile) was prepared by using corresponding reactants. At 25°C, K₂CO₃ (2.22 g, 16.12 mmol) was added to a solution of compounds CM-22**C** (methyl 3-fluoro-4- (piperazin-1 -ylmethyl)benzoate dihydrochloride, 2.62 g, 8.06 mmol) and CM-22**D** (1.25 g, 8.06 mmol) in DMSO (20 mL) and the mixture was stirred at 90°C for 16 hours. The reaction solution was poured into water (60 mL) and the mixture was extracted with EtOAc (50 mL × 2). The organic phase was washed with saturated brine (60 mL × 3), then dried over anhydrous Na₂SO₄, filtered and concentrated. The residue was subjected to reversed phase preparative chromatography to obtain a product, CM-22**E** (methyl 4-((4-(4-cyano-3-(methoxy-*d*₃)phenyl)piperazin-1-yl)methyl)-3-fluorobenzoate, 1.60 g, 4.15 mmol, yield: 51%) as a yellow solid. MS (ESI) *m*/*z* 387.1 [M+H]⁺.

With regard to the remaining steps, reference can be made to the synthesis conditions in example 11 and compound CM-22 was obtained by using corresponding reactants.

¹H NMR(400 MHz, DMSO-*d*₆) δ 10.98 (s, 1H), 7.53-7.28 (m, 7H), 6.58-6.51 (m, 2H), 5.26 (s, 2H), 5.16- 5.08 (m, 1H), 4.44 (d, *J=* 17.6 Hz, 1H), 4.29 (d, *J*= 17.2 Hz, 1H), 3.59 (s, 2H), 3.40-3.32 (m, 4H), 2.96-2.86 (m, 1H), 2.59-2.53 (m, 1H), 2.49-2.40 (m, 5H), 2.01-1.97 (m, 1H).

MS (ESI) m/z 601.3 [M+H]⁺.

### Example 21

### Compound CM-40

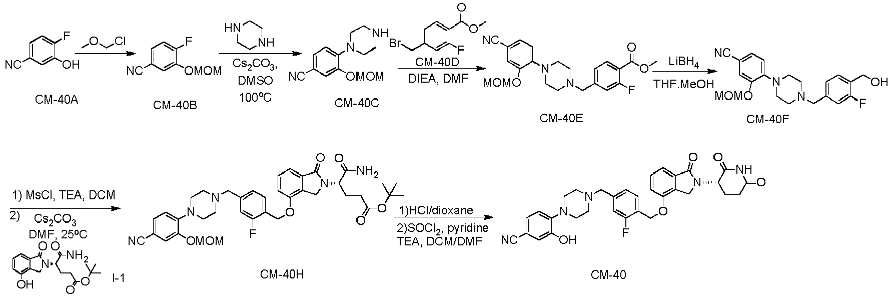

Step A: At 0°C-5°C, chloromethyl methyl ether (4.05 g, 50.32 mmol, CAS: 107-30-2) was slowly added to a mixed solution of compound CM-40**A** (3-hydroxy-4-fluorobenzonitrile, 4.60 g, 33.55 mmol, CAS: 186590-04-5) and DIEA (8.66 g, 67.10 mmol) in DCM (50 mL) and the mixture was reacted at room temperature for 16 hours. Water (60 mL) was added and the resulting mixture was extracted with ethyl acetate (50 mL × 2). The organic phase was washed with saturated brine, then dried over anhydrous sodium sulfate and concentrated to obtain a product, CM-40**B** (4-fluoro-3-(methoxymethoxy)benzonitrile, 7.00 g, crude) as a light yellow liquid, which was directly used in the next reaction.

Step B: Cs₂CO₃ (30.97 g, 95.00 mmol) was added to a solution of compound CM-40**B** (7.00 g, 38.67 mmol) and piperazine (16.63 g, 193.37 mmol) in DMSO (100 mL) and the mixture was stirred at 100°C for 16 hours. The reaction solution was poured into water (200 mL) and the mixture was extracted with EtOAc (150 mL × 2). The organic phase was washed with saturated brine (300 mL × 3), then dried over anhydrous Na₂SO₄, filtered and concentrated to obtain a product, CM-40C (3-(methoxymethoxy)-4-(piperazin-1-yl)benzonitrile, 6.60 g, 56% yield) as a yellow solid, which was directly used in the next reaction.

Step C: DIEA (6.89 g, 53.44 mmol) was added to a solution of compounds CM-40**C** (6.60 g, 26.69 mmol) and CM-40**D** (methyl 4-(bromomethyl)-2-fluorobenzoate, 6.60 g, 26.69 mmol) in DMF (80 mL) and the mixed solution was stirred at 100°C for 2 hours. The reaction solution was concentrated and the residue was subjected to column chromatography (PE/EtOAc=4/1) to obtain a product, CM-40**E** (methyl 4-((4-(4-cyano-2-(methoxymethoxy)phenyl)piperazin-1-yl)methyl)-2-fluorobenzoate, 8.00 g, yield: 72%) as a yellow solid; and
with regard to the remaining steps, reference can be made to example 11 and compound CM-40 was obtained by using corresponding reactants.

¹H NMR (400 MHz, DMSO-*d*₆) δ 10.96 (s, 1H), 10.02 (s, 1H), 7.60-7.47 (m, 2H), 7.44-7.30 (m, 2H), 7.26-7.15 (m, 3H), 7.06-7.01 (m, 1H), 6.94-6.87 (m, 1H), 5.27 (s, 2H), 5.14-5.06 (m, 1H), 4.38 (d, *J=* 17.6 Hz, 1H), 4.22 (d, *J=* 17.6 Hz, 1H), 3.56 (s, 2H), 3.17-3.05 (m, 4H), 2.94-2.85 (m, 1H), 2.60-2.51 (m, 5H), 2.48-2.40 (m, 1H), 2.01-1.92 (m, 1H).

MS (ESI) m/z 584.3 [M+H]⁺.

### Example 22

### Compound CM-32

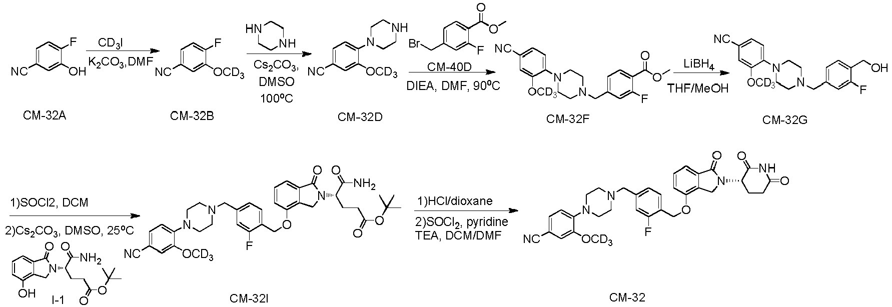

Step A: Deuterated iodomethane (2.06 g, 14.23 mmol) was added to a mixed solution of compound CM-32**A** (3-hydroxy-4-fluorobenzonitrile, 1.50 g, 10.95 mmol) and K₂CO₃ (3.02 g, 21.88 mmol) in DMF (20 mL) and the mixture was reacted at room temperature for 16 hours. Water (60 mL) was added and the resulting mixture was extracted with ethyl acetate (30 mL × 2). The organic phase was washed with saturated brine, then dried over anhydrous sodium sulfate and concentrated to obtain a product, CM-32**B** (4-fluoro-3-(methoxy-*d*₃)benzonitrile, 1.50 g, yield: 89%) as a gray solid, which was directly used in the next reaction.

Step B: Cs₂CO₃ (5.50 g, 16.08 mmol) was added to a solution of compound CM-32**B** (1.30 g, 8.44 mmol) and piperazine (3.63 g, 42.2 mmol) in DMSO (15 mL) and the mixed solution was stirred at 100°C for 16 hours. The reaction solution was poured into water (60 mL) and the mixture was extracted with EtOAc (50 mL × 2). The organic phase was washed with saturated brine (60 mL × 3), then dried over anhydrous Na₂SO₄, filtered and concentrated to obtain a product, CM-32**D** (3-(methoxy-*d*₃)-4-(piperazin-1-yl)benzonitrile, 1.80 g, yield: 97%) as a light yellow solid, which was directly used in the next reaction.

Step C: DIEA (2.99 g, 23.16 mmol) was added to a solution of compounds CM-32D (1.70 g, 7.72 mmol) and CM-40D (methyl 4-(bromomethyl)-2-fluorobenzoate, 2.38 g. 9.66 mmol) in DMSO (15 mL) and the mixed solution was stirred at 90°C for 8 hours. The reaction solution was concentrated and the residue was subjected to column chromatography to obtain a product, CM-32F (methyl 4-((4-(4-cyano-2-(methoxy-*d*₃)phenyl)piperazin-1-yl)methyl)-2-fluorobenzoate, 1.80 g, yield: 60%) as a yellow solid, MS (ESI) m/z 387.5 [M+H]⁺.

With regard to the remaining steps, reference can be made to the synthetic method in example 11 and compound CM-32 was obtained by using corresponding reactants.

¹H NMR (400 MHz, DMSO-*d*₆) δ 10.96 (s, 1H), 7.58-7.50 (m, 2H), 7.41-7.20 (m, 6H), 6.97-6.95 (m, 1H), 5.27 (s, 2H), 5.13-5.08 (m, 1H), 4.38 (d, *J=* 17.6 Hz, 1H), 4.22 (d, *J*=17.6 Hz, 1H), 3.56 (s, 2H), 3.18- 3.01 (m, 4H), 2.94-2.83 (m, 1H), 2.58-2.51 (m, 1H), 2.49-2.38 (m, 5H), 1.98-1.95 (m, 1H).

MS (ESI) m/z 601.2 [M+H]⁺.

With reference to the synthetic method for compound CM-32, compounds CM-15 and CM-36 were obtained by using corresponding reactants.

### Compound CM-15

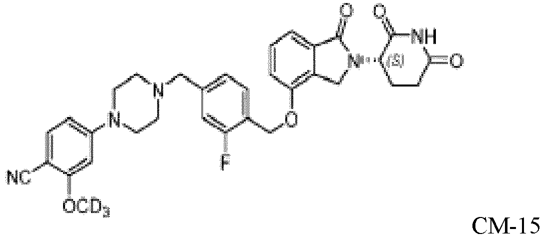

¹H NMR (400 MHz, DMSO-*d*₆) δ 10.95 (s, 1H), 7.58-7.49 (m, 2H), 7.43-7.34 (m, 3H), 7.24-7.21 (m, 2H), 6.57-6.54 (m, 2H), 5.28 (s, 2H), 5.13-5.08 (m, 1H), 4.38 *(d, J=* 17.6 Hz, 1H), 4.22 (d, *J=* 17.6 Hz, 1H), 3.57 (s, 2H), 3.38-3.32 (m, 4H), 2.93-2.85 (m, 1H), 2.60-2.53 (m, 1H), 2.49-2.40 (m, 5H), 1.99-1.95 (m, 1H).

MS (ESI)m/z 601.3 [M+H]⁺.

### Compound CM-36

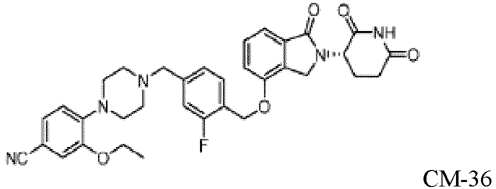

¹H NMR (400 MHz, DMSO-*d*₆) δ 10.97 (s, 1H), 7.57-7.50 (m, 2H), 7.41-7.22 (m, 6H), 6.96-6.94 (m, 1H), 5.27 (s, 2H), 5.13-5.09 (m, 1H), 4.41 (d, *J=* 17.6 Hz, 1H), 4.25 (d, J= 17.6 Hz, 1H), 4.09- 4.04 (m, 2H), 3.56 (s, 2H), 3.12 (m, 4H), 2.95-2.86 (m, 1H), 2.58-2.50 (m, 5H), 2.46-2.40 (m, 1H), 1.99-1,96 (m, 1H), 1.34 (t, *J* = 6.8Hz,3H).

MS (ESI) m/z 612.2 [M+H]⁺.

### Example 23

### Compound CM-41

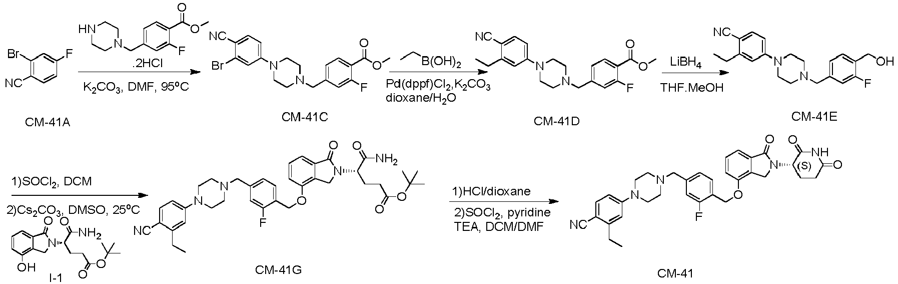

CM-41C (methyl 4-((4-(3-bromo-4-cyanophenyl)piperazin-1-yl)methyl)-2-fluorobenzoate, 3.50 g, 8.10 mmol), ethylboronic acid (1.20 g, 16.20 mmol), Pd₂(dppf)Cl₂ (0.18 g, 0.24 mmol) and potassium carbonate (1.72 g, 16.20 mmol) were added to dioxane/water (30 mL/7 mL) and the mixture was reacted under nitrogen protection at 90°C for 16 hours. The reaction mixture was cooled down to room temperature, filtered through diatomite and then rinsed with ethyl acetate. The filtrate was concentrated to obtain a crude and the crude was subjected to reversed phase preparative chromatography to obtain a product, CM-41**D** (methyl4-((4-(4-cyano-3-ethylphenyl)piperazin-1-yl)methyl)-2-fluorobenzoate, 1.00 g, 2.62 mmol, yield: 32%) as a white solid. MS (ESI) m/z 382.5 [M+H]⁺.

With regard to the remaining steps, reference can be made to the synthetic method in example 11 and compound CM-41 was obtained by using corresponding reactants.

¹H NMR (400 MHz, DMSO-*d*₆) δ 10.96 (s, 1H), 7.60-7.47 (m, 3H), 7.42-7.32 (m, 2H), 7.26-7.18 (m, 2H), 6,93-6.81 (m, 2H), 5.27 (s, 2H), 5.15-5,06 (m, 1H), 4.38 *(d, J=* 17.6 Hz, 1H), 4.22 (d, *J=* 17.6 Hz, 1H), 3.56 (s, 2H), 3.34-3.31 (m, 4H), 2.96-2.84 (m, 1H), 2.68 (q, *J=* 7.2 Hz, 2H), 2.58-2.52 (m, 1H), 2.49-2.37 (m, 5H), 2.01-1.93 (m, 1H), 1.19 (t,7.2 Hz, 3H).

MS (ESI) m/z 596.2 [M+H]⁺.

With reference to the synthetic method of compound CM-41, compound CM-37 was prepared by replacing ethylboronic acid with cyclopropylboronic acid.

### Compound CM-37

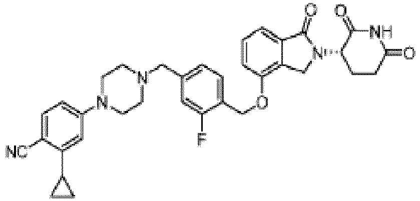

¹H NMR (400 MHz, DMSO-*d*₆) δ 10.96 (s, 1H). 7.60-7.32 (m, 5H), 7.25-7.18 (m, 2H), 6.83-6.78 (m, 1H),6.42(d, *J*= 2.0 Hz, 1H).5.27(s, 2H), 5.15-5.06 (m, 1H), 4.38 (d, *J*=17.6 Hz, 1H), 4.22 (d, *J*=17.6 Hz,1H), 3.55 (s, 2H), 3.32-3.29 (m, 4H), 2.97-2.83 (m, 1H), 2.59-2.52 (m, 1H), 2.49-2.37 (m, 5H), 2.10-1.91 (m, 2H), 1.05-0.98 (m, 2H), 0.85-0.78 (m, 2H).

MS (ESI) m/z 608.2 [M+H]⁺.

### Example 24

### Compound CM-43

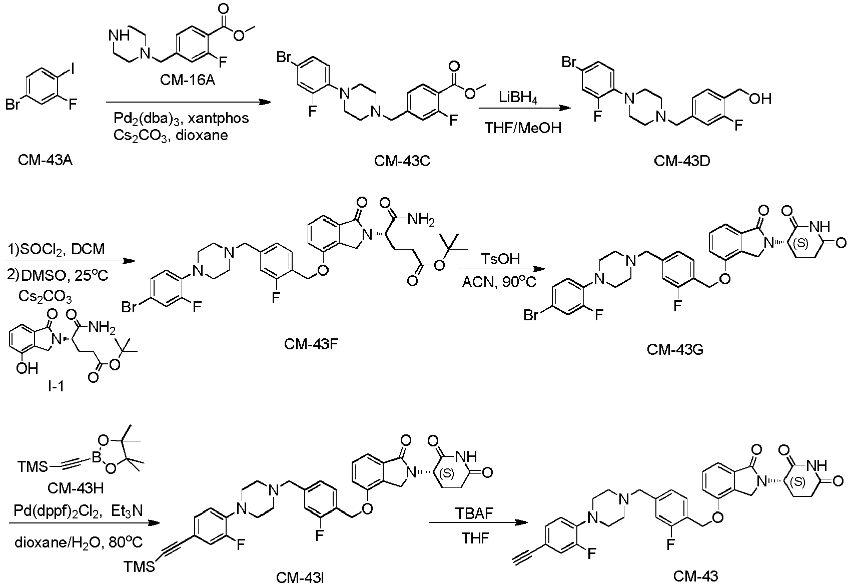

With reference to the synthetic method in example 11, compound CM-43G was obtained by using corresponding reactants.

Compounds CM-43**G** (200 mg, 0.31 mmol) and CM-43**H** (trimethyl((4,4,5,5-tetramethyl-1,3,2-dioxaborolan-2-yl)ethynyl)silane, 210 mg, 0.93 mmol, CAS 159087-46-4) were dissolved in 8 mL of dioxane/water (6 mL/2 ml) and Pd(dppf)₂Cl₂ (91.5 mg, 0.125 mmol) and Et3N (126 mg, 1.25 mmol) were added. The reaction system was subjected to nitrogen replacement 3 times and then heated at 80°C for 2.5 h under nitrogen protection, and the reaction was completed. The reaction solution was filtered through diatomite, then washed with EtOAc (10 mL) and concentrated under reduced pressure and the residue was purified by reversed phase preparative chromatography (10%-80% ACN) to obtain CM-43I ((S)-3-(4-((2-fluoro-4-((4-(2-fluoro-4-((trimethylsilyl)ethynyl)phenyl)piperazin-1-yl)methyl)benzyl)oxy)-1-oxoisoindolin-2-yl)piperidine-2,6-dione, 100 mg, yield: 42%).

TBAF/THF solution (1 M, 1 mmol, 1 mL) was added to a solution of CM-43I (100 mg, 0.15 mmol) in THF (5 mL), the mixed solution was stirred at room temperature for 1 hour, and the reaction was completed. The reaction solution was extracted with EtOAc (10 mL), then washed with water (10 mL × 6) and concentrated and the residue was subjected to reversed phase medium-pressure preparative chromatography and then purified by Prep-HPLC (TFA) to obtain the TFA salt of a product (15 mg). The salt was dissolved in DMF and the resulting mixture was slowly added dropwise to aq. NaHCO₃ (20 mL) to precipitate a white solid. The solid was filtered and vacuum-dried using an oil pump to obtain compound CM-43 (7.8 mg, yield: 9%).

¹H NMR (400 MHz, DMSO-*d*₆) δ 10.97 (s, 1H), 7.58-7.50 (m, 2H). 7.41-7.21 (m, 6H), 7.01-6.97 (m, 1H), 5.27 (s, 2H), 5.13-5.08 (m, 1H), 4.41-4.13 (m, 3H), 3.57 (s, 2H), 3.07-2.95 (m, 4H), 2.95-2.86 (m, 1H), 2.58-2.51 (m, 4H), 2.46-2.32 (m, 2H), 1.98-1.94 (m, 1H).

MS (ESI) m/z [M+H]+ =585.3.

With reference to the synthetic method of compound CM-43, CM-38 was prepared by replacing CM-43H with 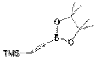 (CAS 608534-34-5).

### Compound CM-38

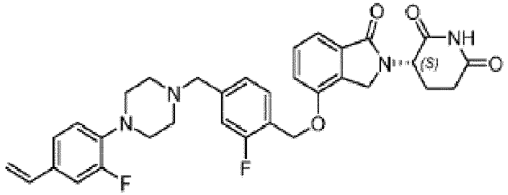

¹H NMR (400 MHz, DMSO-*d*₆) δ 10.96 (s, 1H), 7.58-7.50 (m, 2H), 7.41-7.16 (m, 6H), 7.00-6.95 (m, 1H), 6.67-6.60 (m, 1H), 5.73 (d, *J*=17.6 Hz, 1H), 5.27 (s, 2H), 5.19-5.08 (m, 2H), 4.38 (d, *J*=17.6 Hz, 1H),4.22 (d, *J*= 17.6 Hz, 1H), 3.57 (s, 2H), 3.04-2.95 (m, 4H), 2.95-2.86 (m, 1H), 2.58-2.51 (m, 5H), 2.46-2.39 (m, 1H), 1.99-1.95 (m, 1H).

MS (ESI) m/z [M+H]⁺ = 587.2.

### Example 25

### Compound CM-42

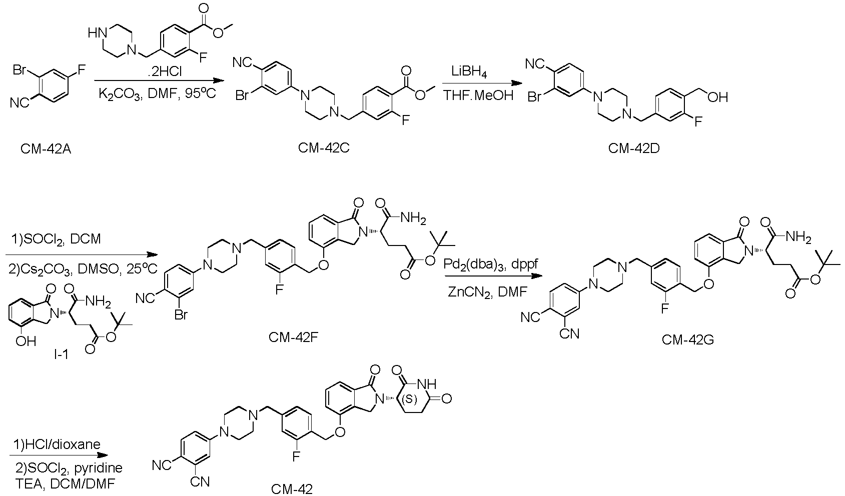

With reference to the method in example 11, compound CM-42F was synthesized. CM-42F (tert-butyl (S)-5-amino-4-(4-((4-((4-(3-bromo-4-cyanophenyl) piperazin-1-yl)methyl)-2-fluorobenzyl)oxy)-1-oxoisoindolin-2-yl)-5-oxopentanoate, 1.00 g, 1.46 mmol), Zn(CN)₂ (1.00 g, 256.23 mmol), Pd₂(dba)₃ (0.12 g, 0.13 mmol) and dppf (0.14 g, 0.26 mmol) were added to DMF (10 mL) and the mixture was reacted under nitrogen protection at 100°C for 5 hours. The reaction mixture was cooled down to room temperature, water (50 mL) was added and the resulting mixture was extracted with ethyl acetate (30 mL×2). The organic phases were combined and then washed with saturated brine (50 mL×3). The organic phase was dried over anhydrous sodium sulfate, then filtered and concentrated to obtain a crude and the crude was subjected to reversed phase preparative chromatography to obtain a product, CM-42**G** (tert-butyl (S)-5-amino-4-(4-((4-((4-(3,4-dicyanophenyl)piperazin-1-yl)methyl)-2-fluorobenzyl)oxy)-1-oxoisoindolin-2-yl)-5-oxopentanoate, 700 mg, yield: 72%) as a white solid.

With regard to the remaining steps, reference can be made to example 11 and compound CM-42 can be obtained by using corresponding reactants.

¹H NMR (400 MHz, DMSO-*d*₆) δ 10.95 (s, 1H), 7.80 (d, *J*= 9.2 Hz, 1H),7.60-7.48 (m, 3H), 7.42-7.18 (m, 5H), 5.27 (s, 2H), 5.15-5.06 (m, 1H), 4.38 *(d, J=* 17.6 Hz,1H), 4.22 (d, J =17.6 Hz, 1H), 3.56 (s, 2H), 3.48-3.42 (m, 4H), 2.96-2.84 (m, 1H), 2.58-2.52 (m, 1H), 2.49-2.37 (m, 5H), 2.01-1.93 (m, 1H).

MS (EST) m/z 593.2 [M+H]⁺.

### Example 26

### Compound CM-44

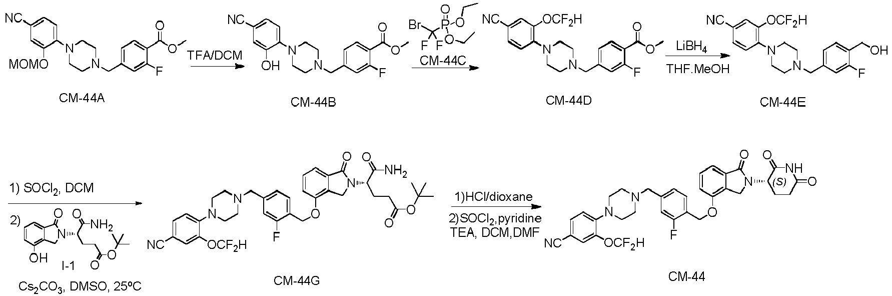

Step A: At 0°C-5°C, TFA (20 mL) was added to a solution of compound CM-44**A** (methyl 4-((4-(4-cyano-2-(methoxymethoxy)phenyl)piperazin-1-yl)methyl)-2-fluorobenzoate, 3.00 g, 7.26 mmol) in DCM (20 mL) and the mixed solution was stirred at room temperature for 1.5 hours. The reaction solution was concentrated and then diluted with EtOAc and aqueous NaHCO₃ solution was added to adjust the mixture to pH = 8-9. The resulting mixture was extracted with EtOAc (30 mL × 3). The organic phases were combined, then washed with saturated brine (50 mL), dried over anhydrous Na₂SO₄, filtered and concentrated to obtain a product, CM-44**B** (methyl 4-((4-(4-cyano-2-hydroxyphenyl)piperazin-1-yl)methyl)-2-fluorobenzoate, 1.70 g, yield: 91%) as a yellow solid. MS (ESI) m/z 370.1 [M+H]⁺

Step B: Compound CM-44**B** (800 mg, 2.17 mmol) was dissolved in THF (8 mL) and the mixture was cooled down to -15°C. Sodium hydride (104 mg, 4.34 mmol) was added in portions and the temperature was maintained below -5°C. After 30 minutes, water (78 mg, 4.34 mmol) was added dropwise. After 10 minutes, CM-44C (diethyl(bromodifluoromethyl)phosphonate, 1.16 g, 4.34 mmol) was added in portions and the mixture was stirred at room temperature for 1 hour. The reaction solution was poured into water and the resulting mixture was extracted with ethyl acetate (30 mL × 3). The organic phase was washed with saturated brine (80 mL), then dried over anhydrous Na₂SO₄, filtered and concentrated and the residue was subjected to reversed phase preparative chromatography to obtain a product, CM-44**D** (methyl 4-((4-(4-cyano-2-(difluoromethoxy)phenyl)piperazin-1-yl)methyl)-2-fluorobenzoate, 0.35 g, 0.83 mmol, yield: 38%) as a white solid.

With regard to the remaining steps, reference can be made to example 11 and CM-44 can be obtained by using corresponding reactants.

¹H NMR (400 MHz, DMSO-d₆) δ 10.95 (s, 1H), 7.68-7.48 (m, 4H), 7.42-6.95 (m, 6H), 5.27 (s, 2H), 5.14- 5.06 (m, 1H),4.38(d, *J* = 17.6 Hz, 1H), 4.22 *(d, J=* 17.6 Hz, 1H), 3.56 (s, 2H), 3.20-3.06 (m, 4H), 2,97-2.82 (m, 1H). 2.62-2.52 (m, 5H), 2.48-2.40 (m, 1H), 2.01-1.92 (m, 1H).

MS (ESI) m/z 634.2 [M+H]⁺.

### Example 27

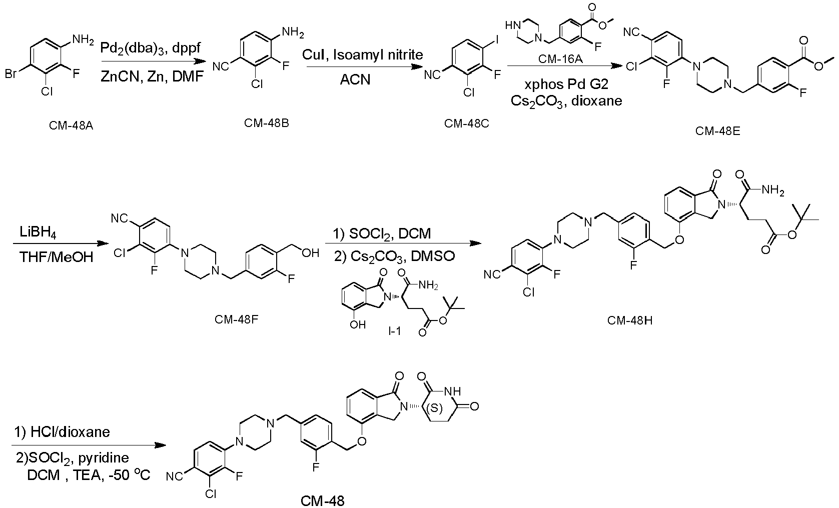

Step A: Compound CM-48A (CAS 115843-99-7, 15.00 g, 66.82 mmol), 1,1'-bis(diphenylphosphino)ferrocene (dppf) (3.70 g, 6.68 mmol), Pd₂(dba)₃ (3.06 g, 3.34 mmol) and Zn(CN)₂ (10.16 g, 86.84 mmol) were added to DMF (150 mL), heated to 100°C under nitrogen protection and reacted for 16 hours. The reaction solution was cooled down and filtered and the filter cake was rinsed with EtOAc (80 mL). The filtrate was concentrated and the residue was subjected to silica gel column chromatography (PE/EA = 2/1) to obtain a product, CM-48B (9.00 g, 52.76 mmol, yield: 79%) as an off-white solid.

Step B: CuI (20.34 g, 106.49 mmol) and isoamyl nitrite (12.48 g, 106.49 mmol) were added to MeCN (90 mL). The reaction solution was heated to 65°C and a solution of CM-48**B** (9.00 g, 52.79 mmol) in acetonitrile (90 mL) was added dropwise. The dropwise addition was completed in 30 minutes and the reaction was continued for 5 hours. The reaction solution was concentrated and the residue was subjected to column chromatography (PE/EA = 20/1) to obtain a product, CM-48C (5.00 g, 17.77 mmol, yield: 33%) as an off-white solid. MS (ESI) m/z 281.9 [M+H]⁺.

Step C: Compounds CM-16A (2.70 g, 10.71 mmol) and CM-48C (3.00 g, 10.71 mmol), xphos Pd G2 (0.86 g, 1.07 mmol) and Cs₂CO₃ (5.24 g, 16.06 mmol) were added to 1,4-dioxane (30 ml) and the mixture was reacted under nitrogen protection at 110°C for 16 hours. The reaction mixture was cooled down to room temperature, filtered through diatomite and then washed with ethyl acetate. The filtrate was concentrated and the residue was subjected to column chromatography (PE/EA=10/1 to 5/1) to obtain a product, CM-48E as a yellow solid (2.07 g, 5.10 mmol, yield: 47.6%).

With regard to the remaining steps, reference can be made to the synthesis in example 11 and compound CM-48 was obtained by using corresponding reactants.

¹H NMR (400 MHz, DMSO-d₆) δ 10.96 (s, 1H), 7.68 (d, *J=* 8.8 Hz, 1H), 7.59-7.49 (m, 2H), 7.41-7.33 (m, 2H), 7.24-7.19 (m, 2H), 7.13-7.07(m, 1H), 5.27 (s, 2H), 5.14-5.07 (m, 1H), 4.38 (d, *J* =17.6 Hz, 1H), 4.22 (d, *J*= 17.6 Hz, 1H), 3.57 (s, 2H), 3.30-3.27 (m, 4H), 2.96-2.85 (m, 1H), 2.60-2.52 (m, 5H), 2.49-2.39 (m, 1H), 2.00-1.94 (m, 1H).

MS (EST) m/z 620.2 [M+H]⁺.

With reference to the synthetic method of compound CM-48, compounds CM-47 and CM-49 were obtained by using corresponding reactants.

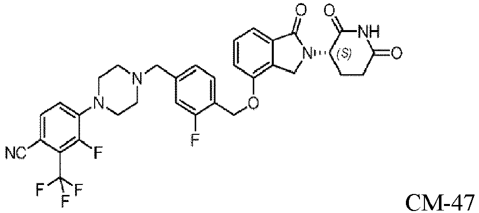

¹H NMR (400 MHz, DMSO-*d*₆) δ 10.96 (s, 1H), 7.80 (d, *J*=8.8 Hz, 1H), 7.59-7.34 (m, 5H), 7.24-7.20 (m, 1H), 5.27 (s, 2H), 5.13-5.08 (m, 1H), 4.36 (d, *J=* 17.2 Hz, 1H), 4.21 (d, *J* =17.6 Hz, 1H), 3.57 (s, 2H), 3.32-3.27 (m, 4H), 2.95-2.86 (m, 1H), 2.59-2.54 (m, 5H), 2.50-2.38 (m, 1H), 1,99-1.96 (m, 1H).

MS (ESI) m/z 654.3 [M+H]⁺.

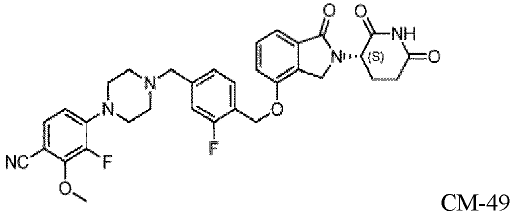

¹H NMR (400 MHz, DMSO-d₆) δ 10.97 (s, 1H), 7.59-7.34(m, 5H), 7.25-7.19 (m, 2H), 6.85-6.79 (m, 1H), 5.27 (s, 2H), 5.14-5.07 (m, 1H), 4.38 (d, *J=* 17.6 Hz, 1H), 4.22 (d, *J* =17.2 Hz, 1H), 3.99 (d, *J=* 1.2 Hz, 1H), 3.57 (s, 2H), 3.22-3.15 (m, 4H), 2.92-2.85 (m, 1H), 2.60-2.52 (m, 5H), 2.49-2.39 (m, 1H), 2.00-1.94 (m, 1H).

MS (ESI) m/z 616.3 [M+H]⁺.

### Example 28

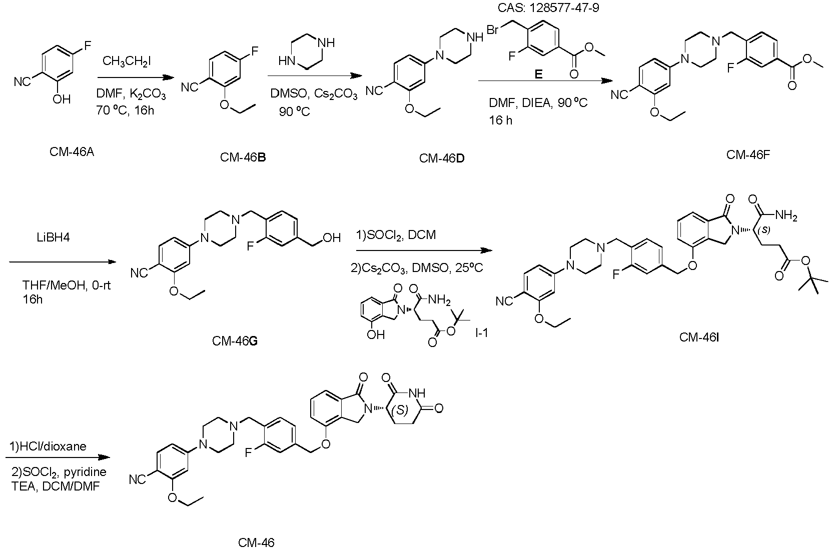

With reference to the synthesis conditions of compound CM-12 in example 11, compound CM-46 was synthesized by using corresponding reactants.

¹H NMR (400 MHz, DMSO) δ 10.97 (s, 1H), 7.51-7.30 (m, 7H), 6.55-6.53 (m, 2H), 5.27 (s, 2H), 5.14-5.09 (m, 1H), 4.43 (d, *J*=17.2 Hz, 1H), 4.27 (d, *J=* 17.6 Hz, 1H), 4.16-4.11 (m, 2H), 3.59 (s, 2H), 3.33 (m, 4H), 2.96-2.87 (m, 1H), 2.61-2.50 (m, 5H), 2.43-2.40 (m, 1H), 2.01-1.98 (m, 1H), 1.33 (t, *J=* 6.8 Hz, 3H).

MS (ESI) m/z 612.3 [M+H]⁺.

With reference to the synthetic method of compound CM-46 in example 28, CM-20, CM-21, CM-23, CM-26 and CM-45 were synthesized by using corresponding reactants.

### Compound CM-20

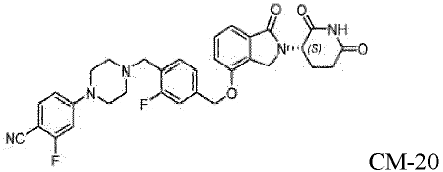

¹H NMR (400 MHz, DMSO-*d*₆) δ 10.97 (s, 1H), 7.61-7.44 (m, 3H), 7.35-7.31 (m, 4H), 6.95-6.91 (m, 1H), 6.85-6.82 (m, 1H), 5.26 (s, 2H), 5.14-5.09 (m, 1H), 4.45 (d, *J*= 17.6 Hz, 1H), 4.29 (d, *J=* 17.6 Hz, 1H), 3.59 (s, 2H), 3.38-3.32 (m, 4H), 2.91-2.87 (m, 1H), 2.60-2.55 (m, 1H), 2.49-2.40 (m, 5H), 2.01-1.98 (m, 1H).

MS (ESI) m/z 586.2 [M+H]⁺.

### Compound CM-21

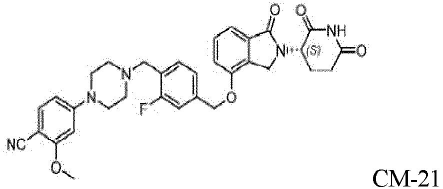

¹H NMR (400 MHz, DMSO-*d*₆) δ 10.99 (s, 1H), 7.49-7.30 (m, 7H), 6.56-6.54 (m, 2H), 5.26 (s, 2H), 5.14-5,10 (m, 1H), 4.47 (d, *J*= 17.2 Hz, 1H), 4,31 (d, *J*= 17.2 Hz, 1H), 3,86 (s, 2H), 3,35-3.33 (m, 4H), 2,96-2.87 (m, 1H), 2.60-2.50 (m, 5H), 2.50-2.40 (m, 1H), 2.01-1.97 (m, 1H).

MS (ESI) m/z 598.2 [M+H]⁺.

### Compound CM-23

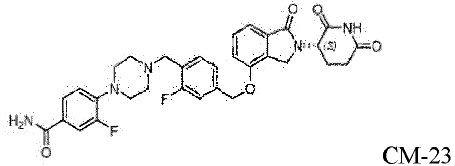

¹H NMR (400 MHz, DMSO-*d*₆) δ 10.98 (s, 1H), 7.86 (s, 1H), 7.67-7.58 (m, 2H), 7.52-7.43 (m, 2H),7.36-7.27 (m, 5H), 7.06-6.99 (m, 1H), 5.26 (s, 2H), 5.15-5.08(m, 1H), 4.45 (d, *J=* 17.6 Hz, 1H), 4.29 (d, *J* = 17.6 Hz, 1H), 3.60 (s, 2H), 3.15-3.00 (m, 4H), 2.98-2.83 (m, 1H), 2.64-2.52 (m, 5H), 2.48-2.39 (m, 1H), 2.04-1.93 (m, 1H).

MS (ESI) m/z 604,2 [M+H]⁺.

### Compound CM-26

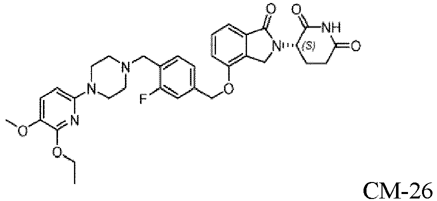

¹H NMR (400 MHz, DMSO-*d*₆) δ 10.97 (s, 1H), 7.51-7.44 (m, 2H), 7.35-7.31 (m, 4H), 7.17-7.15 (m, 1H), 6.18-6.16 (m, 1H), 5.26 (s, 2H), 5.14-5.09 (m, 1H), 4.45 (d, *J* = 17.6 Hz, 1H), 4.31-4.22 (m, 3H), 3.65 (s, 3H), 3.57 (s, 2H), 3.32-3.30 (m, 4H), 2.96-2.87 (m, 1H), 2.67-2.56 (m, 1H), 2.51-2.49 (m, 5H), 2.00-1.96 (m, 1H), 1.28 (t, *J*=6.8 Hz, 3H).

MS (ESI) m/z 618.2 [M+H]⁺.

### Compound CM-45

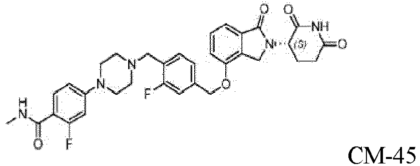

1H NMR (400 MHz, DMSO-d6) δ 10.97 (s, 1H), 7.76-7.72 (m, 1H), 7.59-7.43 (m, 3H), 7.36-7.30 (m, 4H), 6.79-6.69 (m, 2H), 5.26 (s, 2H), 5.15-5.07 (m, 1H), 4.45 (d. J = 17.6 Hz, 1H), 4.29 (d, J= 17.2 Hz, 1H),3.58 (s, 2H), 3.30-3.24 (m, 4H), 2.93-2.87 (m, 1H), 2.74 (d, J = 4.4 Hz, 3H), 2.62-2.52 (m, 5H), 2.49-2.41 (m, 1H), 2.02-1.97 (m, 1H).

MS (ESI) m/z 618.2 [M+H]+.

### Efficacy Examples

### Experimental method for cell proliferation inhibition test by MTS assay:

Proliferating MM.1S myeloma cells (ATCC, Cat # CRL-2974) were resuspended in RPMI-640 medium (Gibco, Cat # A10491-01) and counted using an automated cell counter. The cell suspension was diluted to the needed density and 100 µl (15,000 cells) of the cell suspension was plated per well in a 96-well plate. The plate was cultured in a 37°C, 5% CO2 incubator for 24 hours. On the day of administration, each compound was formulated into 20 mM stock solution with DMSO (Sigma, Cat # D2650), diluted with a medium to the needed concentration, and then added to the corresponding wells for the cell proliferation inhibition test with a drug of 4-fold serial dilution with 10 points. The final concentration of DMSO was 0.5%. After drug addition, the 96-well plate was cultured in a 37°C, 5% CO2 incubator for 72 hours. Then, 20 µl of MTS (Promega, Cat # G3581) was added to each well and the plate was cultured for 2 hours in a 37°C, 5% CO2 incubator, and then the absorbance at 490 nm of each well was recorded using an Ensight instrument. The reading of the cells at Day 0 was used as a negative control and the reading of cells with 0.5% DMSO at Day-3 was used as a positive control. Cell proliferation inhibition curve was plotted using GraphPad Prism 5 software and the IC50 value was calculated. Experiment results can be seen in Table 1.

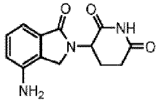

### Control 1

**Table 1. MM.1S cell proliferation inhibition results**

| Compounds | MM.1S IC₅₀ (nM) |
|---|---|
| Control 1 | 23.0691 |
| CM-1 | 0.1025 |
| CM-2 | 0.0139 |
| CM-3 | 0.0064 |
| CM-4 | 0.0153 |
| CM-5 | 0.0063 |
| CM-6 | 0.0409 |
| CM-7 | 0.0054 |
| CM-8 | 0.0211 |
| CM-9 | 0.0136 |
| CM-10 | 0.0105 |
| CM-11 | 0.0091 |
| CM-12 | 0.0067 |
| CM-13 | 0.0271 |
| CM-14 | 0.0148 |
| CM-15 | 0.0062 |
| CM-16 | 0.1886 |
| CM-17 | 0.0139 |
| CM-18 | 0.0188 |
| CM-19 | 0.0338 |
| CM-20 | 0.0132 |
| CM-21 | 0.0111 |
| CM-22 | 0.0136 |
| CM-23 | 0.0581 |
| CM-24 | 0.0388 |
| CM-25 | 0.0291 |
| CM-26 | 0.4648 |
| CM-27 | 0.1339 |
| CM-28 | 0.1020 |
| CM-29 | 0.2006 |
| CM-30 | 0.1092 |
| CM-31 | 0.1657 |
| CM-32 | 0.0706 |
| CM-33 | 0.0781 |
| CM-34 | 0.0766 |
| CM-35 | 0.0962 |
| CM-36 | 0.1479 |
| CM-37 | 0.1193 |
| CM-38 | 1.2090 |
| CM-39 | 0.0631 |
| CM-40 | 0.3778 |
| CM-41 | 0.1158 |
| CM-42 | 0.1143 |
| CM-43 | 0.5762 |
| CM-44 | 0.1265 |

### Experimental method for cell proliferation inhibition test by CTG assay:

The proliferation inhibitory activity of the compounds in the MM.1S myeloma cell model was determined by CellTiter-Glo (CTG) Luminescent Cell Viability Assay (Promega). MM.1S cells (ATCC, Cat # CRL-2974) at exponential phase were resuspended in RPMI-1640 (Gibco, Cat # 11875-093) + 10% FBS + 1% penicillin/streptomycin + 2 mM Glutamax medium and counted using an automated cell counter (ViCellXR). According to the optimized cell plating density in the pre-experiments, 40 µl of cell suspension at a density of 100,000 cells/ml was plated in the corresponding wells of a 384-well plate (4,000 cells/well) to ensure that cells in the control group were at linear growth phase within the five-day experimental period. The plated 384-well plate was cultured in a 37°C, 5% CO₂ incubator for 24 hours. On the day of administration, each compound was formulated into 10 mM stock solution with DMSO (Sigma, Cat # 276855-1L) and the stock solution was further diluted with DMSO to 20 µM and 20 nM working solutions. Drug treatment of MM.1S cells started at a concentration of up to 100 nM and the drug was serial diluted 4-fold with 10 points, with three replicate wells. Nanoliter volumes of compound solutions were added to the corresponding wells of the 384-well plate according to the needed concentration in a non-contact spraying manner by an HPD300 microscale automated dispenser (Tecan). The final concentration of DMSO was 0.5%. After drug addition, the 384-well plate was cultured in a 37°C, 5% CO₂ incubator for 120 hours. After five days of drug treatment, 25 µl of CTG reagent (Promega, Cat # G7573) was added to each well, and the plate was shaken on a plate shaker for 10 minutes to ensure that the cells were fully lysed, and then left to stand at room temperature for 10 minutes to ensure a sufficient reaction of CTG with the substrate and the generation of a stable luminescent signal. The signal is proportional to the amount of ATP in the cell lysate, i.e., the number of metabolically active cells. Chemiluminescence of the cells in the plate was detected by an EnSpire instrument. The reading of the cells at Day 0 was used as a control for cells at growth starting point and the reading of cells with 0.5% DMSO at Day 5 was used as a control for cells under growth. Cell proliferation inhibition curve was plotted using XLFit software and the IC50 value was calculated. Experiment results can be seen in Table 2.

**Table 2. MM.1S cell proliferation inhibition results**

| Compounds | MM.1S IC₅₀ (nM) |
|---|---|
| Control 1 | 119.0859 |
| CM-45 | 0.0938 |
| CM-46 | 0.0687 |
| CM-47 | 0.1351 |
| CM-48 | 0.0365 |
| CM-49 | 0.0606 |

## Claims

1. An isoindoline derivative represented by general formula (I), a pharmaceutically acceptable salt, a solvate, a polymorph, a metabolite, a prodrug or a stereoisomer thereof: 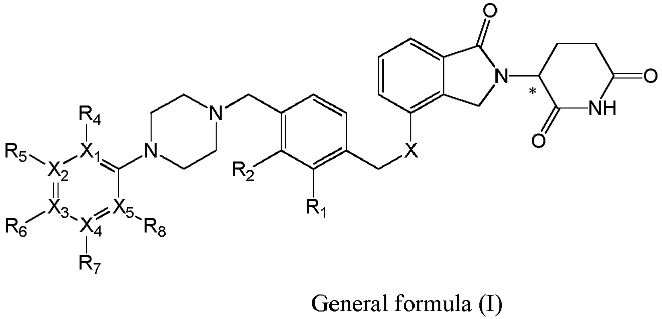
wherein, R₁ and R₂ are each independently selected from H, halogen, -CN, substituted or unsubstituted (C₁-C₁₂)alkyl, substituted or unsubstituted (C₁-C₁₂)alkoxy, or -OH, provided that: R₁ and R₂ are not both H;
X is selected from O or NH;
X₁, X₂, X₃, X₄ and X₅ are each independently selected from C or N;
R₄, R₅, R₆, R₇ and R₈ are each independently absent or selected from H, halogen, substituted or unsubstituted (C₁-C₁₂)alkyl, substituted or unsubstituted (C₁-C₁₂)alkoxy, substituted or unsubstituted (C₃-C₆)cycloalkyl, -CH=CH₂, -C≡CH , -CN, -OH, -NO₂, 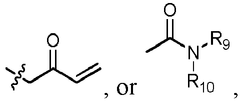 wherein R₉ and R₁₀ are each independently selected from H, D, or substituted or unsubstituted (C₁-C₁₂)alkyl, provided that: at least one of R₄, R₅, R₆, R₇ and R₈ is selected from -CN, -NO₂, 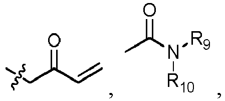 -CH=CH₂, -C≡CH , (C₁-C₁₂)alkyl substituted with one or more halogen, (C₁-C₁₂)alkoxy substituted with one or more halogen, (C₁-C₁₂)alkyl substituted with one or more D, or (C₁-C₁₂)alkoxy substituted with one or more D;
the substituents in the substituted (C₁-C₁₂)alkyl and substituted (C₁-C₁₂)alkoxy are selected from one or more D, one or more halogen, or one or more (C₃-C₆)cycloalkyl;
the carbon labeled with ^{∗} is an asymmetric center, and the asymmetric center refers to an achiral carbon, an (S)-configured carbon, an enriched (S)-configured carbon, a (R)-configured carbon, an enriched (R)-configured carbon or a racemate;
preferably, in general formula (I), at least one of R₁ and R₂ is selected from halogen, - CN, substituted or unsubstituted (C₁-C₁₂)alkyl, substituted or unsubstituted (C₁-C₁₂)alkoxy, or -OH;
preferably, in general formula (I), at least one of R₁ and R₂ is selected from F, Cl, Br,-CN, -CH₃, -OCH₃, -CF₃, -OCF₃;
more preferably, in general formula (I), at least one of R₁ and R₂ is selected from F;
preferably, in general formula (I), the R₄ is selected from -CN, -NO₂, 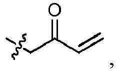 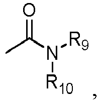 (C₁-C₁₂)alkyl substituted with one or more halogen, (C₁-C₁₂)alkoxy substituted with one or more halogen, (C₁-C₁₂)alkyl substituted with one or more D, or (C₁-C₁₂)alkoxy substituted with one or more D; R₅, R₆, R₇ and R₈ are each independently selected from H or halogen; and R₅, R₆, R₇ and R₈ are more preferably each independently selected from H;
or, in general formula (I), the R₈ is selected from -CN, -NO₂, 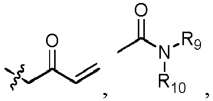 (C₁-C₁₂)alkyl substituted with one or more halogen, (C₁-C₁₂)alkoxy substituted with one or more halogen, (C₁-C₁₂)alkyl substituted with one or more D, or (C₁-C₁₂)alkoxy substituted with one or more D; R₄, R₆, R₇ and R₈ are each independently selected from H or halogen; and R₄, R₆, R₇ and R₈ are more preferably each independently selected from H;
or, in general formula (I), the R₆ is selected from -CN, -NO₂, 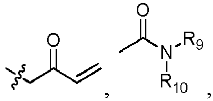 (C₁-C₁₂)alkyl substituted with one or more halogen, (C₁-C₁₂)alkoxy substituted with one or more halogen, (C₁-C₁₂)alkyl substituted with one or more D, or (C₁-C₁₂)alkoxy substituted with one or more D; R₄, R₅, R₇ and R₈ are each independently selected from H or halogen; and R₄, R₅, R₇ and R₈ are more preferably each independently selected from H;
or, in general formula (I), the R₇ is selected from -CN, -NO₂, 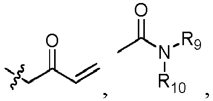 (C₁-C₁₂)alkyl substituted with one or more halogen, (C₁-C₁₂)alkoxy substituted with one or more halogen, (C₁-C₁₂)alkyl substituted with one or more D, or (C₁-C₁₂)alkoxy substituted with one or more D; R₄, R₅, R₆ and R₈ are each independently selected from H or halogen; and R₄, R₅, R₆ and R₈ are more preferably each independently selected from H;
or, in general formula (I), the R₈ is selected from -CN, -NO₂, 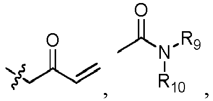 (C₁-C₁₂)alkyl substituted with one or more halogen, (C₁-C₁₂)alkoxy substituted with one or more halogen, (C₁-C₁₂)alkyl substituted with one or more D, or (C₁-C₁₂)alkoxy substituted with one or more D; R₄, R₅, R₆ and R₇ are each independently selected from H or halogen, and R₄, R₅, R₆ and R₇ are more preferably each independently selected from H.

2. The isoindoline derivative represented by general formula (I), the pharmaceutically acceptable salt, the solvate, the polymorph, the metabolite, the prodrug or the stereoisomer thereof according to claim 1, **characterized in that**, R₁ is selected from F, Cl, Br, -CN, -CH₃, -OCH₃, -CF₃, or -OCF₃, and R₂ is selected from H; or R₂ is selected from F, Cl, Br,-CN, -CH₃,- OCH₃, -CF₃ or -OCF₃, and R₁ is selected from H; preferably, R₁ is selected from F, and R₂ is selected from H; or R₂ is selected from F, and R₁ is selected from H.

3. The isoindoline derivative represented by general formula (I), the pharmaceutically acceptable salt, the solvate, the polymorph, the metabolite, the prodrug or the stereoisomer thereof according to claim 1, **characterized in that**,
R₄ is selected from H, F, Cl, Br, -CH₃, -CH₂CH₃, -CF₃, -OCH₃, -OCH₂CH₃, -CH=CH₂, -C≡CH, -OCF₃, -OCH₂F, -OCHF₂, -CD₃, -OCD₃, -CN, -NO₂, 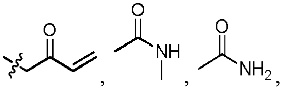 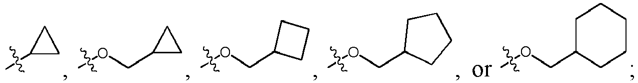 R₄ is preferably selected from H, F, -CN, -OCH₃, -OCF₃, -OCD₃, or -CD₃; R₄ is more preferably selected from H or F; and R₄ is most preferably selected from H;
R₅ is selected from H, F, Cl, Br, -CH₃, -CH₂CH₃, -CF₃, -OCH₃, -OCH₂CH₃, -CH=CH₂, -C≡CH, -OCF₃, -OCH₂F, -OCHF₂, -CD₃,-OCD₃, -CN, -NO₂, 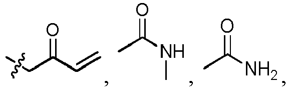 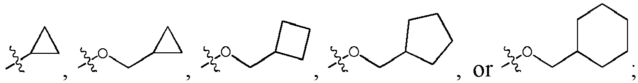 R₅ is preferably selected from H, F, -CN, -OCH₃, -OCF₃, -OCD₃, or -CD₃; R₅ is more preferably selected from H or F; and R₅ is most preferably selected from H;
R₆ is selected from H, F, Cl, Br, -CH₃, -CH₂CH₃, -CF₃, -OCH₃, -OCH₂CH₃, -CH=CH₂, -C≡CH, -OCF₃, -OCH₂F, -OCHF₂, -CD₃, -OCD₃, -CN,-NO₂, 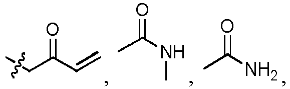 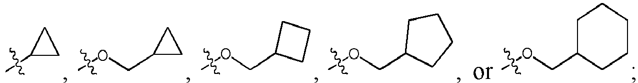 R₆ is preferably selected from H, F, -CN, -OCH₃, -OCF₃, -OCD₃, or -CD₃; R₆ is more preferably selected from F or - CN; and R₆ is most preferably selected from -CN;
R₇ is selected from H, F, Cl, Br, -CH₃, -CH₂CH₃, -CF₃, -OCH₃, -OCH₂CH₃, -CH=CH₂, -C≡CH, -OCF₃, -OCH₂F, -OCHF₂, -CD₃, -OCD₃, -CN, -NO₂, 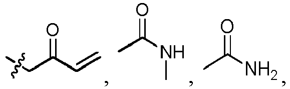 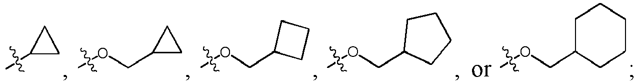 R₇ is preferably selected from H, F, -CN, -OCH₃, -OCF₃, -OCD₃, or -CD₃; R₇ is more preferably selected from H, F or -OCH₃; and R₇ is most preferably selected from -OCH₃;
and R₈ is selected from H, F, Cl, Br, -CH₃, -CH₂CH₃, -CF₃, -OCH₃, -OCH₂CH₃, - CH=CH₂, -C≡CH, -OCF₃, -OCH₂F, -OCHF₂, -CD₃, -OCD₃, -CN, -NO₂, 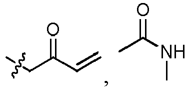 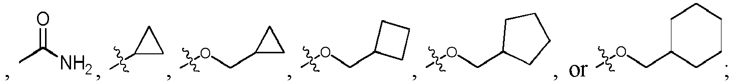 R₈ is preferably selected from H, F, -CN, -OCH₃, -OCF₃, -OCD₃, or -CD₃; R₈ is more preferably selected from F or H; and R₈ is most preferably selected from F.

4. The isoindoline derivative represented by general formula (I), the pharmaceutically acceptable salt, the solvate, the polymorph, the metabolite, the prodrug or the stereoisomer thereof according to claim 1, **characterized in that**, X is O.

5. The isoindoline derivative represented by general formula (I), the pharmaceutically acceptable salt, the solvate, the polymorph, the metabolite, the prodrug or the stereoisomer thereof according to claim 1, **characterized in that**, X₁, X₂, X₃, X₄, and X₅ are each independently C.

6. The isoindoline derivative represented by general formula (I), the pharmaceutically acceptable salt, the solvate, the polymorph, the metabolite, the prodrug or the stereoisomer thereof according to any one of claims 1-5, **characterized in that**, the halogen is F, Cl, Br or I.

7. The isoindoline derivative represented by general formula (I), the pharmaceutically acceptable salt, the solvate, the polymorph, the metabolite, the prodrug or the stereoisomer thereof according to any one of claims 1-5, **characterized in that**, the compound of general formula (I) is any one of the following compounds:

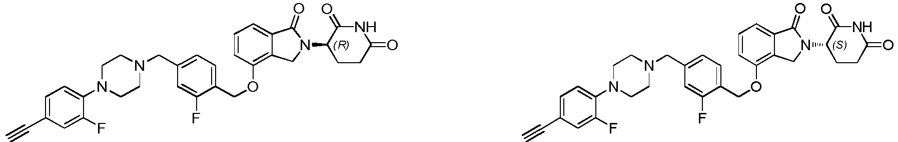

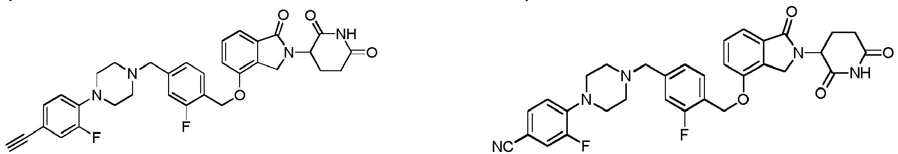

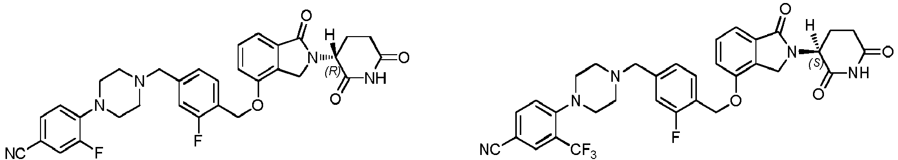

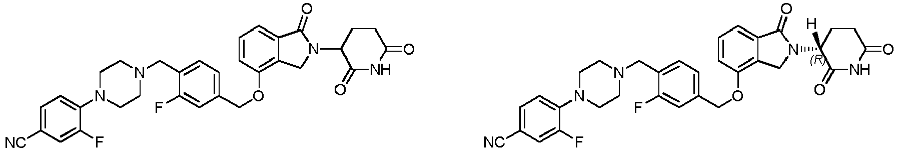

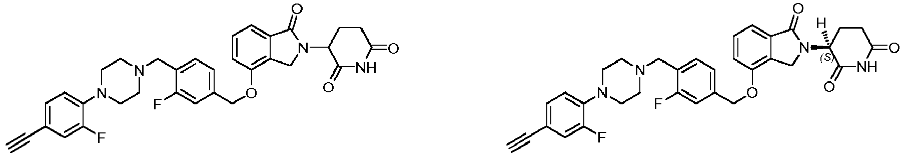

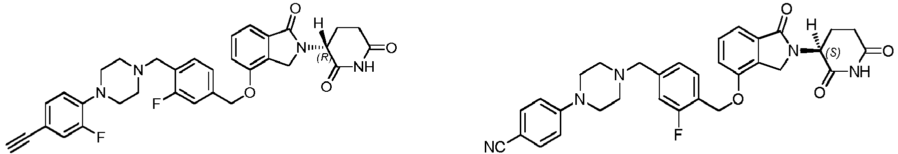

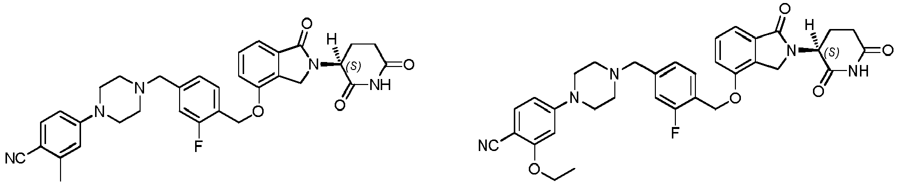

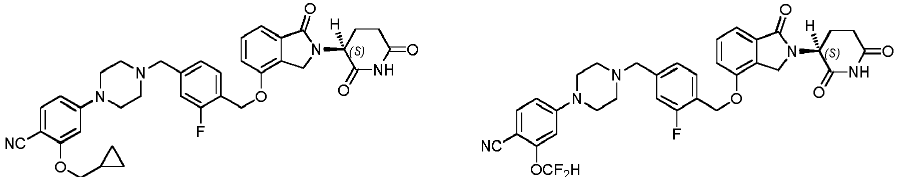

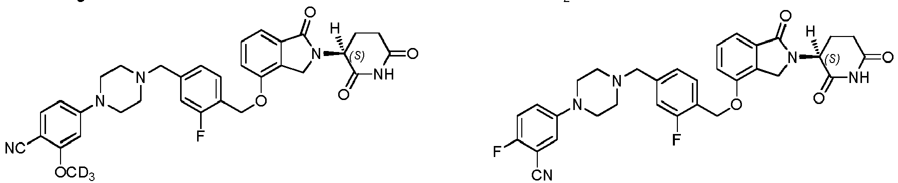

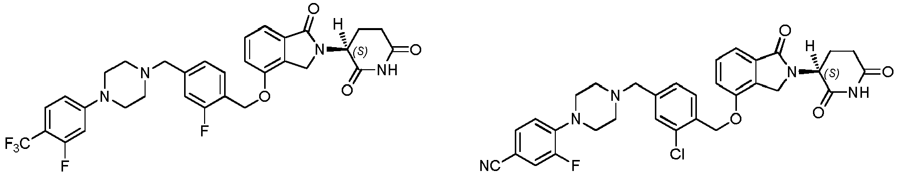

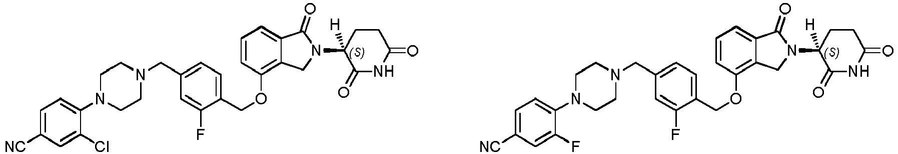

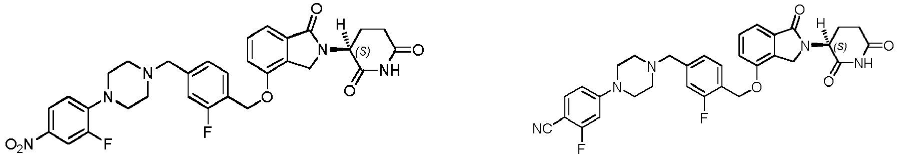

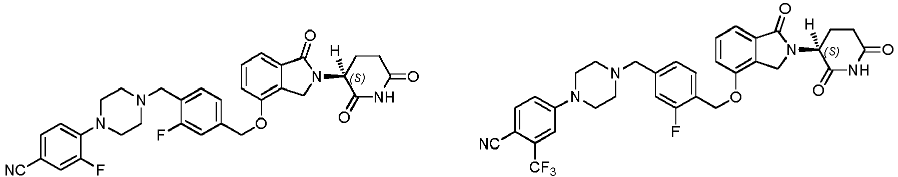

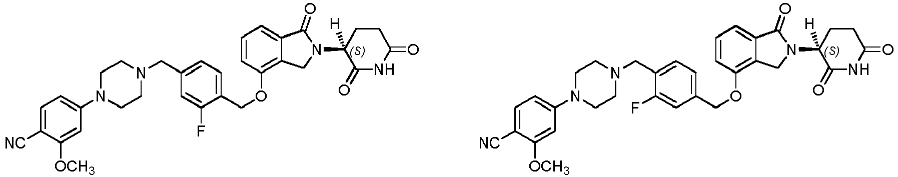

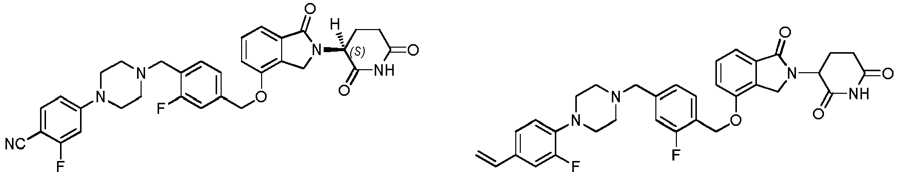

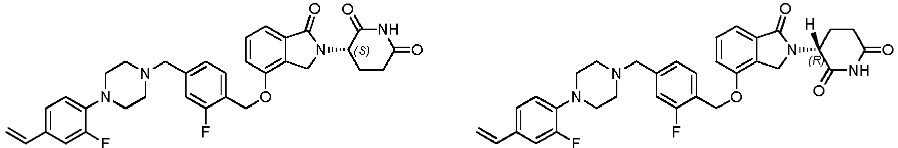

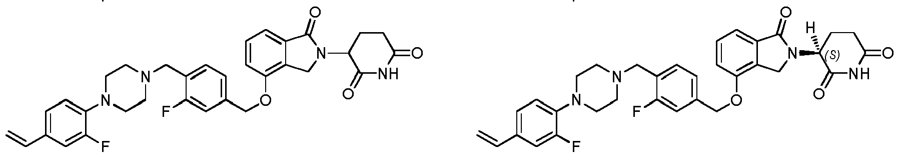

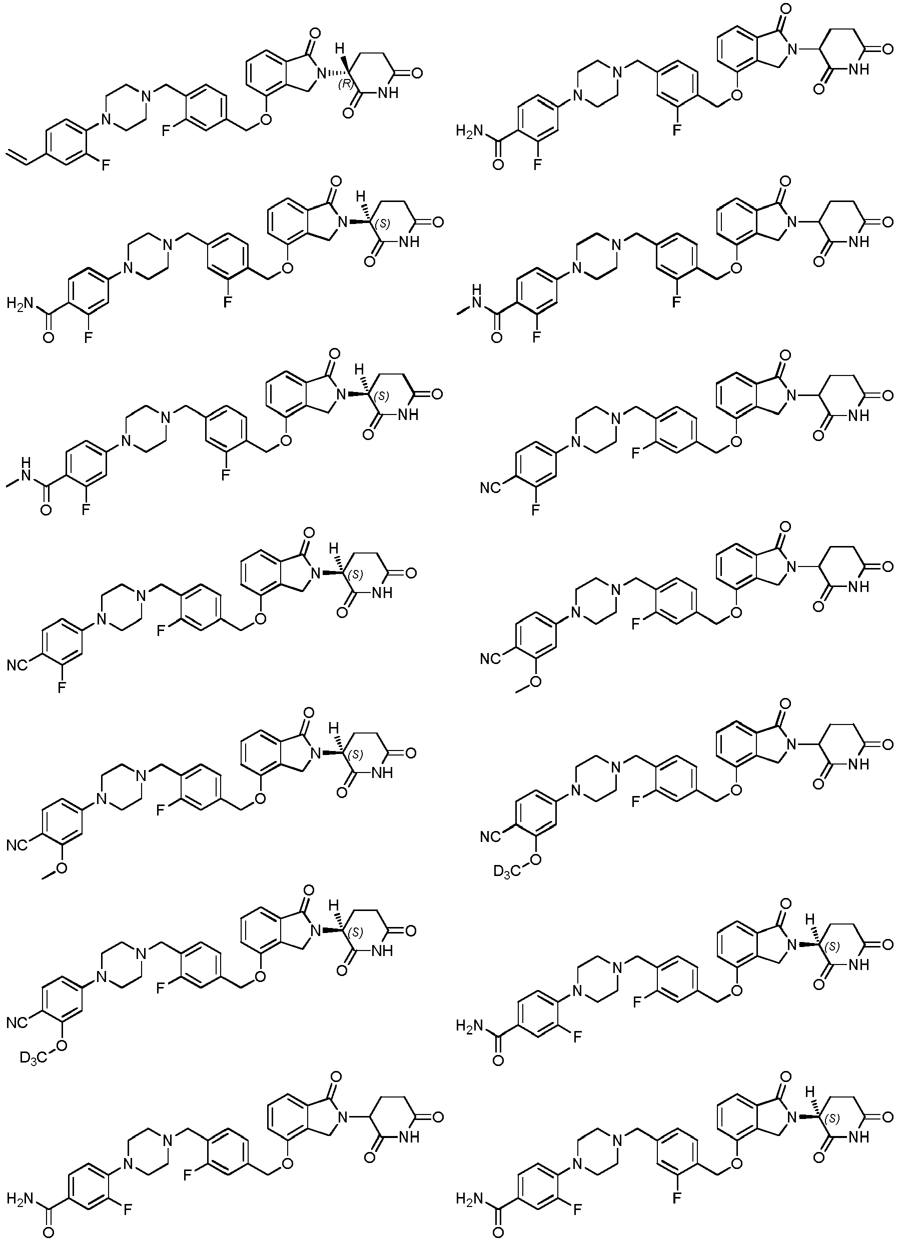

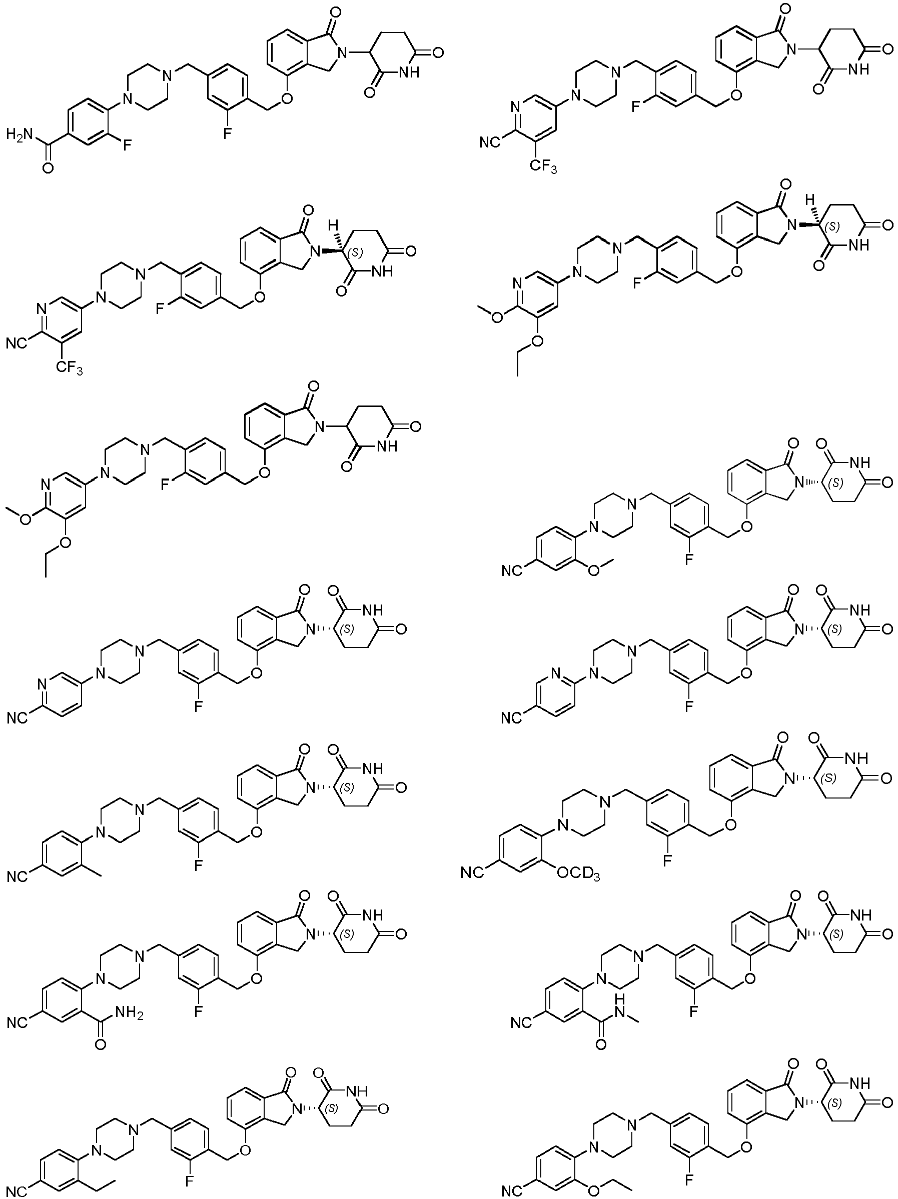

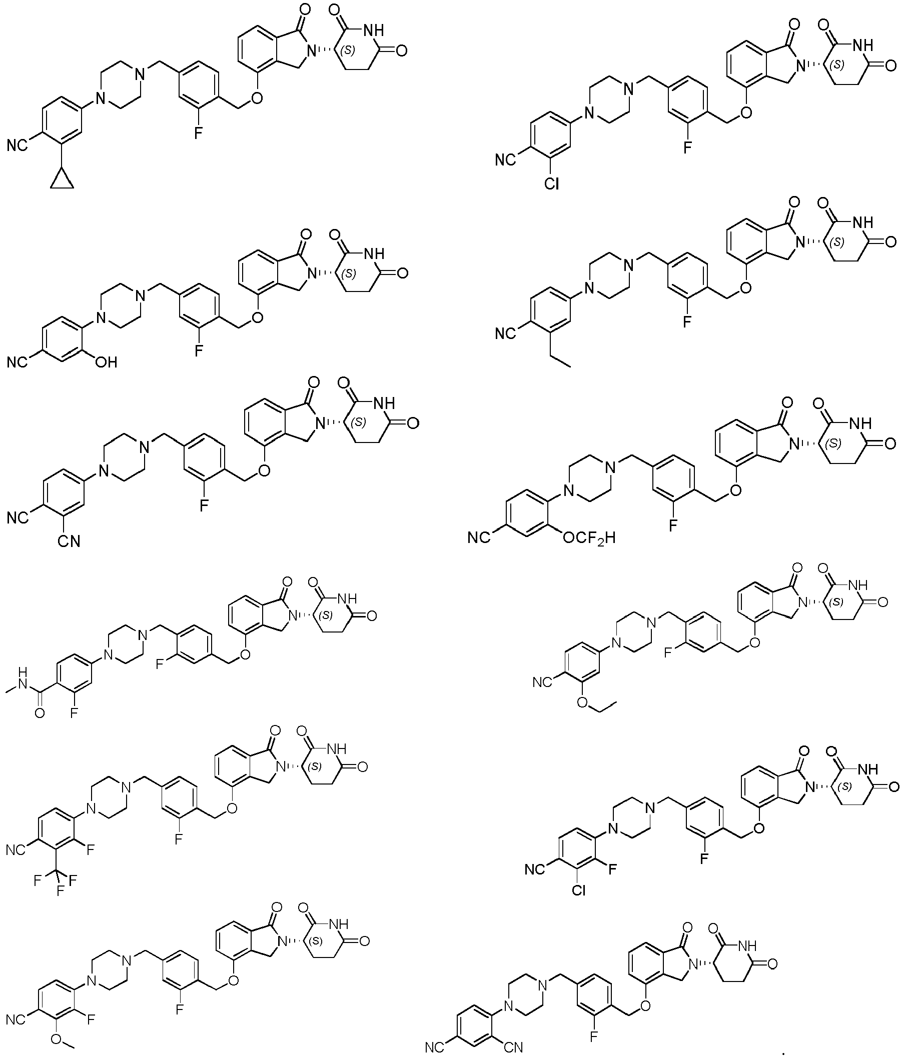

8. A method for preparing the isoindoline derivative represented by general formula (I) according to any one of claims 1-7, **characterized in that**, the method comprises the following step:
obtain the compound of general formula (I) from compound A-01 by reaction of 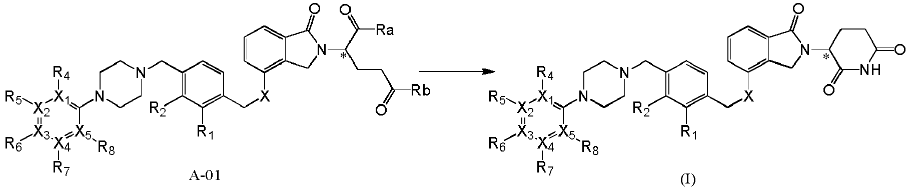 wherein, the definitions of X, X₁-X₅, ^{∗}, R₁, R₂, R₄, R₅, R₆, R₇ and R₈ are all as described in any one of claims 1-7, one of Ra and Rb is 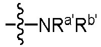 and the other is 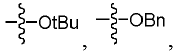 or 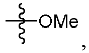 and R^{a'} and R^{b'} are independently H.

9. A pharmaceutical composition, comprising a therapeutically and/or prophylactically effective amount of the isoindoline derivative represented by general formula (I), the pharmaceutically acceptable salt, the solvate, the polymorph, the metabolite, the prodrug or the stereoisomer thereof according to any one of claims 1-7.

10. The pharmaceutical composition according to claim 9, **characterized in that**, the pharmaceutical composition further comprises additional therapeutic agents.

11. Use of the isoindoline derivative represented by general formula (I), the pharmaceutically acceptable salt, the solvate, the polymorph, the metabolite, the prodrug or the stereoisomer thereof according to any one of claims 1-7 in the preparation of a drug for treating related diseases, disorders or conditions by inducing ubiquitination and degradation of target proteins in cells.

12. Use according to claim 11, **characterized in that**, the diseases, disorders or conditions are cancers, preferably multiple myeloma.
